# EUROPEAN PATENT SPECIFICATION

(11) **EP 2 432 897 B1**
(45) Date of publication and mention of the grant of the patent: **17.04.2013**
(21) Application number: 10726724.7
(22) Date of filing: 20.05.2010
(51) Int. Cl.: C12Q 1/68, G01N 33/569

(54) **METHODS FOR WHOLE-CELL ANALYSIS OF GRAM-POSITIVE BACTERIA**
VERFAHREN FÜR GANZZELLIGE ANALYSE VON GRAMPOSITIVEN BAKTERIEN
PROCÉDÉS D'ANALYSE DE CELLULES ENTIÈRES DE BACTÉRIES GRAM-POSTITIVES

(30) Priority: 10.01.2010 US 293674 P; 20.05.2009 US 179900 P
(43) Date of publication of application: 28.03.2012
(73) Proprietor: AdvanDx, Inc., Woburn, MA 01801 (US)
(72) Inventor: STENDER, Henrik, DK-2820 Gentofte (DK); TRNOVSKY, Jan, Saugus MA 01906 (US); KLIMAS, Lisa, L., East Boston MA 02128 (US); RASMUSSEN, Anne, K., I., DK-2930 Klampenborg (DK)
(74) Representative: Grund, Martin
(86) International application number: PCT/US2010/035492
(87) International publication number: WO 2010/135480

(56) References cited:
- EP-A1- 0 497 464
- WO-A1-99/54502
- WO-A2-2007/044091
- WO-A2-2008/143972
- US-A1- 2004 229 268
- US-A1- 2008 108 064
- HULETSKY A ET AL: "New real-time PCR assay for rapid detection of methicillin-resistant Staphylococcus aureus directly from specimens containing a mixture of staphylococci" JOURNAL OF CLINICAL MICROBIOLOGY, AMERICAN SOCIETY FOR MICROBIOLOGY, WASHINGTON, DC, US LNKD- DOI:10.1128/JCM.42.5.1875-1884.2004, vol. 42, no. 5, 1 May 2004 (2004-05-01), pages 1875-1884, XP003003502 ISSN: 0095-1137
- FURAKAWA ET AL.: "Comprehensive Analysis of Cell Wall-Permeabilizinq Conditions for Highly Sensitive Fluorescence In Situ Hybridization", MICROBES ENVIRON., vol. 21, no. 4, 2006, pages 227-234,
- COLEMAN ET AL.: "mRNA-tarqeted fluorescent in-situ hybridization (FISH) of Gram-neqative bacteria without template amplification or tyramide siqnal amplification", J. MICROBIOLOGICAL METHODS, vol. 71, 2007, pages 246-255,
- HAHN ET AL.: APPLIED AND ENVIRONMENTAL MICROBIOLOGY, vol. 59, no. 8, August 1993 (1993-08), pages 2753-2757,
- HONERTAGE ET AL.: "Detection of mRNA of nprM in Bacillus meqaterium ATTC 14581 qrown in soil by whole-cell hvbridization", ARCH. MICROBIOL., vol. 163, 1995, pages 235-241,
- WAGNER ET AL.: "In situ detection of a virulence factor mRNA and 16S rRNA in Listeria", FEMS MICROBIOL. LETT., vol. 160, no. 1, March 1998 (1998-03), pages 159-168,

## Description

### Cross Referent to Related Applications

This application claims the benefit of United States Provisional Patent Application No. 61/179,900, filed on May 20, 2009 and United States Provisional Patent Application No. 61/293,674 filed on January 10, 201.

### Brief Description of Drawings

*This patent or application file contains at least one drawing executed in color. Copies of this patent or patent application publication with color drawing(s) will be provided by the Office upon request and payment* of *the necessary fee.*

*The skilled artisan will understand that the drawings, described below, are for illustration purposes only. The drawings are not intended to limit the scope* of *the present teaching in any way.*

*In the drawings, the sizes and relative positions of elements in the drawings are not necessarily drawn to scale. For example, the shapes of various elements and angles may not be drawn to scale, and some of these elements are arbitrarily enlarged and positioned to improve drawing legibility. Further, the particular shapes of the elements as drawn may not be intended to convey any information regarding the actual shape of the particular elements, and may have been selected solely for ease of recognition in the drawings.*

Fig. 1 contains five microscope images (viewed with a green (FITC) filter) of five different slides (Slides A-E) comprising bacteria wherein each slide differs in its bacteria and/or treatment conditions.

Fig. 2 contains four microscope images of two different slides (Slide A and Slide B) comprising stained bacteria treated identically (as compared with the other slide) wherein each slide contains a different bacteria. Images of each slide were obtained using a dual band filter (images A1 and B1) and a green (FITC) filter (Images A2 and B2).

Fig. 3 contains three microscope images of a single slide comprising stained bacteria, wherein each image was taken from the same section of the slide and was obtained with a green/red dual band filter (Image A, FITC & Texas Red), blue (DAPI) filter (Image B) or green (FITC) filter (Image C).

Fig. 4 contains two microscopic images. In Image A no bacteria are seen because there was no signal to amplify. In Image B, bacteria are visible as there was signal to amplify.

### Description

### 1. Field

This application relates to the field of whole-cell analysis and, in some embodiments, pertains to the analysis of methicillin-resistant *staphylococcus aureus* (MRSA) bacteria and/or methicillin-resistant coagulase-negative *staphylococci* (MR-CNS). The scope of the present invention is determined by the attached claims.

### 2. Introduction

Bacteria in clinical samples (e.g. nasal or wound swabs, blood or urine samples/cultures, etc.) are commonly identified phenotypically, genotypically or by using immuno-based methods. Certain types of bacteria, such as methicillin-resistant *staphylococcus aureus* (MRSA), are rapidly spreading worldwide in hospitals and more recently in the community and thus pose a serious risk to human health. Resistance to methicillin (and to all β-lactam antibiotics) in bacteria, such as *staphylococcus aureus* (S. *aureus*) is primarily associated with the acquisition of the mecA gene that encodes for the penicillin-binding protein 2a (PBP2a, also known as PBP2'). The PBP2a protein is involved in bacterial cell wall synthesis (See: Pinho et al., "An acquired and a native Penicillin-binding protein cooperate in building the cell wall of drug-resistant staphylococci", PNAS, 98(19): 10886-10891 (Sept. 2001)). Rapid and accurate identification of MRSA is considered to be critical in preventing and treating disease caused by S. *aureus.*

Phenotype assays often involve culturing organisms in the presence of antibiotics and checking for bacterial growth. This testing typically involves at least two days in order to obtain results (one day for isolation and one day for sensitivity testing). Because some phenotypic characteristics (e.g. MRSA's drug resistance) are manifested through a complicated mechanism, culture conditions (salinity, temperature, inoculum size, etc.) can affect the outcome quite substantially. In some cases, this can delay diagnosis or even cause misdiagnosis.

Immuno-based methods for the detection of the PBP2a protein has been described (e.g. Cavassini et al., "Evaluation of MRSA-Screen, a Simple Anti-PBP 2a Slide Latex Agglutination Kit, for Rapid Detection of Methicillin Resistance in Staphylococcus aureus", J. Clinical Microbiology, 37(5): 1591-1594 (May, 1999)). This **cell-free assay** is capable of rapid detection of the PBP2a protein in a sample of cell-lysate and thereby confirms methicillin-resistance of at least some of the organisms of the sample. However, this assay is often used to analyze organisms grown in culture (which requires a day or two to grow) and is not very sensitive (requires approximately 10⁷ colony forming units for reliable identification).

The most rapid and sensitive genotypic identification methods employ nucleic acid analysis of the bacteria in combination with nucleic acid amplification techniques. For example, several assays utilize the polymerase chain reaction (PCR) or other target amplification methods (e.g. ligase chain reaction). Some examples of PCR assays adapted for MRSA detection can be found in US 5,702,895 (Matsunaga et al.), 6,156,507 (Hiramatsu et al.) and 7,074,599 (Uhl et al.). These PCR assays are **cell-free assays**. A PCR assay for determining MRSA and MSSA is also found in Gr6bner et al., "Evaluation of the BD GeneOhm StaphSR Assay for Detection of Methicillin-Resistant and Methicillin-Susceptible Staphylococcus aureus Isolates from Spiked Positive Blood Culture Bottles", J. Clin. Microbiol., 47(6): 1689-1694 (2009).

One drawback to cell-free assays is the loss of cell morphology, which morphology is often valuable in bacterial identification. Cell-free assays are also less likely to be useful in determining (or at least involve more complex design to determine) mixed populations of organisms (See: Gröbner et al. (2009) at 1691. col. 1, second paragraph under the heading: *"Analysis of blood culture bottles spiked with mixtures of staphylococcal isolates"* for a discussion of the deficiencies of PCR assays for determining mixed populations). One avenue to preservation of morphology, as well as to more easily identify mixed populations of bacteria in a sample and/or eliminate the potential risk of false-positive results due to mixed populations, is to perform whole-cell analysis such as *in-situ* (inside the cell) analysis.

In order to determine traits (such as methicillin-resistance for example) within bacteria by whole-cell analysis, the chromosomal deoxyribonucleic acid (DNA), the mRNA or cellular proteins of the bacteria are typically analyzed. Some traits are also associated with native plasmids. The invention disclosed herein is not directed to the analysis of cellular protein to thereby determine a trait.

While many reports exist for the in-situ hybridization (ISH) analysis of mRNA in eukaryotic cells, reports of the ISH-based analysis of mRNA or chromosomal DNA in bacteria appear to be less prevalent. At least in part a result of the properties of their cell wall, ISH-based analysis of gram-positive bacteria has proven particularly problematic (See for example: Furakawa et al., "Comprehensive Analysis of Cell Wall-Permeabilizing Conditions for Highly Sensitive Fluorescence In Situ Hybridization", Microbes Environ., 21(4): 227-234 (2006)). Moreover, ISH-based analysis of mRNA and chromosomal DNA are also complicated by low copy number and the instability of mRNA within bacteria (See for example the Introduction to: Coleman et al., "mRNA-targeted fluorescent in-situ hybridization (FISH) of Gram-negative bacteria without template amplification or tyramide signal amplification", J. Microbiological Methods, 71: 246-255 (2007)).

There are at least three reports of the successful ISH-based analysis of mRNA in a gram-positive bacteria (See: Hahn et al., "Detection of mRNA in Streptomyces Cells by Whole-Cell Hybridization with Digoxigenin-Labeled Probes", Applied and Environmental Microbiology, 59(8): 2753-2757 (Aug. 1993); Wagner et al., "In situ detection of a virulence factor mRNA and 16S rRNA in Listeria", FEMS Microbiol. Lett., 160(1): 159-168 (Mar. 1998); and Hönerlage et al., "Detection of mRNA of nprM in Bacillus megaterium ATTC 14581 grown in soil by whole-cell hybridization", Arch. Microbiol., 163: 235-241 (1995)).

In Hahn et al., *Streptomyces violacelatus* (*S. violacelatus*) bacteria were engineered to contain an inserted plasmid (plasmid pIJ673) comprising the thiostrepton resistance (tsr) gene (Abstract) which permitted both ribosomal RNA (rRNA) and messenger RNA (mRNA; mRNA being produced in high copy number by the inserted plasmid) to be targets (see: page 2753, col. 2). Accordingly, the bacteria were not naturally occurring. However, native bacteria (i.e. bacteria without the inserted plasmid) did not show any hybridization signal (see: page 2755-2756, bridging paragraph). Samples were enzymatically treated to permeabilize the gram-positive bacteria. Hybridization reactions with mRNA-directed probes were performed over 1.6 hours (see: page 2754, col. 2, bottom). It is also noteworthy that in addition to increasing the mRNA content of the bacteria by use of the plasmid, signal amplification was still required. Specifically, a water-insoluble dye, which was generated by activity of alkaline phosphatase conjugated to an anti-digoxigenin antibody linked to the transcript probe via interaction with the multiple digoxigenin labels (per probe), was used to stain the cells for ISH analysis (see: page 2754-2755, bridging paragraph).

Similarly in Wagner et al., enzymatic digestion of the cell wall was performed to permeabilize the bacteria. It is also noteworthy that attempts to detect the mRNA target using four single labeled *iap*-mRNA-directed probes and a very bright fluorescent dye was unsuccessful (See: page 166, col. 1-2, bridging paragraph). Also, attempts to detect the mRNA target using single (horseradish peroxidase) enzyme labeled probes in combination with fluorescein tyramide (a signal amplification technique) proved unsatisfactory (See: page 166-167). However, a transcript probe, comprising multiple digoxigenin labels combined with anti-digoxigenin antibody fragments conjugated to horseradish peroxidase (a signal amplification technique), was used to detect, via catalytic deposition of fluorescein tyramide, *iap*(invasion associated protein)-mRNA in Listeria monocytogenes cells (see: Abstract and page 167). Hybridization reactions with digoxigenin-labeled mRNA-directed probes were performed over 5 hours (see: page 162, col. 2, last paragraph).

Likewise in Honerlage et al., cells of *Bacillus megaterium* were treated enzymatically to permeabilize the bacteria (See: page 237, col. 1 under the heading "Whole-cell hybridization"). Also, a transcript probe, comprising multiple digoxigenin labels (see: "Probes" at page 236, col. 2) combined with anti-digoxigenin antibody fragments conjugated to alkaline phosphatase (a signal amplification technique), was used (in combination with nitroblue tetrazolium and 5-bromo-4-chloro-3-indolytphosphate) to generate stained cells and to detect the mRNA of nprM in *Bacillus megaterium.* Hybridizations with transcript probes were performed for 16 hours.

From the foregoing, it is clear that for successful analysis of mRNA in gram-positive bacteria, Hahn et al., Wagner et al. and Hönerlage et al. all utilized; 1) enzymatic treatments to permeabilize the bacterial cells; 2) transcript probes (which are typically long (i.e. greater than 100bp in length)) with multiple digoxigenin labels); 3) indirect detection of said multiple digoxigenin labels by enzyme-based signal amplification techniques; and 4) fairly long probe hybridization steps (i.e. 5-16 hours).

There does not appear to be any literature report of the unambiguous detection of mRNA in whole cells in gram-positive bacteria in the absence of the use of indirect detection of labeled transcript probes comprising multiple labels and/or application of amplification techniques (e.g. signal amplification and/or in-situ nucleic acid amplification such as in-situ PCR). Moreover, there does not appear to be any report whatsoever of the detection of mRNA in whole cells of *staphylococci* bacteria. This apparent lapse in the scientific literature is likely due, at least in part, to the aforementioned low copy number of mRNA (as well as chromosomal DNA) and its instability in bacteria as well as with the difficulties associated with permeabilization of the cell wall of gram-positive bacteria which difficulty complicates whole-cell (e.g. ISH and fluorescence in-situ hybridization (FISH)) analysis techniques.

### 3. Definitions

*For the purposes of interpreting this specification and the appended claims, the following definitions will apply and whenever appropriate, terms used in the singular will also include the plural and vice versa. In the event that any definition set forth below conflicts with the usage of that word in any other document, the definition set forth below shall always control for purposes of interpreting the scope and intent of this specification and its associated claims. Notwithstanding the foregoing, the scope and meaning of any document referred to herein should not be altered by the definition presented below. Rather, said document should be interpreted as it would be by the ordinary practitioner based on its content and disclosure with reference to the content of the description provided herein.*

*The use* of *"or" means "and*/*or" unless stated otherwise or where the use of "and*/*or" is clearly inappropriate. The use of "a" means "one or more" unless stated otherwise or where the use of "one or more" is clearly inappropriate. The use* of *"comprise," "comprises," "comprising" °include," "includes,"and "including" are interchangeable and not intended to be limiting. Furthermore, where the description of one or more embodiments uses the term "comprising, " those skilled in the art would understand that in some specific instances, the embodiment or embodiments can be alternatively described using language "consisting essentially of" and*/*or "consisting of."*

As used herein, *"antibody"* refers to an immunoglobulin protein or to a fragment or derivative thereof which is capable of participating in antibody/antigen binding interaction(s). A discussion of the technical features of antibodies, their fragments, methods for detection of antibodies/antibody fragments and related topics can be found in the Pierce Catalog and Handbook, 1994 (Section T). Antibodies include, for example, various classes and isotypes of immunoglobulins, such as IgA, IgD, IgE, IgG1, IgG2a, IgG2b, IgG3, and IgM. Antibody fragments include molecules such as Fab, scFv, F(ab')₂ and Fab' molecules. Antibody derivatives include antibodies or fragments thereof having additions or substitutions, such as chimeric antibodies. Antibodies can be derived from human or animal sources, from hybridomas, through recombinant methods, or in any other way known to the art.

As used herein, *"cell permeabilizing reagent or reagents"* refers to a reagent, two or more reagents, a mixture of reagents or a formulation used to treat bacterial cells to thereby modify the cell's wall/outer membrane so that other analysis reagents (e.g. probes, detector reagents, antibodies, etc.) can penetrate (and thereby enter) said bacterial cells. Some examples of enzymes that can be used as cell permeabilizing reagents include the enzymes: lysostaphin, lysozyme, and proteinases (e.g. proteinase-K and/or achromopeptidase). When more than one reagent is used to permeabilize bacterial cells, the permeabilizing reagents can be added sequentially, simultaneously, or a combination of some reagents being added sequentially and some being added simultaneously. In short, there is no limitation on the manner in which the reagent or reagents are contacted with the bacteria so long as the process adequately permeabilizes the bacterial cells. In some embodiments, methods disclosed herein can be practiced by contacting the sample with a *cell permeabilizing reagent or reagents.*

As used herein, *"chimera"* refers to an oligomer comprising subunits of two or more different classes of subunits. For example, a chimera can comprise subunits of deoxyribonucleic acid (DNA) and locked nucleic acid (LNA), can comprise subunits of DNA and ribonucleic acid (RNA), can comprise subunits of DNA and peptide nucleic acid (PNA), can comprise subunits of DNA, LNA and PNA or can comprise subunits of RNA and LNA, etc. It is to be understood that what the literature refers to as LNA probes are typically chimeras (according to this definition), since said "LNA probes" usually incorporate only one or a few LNA nucleotides into an oligomer. The remaining nucleotides are typically standard DNA or RNA nucleotides.

As used herein, *"chromosomal DNA- mRNA- and*/*or native plasmid-directed labeled probe or probes"* refers to a probe or probes that are each labeled with one or more labels (in some embodiments the probe or probes will comprise only a single label), where said probe or probes are selected to bind with a high degree of specificity to a target in the chromosomal DNA, the mRNA and/or a native plasmid of bacteria sought to be determined in the assay (e.g. the select gram-positive bacteria). The chromosomal DNA, mRNA and/or native plasmid target is selected because it codes for (and/or is associated with) the select trait sought to be determined in the assay.

As used herein, *"determining"* refers to making a decision based on investigation, data, reasoning and/or calculation. Some examples of determining include detecting, identifying and/or locating (bacteria and/or traits) as appropriate based on the context/usage of the term herein.

As used herein, *"fixation"* refers to specimen preservation and/or sterilization where cellular nucleic acid (DNA and RNA) integrity and cellular morphology are substantially maintained. Fixation can be performed either chemically using one or more solutions containing one or more fixing agent(s) and/or mechanically, such as for example by preparation of a smear on a microscope slide and subsequently heating the smear either by passing the slide through a flame or placing the slide on a heat block.

As used herein, *"fixative reagent or reagents"* refers a reagent, two or more reagents, a mixture of reagents, a formulation or even a process (with or without associated use of reagent(s) (including mixture(s) or formulation(s)) to treat bacterial cells to thereby preserve and/or prepare said bacterial cells for microscopic analysis. Some examples of fixative reagents include paraformaldehyde, gluteraldehyde, methanol and ethanol. When more than one reagent is used to fix bacteria, the reagents can be added sequentially, simultaneously, or a combination of some reagents being added sequentially and some being added simultaneously. In some embodiments, methods disclosed herein can be practiced by contacting the sample with a *fixative reagent or reagents.*

As used herein, *"heterogeneous"* and *"homogeneous"* is made with reference to a strain of bacteria and refers to whether or not some or all bacteria of the same strain exhibit expression (or the same degree of expression) of a select trait. In particular, the bacteria of a homogeneous strain exhibit expression (or roughly the same degree of expression) of said trait whereas a heterogeneous strain does not.

As used herein, *"in the aggregate"* refers to considering relevant subject matter as a whole rather than piecemeal.

As used herein, *"label"* refers to a structural unit (or structural units as the case may be) of a composition (e.g. a hybridization probe) that renders the composition detectable by instrument and/or method. Non-limiting examples of labels include fluorophores, chromophores, haptens, radioisotopes and quantum dots. In some embodiments, two or more of the foregoing can be used in combination to render the composition detectable or independently (uniquely) detectable. Some words that are synonymous (i.e. interchangeable) with *"label" are "detectable moiety", "tag"* and *"marker".*

As used herein, *"mRNA inducing reagent or reagents"* refers to a reagent, two or more reagents, a mixture of reagents or a formulation that when brought into contact with live gram negative bacteria or gram-positive bacteria (for example in a culture) induce the bacteria to produce mRNA and thereby increase the concentration of mRNA in said bacterial cells. By increasing the concentration of mRNA in the bacteria, formation, and thus determination, of probe/mRNA complexes (and related staining of bacteria) in the whole-cell assays can potentially be increased. In some embodiments, methods disclosed herein can be practiced by contacting the sample with a *mRNA inducing reagent or reagents.* An example of a "mRNA inducing reagent or reagents" is an antibiotic.

As used herein, *"mixed population"* refers to a mixture of two or more different strains of bacteria.

As used herein, *"native plasmid"* refers to a plasmid that exists in a bacterium in its natural state (i.e. as the bacteria are obtained from the environment and/or a natural source (e.g. a host organism such as a human being)). A native plasmid is to be distinguished from a plasmid that has been intentionally inserted into a bacteria by human intervention/manipulation (See for example the engineered S. *violacelatus* bacteria with the inserted plasmid as described by Hahn et al., Applied and Environmental Microbiology, 59(8): 2753-2757 (Aug. 1993)).

As used herein, *"nucleic acid" refers* to a nucleobase containing polymer formed from nucleotide subunits composed of a nucleobase, a ribose or 2'-deoxyribose sugar and a phosphate group. Some examples of nucleic acid are DNA and RNA.

As used herein, *"nucleic acid analog"* refers to a nucleobase containing polymer formed from subunits wherein the subunits comprise a nucleobase and a sugar moiety that is not ribose or 2'-deoxyribose and/or a linkage (between the sugar units) that is not a phosphate group. A non-limiting example of a nucleic acid analog is a locked nucleic acid (LNA: See for example, US 6,043,060, 7,053,199, 7,217,805 and 7,427,672). See: Janson and During, "Peptide Nucleic Acids, Morpholinos and Related Antisense Biomolecules", Chapter 7, "Chemistry of Locked Nucleic Acids (LNA)", Springer Science & Business, 2006 for a summary of the chemistry of LNA.

As used herein, *"nucleic acid mimic"* refers to a nucleobase containing polymer formed from subunits that comprise a nucleobase and a backbone structure that is not a sugar moiety (or that comprises a sugar moiety) but that can nevertheless sequence specifically bind to a nucleic acid. An example of a nucleic acid mimic is peptide nucleic acid (PNA: See for example, 5,539,082, 5,527,675, 5,623,049, 5,714,331, 5,718,262, 5,736,336, 5,773,571, 5,766,855, 5,786,461, 5,837,459, 5,891,625, 5,972,610, 5,986,053, 6,107,470, WO92/20702 and WO92/20703). Another example of a nucleic acid mimic is a morpholino oligomer. (See Janson and During, "Peptide Nucleic Acids, Morpholinos and Related Antisense Biomolecules", Chapter 6, "Morpholinos and PNAs Compared", Springer Science & Business, 2006 for a discussion of the differences between PNAs and morpholinos. A further example of a nucleic acid mimic is the pyrrolidinyl polyamide (PP). A PP is an oligomeric polymer comprising a nucleobase and polyamide backbone as described in US Pat. Nos. 6,403,763, 6,713,603, 6,716,961 and 7,098,321 as well as Vilaivan et al., "Hybridization of Pyrrolidinyl Peptide Nucleic Acids and DNA: Selectivity, Base-Pairing Specificity and Direction of Binding", Organic Letters, 8(9): 1897-1900 (2006).

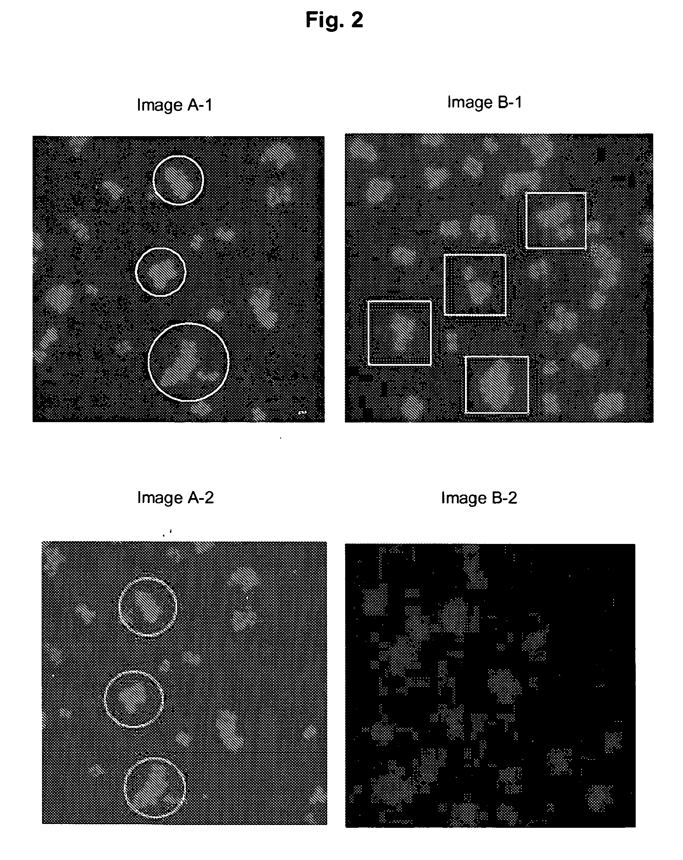

As used herein, *"nucleobase"* refers to those naturally occurring and those non-naturally occurring heterocyclic moieties commonly known to those who generate polymers that can sequence specifically bind to nucleic acids. Non-limiting examples of suitable nucleobases include: adenine, cytosine, guanine, thymine, uracil, 5-propynyl-uracil, 2-thio-5-propynyl-uracil, 5-methylcytosine, pseudoisocytosine, 2-thiouracil and 2-thiothymine, 2-aminopurine, N9-(2-amino-6-chloropurine), N9-(2,6-diaminopurine), hypoxanthine, N9-(7-deaza-guanine), N9-(7-deaza-8-aza-guanine) and N8-(7-deaza-8-aza-adenine). Other non-limiting examples of suitable nucleobase include those nucleobases illustrated in FIGS. 2(A) and 2(B) of Buchardt et al. (WO92/20702 or WO92/20703).

As used herein, *"one or more probe*/*chromosomal DNA, probe*/*mRNA and*/*or probe*/*plasmid complexes"* refers to a complex or complexes formed by: 1) binding of a probe or probes to a target in a molecule or molecules of the chromosomal DNA of a bacterial cell or cells of a sample; 2) binding of a probe or probes to a target in a molecule or molecules of the mRNA of a bacterial cell or cells of a sample; and/or 3) binding of a probe or probes to a target in a molecule or molecules of the nucleic acid of a native plasmid of a bacterial cell or cells of a sample. Typically, formation of the probe/chromosomal DNA, probe/mRNA and/or probe/plasmid complex or complexes is used herein to determine the select trait within bacteria of a sample.

As used herein, *"one or more probe*/*rRNA complexes"* refers to a complex or complexes formed by binding of a probe or probes (e.g. hybridization probe or probes) to rRNA of a bacterial cell or cells of a sample. Formation of a probe/rRNA complex or complexes can be used herein to determine the select gram-positive bacteria in the sample. Formation of a probe/rRNA complex or complexes can also be used herein to determine other bacteria in the sample, whether or not they are gram-positive.

As used herein *"one or more second probelrRNA complexes"* refers to a complex or complexes formed by binding of a second probe or second probes (i.e. a probe different from any previously mentioned probe or probes) to a target within a rRNA molecule or molecules of a bacterial cell or cells of a sample. Typically, formation of the second probe/rRNA complexes is used herein to determine another (or second) select gram-positive bacteria or another bacteria (e.g. a gram-negative bacteria) in the sample. It is to be understood that a third, fourth, fifth, sixth (etc.) probe directed to rRNA (of bacteria of interest) could be used in any assay method described herein, wherein each different probe directed to rRNA can be selected to determine a different select bacteria (some of which may not be gram-positive bacteria) that may be present in the sample. Often each of the second, third, fourth, fifth, sixth (etc.) probe is independently detectable from other probes used in practice of the method such that the method is practiced as a multiplex method. It is also to be understood that that the third probe would form a third probe/rRNA complex or complexes, the fourth probe would form a fourth probe/rRNA complex or complexes, the fifth probe would form a fifth probe/rRNA complex or complexes, the sixth probe would form a sixth probe/rRNA complex or complexes, etc.

As used herein *"one or more washing reagents"* refers to a reagent, two or more reagents, a mixture of reagents or a formulation that is used to remove various reagents and/or compositions from the sample and/or bacterial cells of the sample. In some embodiments, methods disclosed herein can be practiced by including one or more steps pertaining to contacting the sample with *one or more washing reagents.*

As used herein *"pre-hybridization step*" refers to the process of treating (e.g. contacting) a sample with a hybridization buffer that lacks a/the hybridization probe or probes for a period of time before treating (e.g. contacting) the sample with a hybridization buffer that contains a/the hybridization probe or probes. In some embodiments, methods disclosed herein can be practiced with, or without, a pre-hybridization step.

As used herein *"probe"* or *"hybridization probe"* refers to a composition that binds to a select target. A *"hybridization probe"* is a probe that binds to its respective target by hybridization. Non-limiting examples of probes include nucleic acid oligomers, (e.g. DNA, RNA, etc.) nucleic acid analog oligomers (e.g. locked nucleic acid (LNA)), nucleic acid mimic oligomers (e.g. peptide nucleic acid (PNA)), chimeras, antibodies and antibody fragments.

As used herein, *"quantum dot"* refers to an inorganic crystallite between about 1 nm and about 1000 nm in diameter or any integer or fraction of an integer there between, generally between about 2 nm and about 50 nm or any integer or fraction of an integer there between, more typically about 2 nm to about 20 nm (such as 2, 3, 4, 5, 6, 7, 8, 9, 10, 11, 12, 13, 14, 15, 16, 17, 18, 19, or 20 nm). A semiconductor nanocrystal is capable of emitting electromagnetic radiation upon excitation (i.e., the semiconductor nanocrystal is luminescent) and includes a "core" of one or more first semiconductor materials, and may be surrounded by a "shell" of a second semiconductor material. A semiconductor nanocrystals core surrounded by a semiconductor shell is referred to as a "core/shell" semiconductor nanocrystal. The surrounding "shell" material typically has a bandgap energy that is larger than the bandgap energy of the core material and can be chosen to have an atomic spacing close to that of the "core" substrate. The core and/or the shell can be a semiconductor material including, but not limited to, those of the group II-VI (ZnS, ZnSe, ZnTe, CdS, CdSe, CdTe, HgS, HgSe, HgTe, MgS, MgSe, MgTe, CaS, CaSe, CaTe, SrS, SrSe, SrTe, BaS, BaSe, BaTe, and the like) and III-V (GaN, GaP, GaAs, GaSb, InN, InP, InAs, InSb, and the like) and IV (Ge, Si, and the like) materials, and an alloy or a mixture thereof. In the scientific and patent literature the terms *"semiconductor nanocrystal," "quantum dot", "Qdot™ nanocrystal"* or simply *"nanocrystal"* are used interchangeably. For purposes of this specification, these terms are also equivalents of *"quantum dot" as* defined above.

As used herein *"rRNA-dire cted probe orprobes"* refers to a probe or probes that are selected to bind to a target or targets within a molecule or molecules of rRNA. The rRNA-directed probe or probes may be labeled with a detectable moiety or moieties or may be unlabeled. If unlabeled, complexes formed by binding of the rRNA probe to its target inside a bacteria can, for example, be detected using a labeled antibody to the probe/rRNA complex or complexes (See for example: US Pat. No. 5,612,458 to Hyldig-Nielsen).

Typically, the rRNA target is selected to differentiate between bacteria in the sample and thereby permit the determination of the select gram-positive bacteria (or other bacteria) of the sample. Using probes directed to a target within the rRNA of a bacteria to differentiate between (and thereby determine) bacteria in a sample has long been used in ISH and FISH based assays (See for example: Amann, R., "Methodological Aspects of Fluorescence In Situ Hybridization", Bioscience Microflora, 19(2): 85-91 (2000) and Pernthaler et al., "Fluorescence in situ Hybridization (FISH) with rRNA-targeted Oligonucleotide Probes", Methods in Microbiology, 30: 207-226 (2001)). With respect to the use of rRNA-directed PNA and LNA probes in FISH see: Cerqueira et al., "DNA Mimics for the Rapid Identification of Microorganisms by Fluorescence in situ Hybridization (FISH)", Int. J. Mol. Sci., 9: 1944-1960 (2008). With respect to the use of rRNA-directed PNA probes for determination of staphylococci in blood samples, see: Forrest et al., "Impact of rapid in situ hybridization testing on coagulase-negative staphylococci positive blood cultures", Journal ofAntimicrobial Chemotherapy, 58: 154-158 (2006).

As used herein *"second rRNA-directed probe or probes"* refers to a second probe or probes that selectively binds to a different (i.e. second) target or targets within a molecule or molecules of rRNA. The second rRNA target may exist in the same bacteria as did another (first) probe or probes used in the assay but more typically the second rRNA-directed probe or probes will be directed to a target in a different bacteria of interest such that formation and determination of a second probe/rRNA complex or complexes is used to determine a second (different) select bacteria in the sample. The second select bacteria can be a gram-positive bacteria or a gram-negative bacteria.

As used herein *"select gram-positive bacteria"* refers to bacteria of interest (e.g. a gram-positive bacteria sought to be determined by practice of a method disclosed herein) that can be determined by practice of a method described herein. Typically, the gram-positive bacteria are selected for analysis because said bacteria may possess a select trait. For example, the select trait may be of clinical significance. The select gram-positive bacteria may be, for example, of a particular species, subspecies, or genus. The select gram-positive bacteria may, for example, also be a recognized group such as coagulase-negative staphylococci (CNS) or gram-positive cocci. In some embodiments, the select gram-positive bacteria are *S*. *aureus* and the select trait is methicillin-resistance.

As used herein *"select trait"* refers to a trait of interest to be determined by practice of a method described herein. In some embodiments, the select trait is methicillin-resistance.

As used herein *"signal amplification"* is discussed with respect to a label or labels associated (directly or indirectly) with a probe and refers to use of specific detection methodologies to increase the signal by a factor of at least two for each label associated with the probe. Signal amplification often (but not necessarily) involves the use of enzymes. Some non-limiting examples of signal amplification include tyramide signal amplification (TSA, also known as catalyzed reporter deposition (CARD)), Enzyme Labeled Fluorescence (ELF-97 - product and information available from Invitrogen, Carlsbad, CA), Branched DNA (bDNA) Signal Amplification (See: Collins et al., "A branched DNA signal amplification assay for quantification of nucleic acid targets below 100 molecules/ml", Nucl. Acids Res., 25(15): 2979-2984 (1997) and Zheng et al., "Direct mecA Detection from Blood Culture Bottles by Branched-DNA Signal Amplification", J. Clin. Microbiol., 37(12): 4192-4193 (1999)), and rolling-circle amplification (RCA - See: Maruyama et al., "Visualization and Enumeration of Bacteria Carrying a Specific Gene Sequence by In Situ Rolling Circle Amplification", Applied and Environmental Microbiology, 71(12): 7933-7940 (Dec. 2005) and Smolin et al., "Detection of Low-Copy-Number Genomic DNA Sequences in Individual Bacterial Cells by Using Peptide Nucleic Acid-Assisted Rolling-Circle Amplification and Fluorescence In Situ Hybridization", Applied and Environmental Microbiology, 73(7): 2324-2328 (2007)).

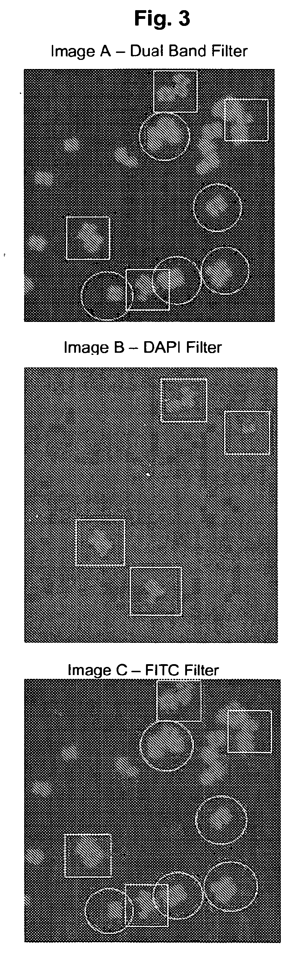

As used herein *"stained"* means that a bacterial cell is directly or indirectly marked for detection with a label or labels. For example, the bacteria can be stained with one or more fluorescently labeled hybridization probes such that the bacterial cell or cells can, for example, be detected using a fluorescent microscope as described in US 6,664,045 (See in particular Figs. 3 (of US 6,664,045) and the discussion associated therewith in Example 10 at col. 24-25). As is apparent in the various panels of Fig. 3 of US 6,664,045, different bacteria of a sample can be stained with independently detectable labels (or combinations of independently labels) such that different types of bacteria in the sample appear, for example, as different colors (or otherwise possess differing detectable properties). With specific reference to Fig. 3 of US 6,664,045 for example, *S*. *aureus* bacteria are characterized as stained red (only), *E*. *coli are* characterized as stained green and red, *P. aeruginosa* are characterized as stained green (only) and *S*. *typimurium* are characterized as stained blue. Thus four different bacteria are determined in Fig. 3 of US 6,664,045 using different rRNA directed labeled probes whereby the probe for each different bacteria is labeled with a uniquely label or combination of labels. Figs. 2 and 3 of the present application also exhibit different types of bacteria which comprise unique independently detectable (fluorescent) stains.

As used herein, *"target"* or *"select target"* are interchangeable and refer to a molecule (or part of a molecule such as a select nucleic acid sequence) of a bacteria, such as a rRNA, mRNA, chromosomal DNA, plasmid DNA or an antigen, to which a probe is designed to specifically bind.

As used herein *"trait"* refers to any characteristic or property of bacteria that can be determined by analysis of the chromosomal DNA, mRNA and/or native plasmid DNA of said bacteria. An example of one such trait is methicillin-resistance. Said trait is dependent on the presence of the mecA gene (i.e. the chromosomal DNA) and expression of said gene (e.g. by production of mRNA from said gene).

As used herein, *"under conditions suitable for a [or "the"] probe to bind to a [or "the"] target"* refers to conditions under which a probe binds to its respective target in a specific manner such that non-specific binding of probe to non-target moieties is minimized or eliminated. It is also to be understood that *"the"* can be replaced by *"said"* as appropriate (above and anywhere else in this specification) to indicate/acknowledge antecedent basis.

As used herein, the phrase *"uniquely identifiable"* is used with reference to a situation where two or more conditions of interest are distinguishable. For example, in a sample comprising at least two bacteria, one bacteria may comprise red fluorescent markers and another bacteria may comprise green fluorescent markers. Accordingly, said two bacteria are *"uniquely identifiable"* (i.e. uniquely stained) using, for example, a properly equipped microscope (See for example: Figs. 3 of US 6,664,045 as discussed above) since the two bacteria can be distinguish using the microscope.

Bacteria may be uniquely identifiable for other reasons, such as morphology. For example one type of bacteria may be rod-shaped and the other a cocci. In some embodiments, color and morphology can be used to distinguish/determine uniquely identifiable bacteria in a sample.

As used herein, *"whole-celf'* refers to cells (e.g. bacteria) in a morphologically recognizable form. *"Whole-cell"* is not intended to imply that the cell comprises all of its original components as it is well-known that when cells are permeabilized they "leak" cellular constituents (See: Hoshino et al., Applied and Environmental Microbiology, 74(16): 5068-5077 (2008) at page 5074, col. 1 and Maruyama et al., Applied and Environmental Microbiology, 71(12): 7933-7940 (Dec. 2005) at page 7937, col. 1). Such "leakage" is not intended to infer that an assay performed with cells that have leaked is not a whole-cell assay as discussed herein. Rather, *"whole-cell"* is intended to refer to substantially intact cells such that they retain their morphologically recognizable form. For example, cocci are spherical whereas other bacteria can be rod-like.

As used herein, *"within bacteria"* or *"within the bacteria"* refers to inside of any structure (including multiple structures) of whole (intact) bacteria, such as the outer membrane, nuclear membrane, cell wall, cytoplasm and/or nucleus. For example, formation of one or more probe/rRNA complexes within bacteria of the sample can refer to formation of one or more probe/rRNA complexes inside of the outer membrane, nuclear membrane, cell wall and/or nucleus of said bacteria. Similarly, the probe/rRNA complex(es) can form in the cytoplasm and their presence can be used to determine the select gram-positive bacteria (e.g. staphylococcus aureus) from other bacteria in a sample (See for example: Fig. 3 and the discussion of Fig. 3 in Example 3). However, as used herein, *"within bacteria"* is also, as appropriate, intended to encompass structures in contact with the outer surface of intact bacteria. For example, *"within bacteria"* is also intended to encompass, for example, antibody probes linked to the outer surface of a bacterium (for example as a consequence of binding to a surface protein), wherein said antibody probes, for example, are used to determine select bacteria in a sample.

### 4. General

*It is to be understood that the discussion set forth below in this "General" section can pertain to some, or to all, of the various embodiments of the invention described herein.*

### Synthesis, Modification and Labeling of Nucleic Acids and Nucleic Acid Analogs

Nucleic acid oligomer (oligonucleotide and oligoribonucleotide) synthesis has become routine. For a detailed description of nucleic acid synthesis please see Gait, M. J., "Oligonucleotide Synthesis: a Practical Approach" IRL Press, Oxford England (1984). Persons of ordinary skill in the art will recognize that labeled and unlabeled oligonucleotides (DNA, RNA and synthetic analogues thereof) are readily available. They can be synthesized using commercially available instrumentation and reagents or they can be purchased from commercial vendors of custom manufactured oligonucleotides.

### PNA Synthesis and Labeling

Methods for the chemical assembly of PNAs are well-known (See: U.S. Pat. Nos. 5,539,082, 5,527,675, 5,623,049, 5,714,331, 5,718,262, 5,736,336, 5,773,571, 5,766,855, 5,786,461, 5,837,459, 5,891,625, 5,972,610, 5,986,053 and 6,107,470. Some non-limiting methods for labeling PNAs are described in U.S. Pat. No. 6,110,676, WO99/22018, WO99/21881, WO99/4929 and WO99/37670 are otherwise well known in the art of PNA synthesis. Chemicals and instrumentation for the support bound automated chemical assembly of peptide nucleic acids are commercially available. Likewise, labeled and unlabeled PNA oligomers are available from commercial vendors of custom PNA oligomers (See: See the worldwide web at: panagene.com/pna-oligomers.php, See the worldwide web at: biosyn.com/pna_custom.aspx or See the worldwide web at: crbdiscovery.com/pna/). Additional information on PNA synthesis and labeling can be found in Peter E. Nielsen, "Peptide Nucleic Acids", Taylor and Francis, (2004).

Because a PNA is a polyamide, it has a C-terminus (carboxyl terminus) and an N-terminus (amino terminus). For the purposes of the design of a hybridization probe suitable for antiparallel binding to a target (the preferred orientation), the N-terminus of the PNA oligomer is the equivalent of the 5'-hydroxyl terminus of an equivalent DNA or RNA oligonucleotide.

### Chimera Synthesis and Labeling/Modification

Chimeras are oligomers comprising subunits of different monomer types. In general, it is possible to use labeling techniques (with or without adaptation) applicable to the monomer types used to construct the chimera. Various labeled and unlabeled chimeric molecules are reported in the scientific literature or available from commercial sources (See: US 6,316,230, See the worldwide web at: biosyn.com/PNA_ Synthesis.aspx, WO2001/027326 and See the worldwide web at: sigmaaldrich.com/ life-science/custom-oligos/dna-probes/product-lines/Ina-probes.html). Therefore, persons of skill in the art can either prepare labeled chimeric molecules or purchase them from readily available sources.

### Labels

Non-limiting examples of labels (i.e. detectable moieties or markers) suitable for labeling probes used in the practice of this invention include a chromophore, a fluorophore, a spin label, a radioisotope, an enzyme, a hapten, a chemiluminescent compound, a quantum dot or combinations of two or more of the foregoing.

Some examples of haptens include 5(6)-carboxyfluorescein, 2,4-dinitrophenyl, digoxigenin, and biotin.

Some examples of fluorochromes (fluorophores) include 5(6)-carboxyfluorescein (Flu), 6-((7-amino-4-methylcoumarin-3-acetyl)amino)hexanoic acid (Cou), 5(and 6)-carboxy-X-rhodamine (Rox), Cyanine 2 (Cy2) Dye, Cyanine 3 (Cy3) Dye, Cyanine 3.5 (Cy3.5) Dye, Cyanine 5 (Cy5) Dye, Cyanine 5.5 (Cy5.5) Dye Cyanine 7 (Cy7) Dye, Cyanine 9 (Cy9) Dye (Cyanine dyes 2, 3, 3.5, 5 and 5.5 are available as NHS esters from GE Healthcare, Life Sciences, Piscataway, NJ), JOE, Tamara or the Alexa dye series (Life Technologies, Carlsbad, CA).

Some examples of enzymes include polymerases (e.g. Taq polymerase, Klenow PNA polymerase, T7 DNA polymerase, Sequenase, DNA polymerase 1 and phi29 polymerase), alkaline phosphatase (AP), horseradish peroxidase (HRP) and soy bean peroxidase (SBP).

Some examples of radioisotopes include ¹⁴C, ³²P, ¹²⁹I and ⁹⁹Tc.

In some embodiments, spin labels can be used as labels. Spin labels are organic molecules which possess an unpaired electron spin, usually on a nitrogen atom. For example, probes can be labeled with a spin label as described in US Patent application No. 7,494,776. Said labeled probe can then, for example, be used to stain bacteria for determination.

### Independently Detectable Labels/Multiplex Analysis

In some embodiments, a multiplex method (assay) is performed. In a multiplex assay, numerous conditions of interest are simultaneously or sequentially examined. Multiplex analysis relies on the ability to sort sample components or the data associated therewith, during or after the assay is completed. A multiplex assay (as used herein), commonly relies on use of two or more uniquely identifiable probes.

In a multiplex assay, one or more distinct independently detectable labels (typically each distinct label (or a distinct combination of labels) is linked to a different probe) are used to uniquely mark (i.e. stain) two or more different bacteria of interest. In some cases, two (or more) unique labels may be directed to the same bacteria thereby generating a unique stain that results from the presence of the two (or more) unique labels in the bacteria. The ability to differentiate between and/or quantify each of the uniquely stained bacteria provides the means to multiplex the assay because the data that correlates with each uniquely marked (i.e. stained) bacteria can be correlated with a condition or conditions sought to be determined (e.g. select bacteria or select trait).

In practicing methods described herein, it is possible to uniquely mark bacteria so that two (or more) conditions of interest can be determined for the bacteria of the sample. For example, in the practice of some embodiments, it is possible to use a unique label to mark S. aureus bacteria in a sample as well as use a unique label to mark bacteria in the sample that are methicillin-resistant. Thus, by analysis of the sample it is possible to determine whether the sample contains: 1) S. aureus bacteria (that are not methicillin-resistant); 2) non-S. *aureus* methicillin-resistant bacteria (e.g. MR-CNS); and/or 3) methicillin-resistant S. *aureus* bacteria. In some embodiments, the sample can be characterized as heterogeneous or homogeneous for these three conditions. In some embodiments, the number of bacteria in each group can be estimated, quantified or identified as representing a particular percentage of the bacteria of the sample.

Methods can be multiplexed in many ways and multiplexing is limited only by the number of independently detectable labels (or independently detectable probes) that can be used or detected in an assay. For example, some assays may be designed to detect and identify the presence of several (e.g. two, three, four, five, six or more) different bacteria (in some embodiments all gram-positive and in some embodiments mixtures of gram-positive and gram-negative bacteria) in a sample and also determine whether any of those bacteria possess one or both of two (or more) different traits of interest. For example, a multiplex assay for five bacteria and two traits would require at least 7 (5 + 2) uniquely labeled probes (or 7 unique combinations of labels) and the ability to differentiate at least 10 (5 x 2) or as many as 20 (5 x 4) possible different types of stained bacteria. Put in the context of an embodiment of the present invention, the method could use 5 uniquely labeled rRNA-directed probes to determine each of the five different bacteria and 2 uniquely labeled mRNA-directed labeled probes to determine each different trait.

Some representative multiplex assays are described in Example 3 and the uniquely identifiable properties of representative bacteria are visible with reference to Fig. 3.

### Whole-Cell Assays:

Methods disclosed herein involve whole-cell assays. Whole-cell assays are performed on intact or substantially intact cells. Some examples of whole-cell assays are in-situ hybridization (ISH), fluorescence in-situ hybridization (FISH) and immunocytochemistry (ICC) assays. In some embodiments, a whole-cell assay is not strictly an ISH, FISH or ICC assay. For example, whole-cell assays may involve a combination of two or more of these different assay formats (See: Goldbard et al., US Pat. No. 6,524,798 entitled: *"High Efficiency Methods For Combined Immunocytochemistry And In-Situ Hybridization").* More specifically, some embodiments of this invention contemplate use of oligomer (hybridization) probes used in combination with, for example, antibody probes. To the extent that the assay formats and/or components used in said assays are not mutually incompatible, this invention contemplates any combination of combined whole-cell assay formats. As discussed in more detail below, combining the assays may involve some degree of harmonization of the binding conditions where different probe types are used in the practice of a method step. Alternatively, reprobe cycling of the sample may also be used wherein conditions are fixed for one probe type such that the reprobing cycle (the first cycle would actually be a probing cycle) is completed with said probe type and a new reprobing cycle is performed with the second (different) probe type (See Williams et al., US Pat. No. 2005/0123959 for a discussion of whole-cell analysis using sequential steps of analysis - as used herein "reprobing cycle or reprobing cycles"). Depending on the method, probe/target complexes can be determined after each reprobing cycle, after some of the reprobing cycles or after all of the reprobing cycles.

### ISH:

As used herein "in situ hybridization (ISH)" refers to methods practiced using a hybridization probe directed to a nucleic acid target. The probe may be a nucleic acid (e.g. RNA, DNA), a nucleic acid analog (e.g. LNA), a nucleic acid mimic such as PNA, morpholino or PP or a chimera (e.g., a DNA-RNA chimera, PNA-DNA chimera, a PNA-RNA chimera, a LNA-DNA chimera, etc.). The most widely used ISH method is "fluorescence in situ hybridization" or "FISH", in which the probe comprises one or more fluorescent labels.

Briefly, conventional in situ hybridization assays generally comprises one or more of the following steps: (1) prehybridization treatment of the cell to increase accessibility of target DNA or RNA (e.g., denaturation with heat or alkali and/or treatment with a cell permeabilization reagent or reagents); (2) steps to reduce nonspecific binding (e.g., by blocking the hybridization capacity of repetitive sequences, e.g., using human genomic DNA); (3) pre-hybridization involving contacting the sample with hybridization solution not containing the hybridization probe; (4) hybridization of one or more hybridization probes to the nucleic acid within the bacteria; (5) washes to remove probes not bound to their respective targets; and (6) detection/determination of the probe/target complexes (e.g. by determining the stained bacteria). The reagents used in each of these steps and conditions for their use vary depending on the particular application.

ISH may be carried out using a variety of detectable or detectably labeled probes (e.g., ³⁵S-labeled probes, fluorescently labeled probes, enzyme labeled probes) capable of hybridizing to a cellular nucleic acid sequence. When fluorescently labeled probes are used, the technique is called FISH. The ISH probes may be labeled directly (e.g., by use of a covalently linked fluorescent-label) or indirectly (e.g., through a ligand-labeled antiligand system).

### Immunocytochemistry (ICC):

As used herein, immunocytochemistry refers to the use of antibody or antibody fragments to stain bacteria of a sample through the interaction of an antibody probe (or antibody fragment probe) and an antigen within bacteria. The staining may occur by use of only primary antibodies or it may involve the use of (labeled) secondary antibodies. For the avoidance of any doubt however, this invention does not pertain to the use of a primary antibody directed to a protein antigen within bacteria, wherein the protein antigen is associated with a select trait and wherein the determining said antibody/antigen complex is used to determine said select trait.

As used herein, immunocytochemistry (to the extent that it is used) will commonly be directed to determining select bacteria in a sample. Hence, the antibody (or antibody fragment) probe can be directed to an antigen target that is specific for the select bacteria. The antibody probe can be labeled (i.e. direct detection) or the antibody probe/antigen target complex formed by the binding of the antibody probe to its respective antigen target within the bacteria can be determined by use of labeled secondary antibody that binds to said antibody probe/antigen target complex (i.e. indirect detection).

Notwithstanding the foregoing, immunocytochemistry can be used to determine traits within bacteria. However, with respect to the determination of traits, the antibody probe(s) is/are directed to the probe/target complexes formed by the binding of the chromosomal DNA, mRNA or native plasmid-directed probe or probes to their respective target(s) within the bacteria. Hence, in this context, the immunocytochemistry is used for indirect staining of said probe/target complexes associated with the trait(s) of the bacteria.

No matter what is being targeted, at least one antibody is labeled with at least one detectable moiety such that when said labeled antibody binds, the bacteria is stained. Moreover, ICC can be combined with ISH or FISH procedures to thereby determine select bacteria and/or select traits according to the methods disclosed herein.

### Samples:

Bacteria are everywhere. A sample comprising bacteria can come from any source. The source of a sample is not intended to be a limitation associated with the practice of any method disclosed herein.

Samples can be environmental samples such as samples from soil or water. Samples can come from consumer staples such as food, beverages or cosmetics. Samples can come from crime scenes (e.g. for forensic analysis). Samples can come from war zones or from sites of a suspected terrorist attack (For example, for testing of pathogenic bacteria, including weaponized bacteria (e.g. *B. anthracis*). Samples can come from clinical sources. Samples from clinical sources can come from any source such as a human, a plant, a fish or an animal. Some non-limiting examples of clinical samples (from clinical sources) include blood, pus, sputum, spinal fluid, amniotic fluid, stool, urine, nasal swabs, throat swabs and the like. Samples (including clinical samples) can include bacterial cultures and subcultures, or portions thereof. Samples can include samples prepared, or partially prepared, for a particular analysis. For example, the sample may be a specimen that has been fixed and/or stored for a period of time.

### Probes:

Unless expressly limited by specific language or discussion herein, any PNA probe that can be used to select for a desired condition of interest (e.g. select bacteria or select trait) based on selective binding of said probe to its respective target can be used in the practice of embodiments of this invention. In some embodiments, the probe or probes is/are 10 to 20 nucleobase subunits in length. Probes are described herein in terms of "nucleobase subunits in length" since only nucleic acids comprise nucleotides whereas all of these different oligomer types comprise one nucleobase per subunit. Probes used in embodiments of this invention can be prepared by denovo synthesis or by other methods.

It is to be understood that numerous probes exist in the biological arts for detecting specific bacteria or traits. Consequently, the nature of the probe (for purposes of this invention) is not intended to be limited except as expressly disclosed herein.

In some embodiments, probes used herein can be unlabeled provided that there is an available mechanism for determining the probe/target complex formed by binding of the probe to its respective target. For example, an unlabeled (primary) antibody-based probe can be determined by use of a secondary detectably labeled antibody that binds to said unlabeled (primary) antibody-based probe (See for example: US 6,524,798 at col. 3, lines 28-40 and US 7,455,985 at col. 12, lines 12-63). For example, said unlabeled (primary) antibody-based probe may be used to determine the select gram-positive bacteria. Thus, the complex (i.e. labeled secondary antibody/primary antibody/target complex) formed upon binding of all molecules can be determined (and hence the select bacteria) by determining said label of said secondary antibody. Other types of unlabeled probes can similarly be determined by use of a labeled molecule that selectively binds to said unlabeled probe or the complex formed by binding of said unlabeled probe to its respective target (See for example: US Pat. No. 5,612,458 to Hyldig-Nielsen which discusses the use of antibodies to PNA-DNA complexes, etc).

Probes can be labeled with at least one detectable moiety (i.e. at least one label). In some embodiments, each probe will comprise only one label. In some embodiments, the probes used to determine the select trait (e.g. methicillin-resistance) will comprise only one label. In the methods of the invention, mixtures of PNA probes (e.g. mixtures of mRNA-directed probes) are used wherein each probe comprises one label or two labels (i.e. a mixture of single labeled and/or dual labeled probes). In some embodiments, one or more probes may comprise a single label and one or more probes may comprise multiple labels. In some embodiments, one or more of the probes can be unlabeled and one or more probes may comprise one or more labels.

In some embodiments, the label or labels can be determined directly. In some embodiments, the label or labels can be determined indirectly. In some embodiments, some of the labels can be determined directly and some determined indirectly.

Determining a label directly involves determining a property of the label without use of another molecule/compound. For example, determining a fluorescent label may involve viewing a treated sample using a fluorescent microscope, using a slide scanner or using a flow cytometer. Because it is the fluorescence of the label itself that is being observed/measured in the microscope, scanner or cytometer, the determination is said to be direct.

By comparison, indirect determination involves use of an ancillary molecule/compound that recognizes the label of the labeled probes whereby the ancillary molecule/compound (or a label thereon) is determined as a surrogate for determining the label of the labeled probe. For example, the label can be a hapten like digoxigenin. Several of the references listed in Section 8 below describe indirect methods for determining digoxigenin. In general, these method involve the use of an anti-digoxigenin molecule (antibody) conjugated to a secondary label (e.g. an enzyme like horseradish peroxidase, alkaline phosphatase or a fluorophore like fluorescein). Because it is the properties of the secondary label of the ancillary molecule (i.e. the anti-digoxigenin molecule) that is determined, this is characterized as an indirect detection method.

In practice, some probes used in embodiments of the present invention are chosen to determine a select bacteria in a sample. We refer to these as a [or "the"] *"bacteria-directed"* probe or probes. By *"bacteria-direcfed"* we refer to a probe or probes that find with specificity to a target within a bacteria, select bacteria or select gram-positive bacteria. Moreover, said bacteria-directed probe or probes are said to be *"capable of determining a [or "the"] select bacteria in a [or "the"] sample"* because said bacteria-directed probe or probes selectively bind to a target within the bacteria so that said select bacteria can be determined (for example by fluorescence microscopy or flow cytometry) based on formation of the probe/target complex. Thus, said bacteria-directed probe or probes are used for determining said select bacteria in said sample.

In the methods of the present invention, the select bacteria is a select gram-positive bacteria (e.g. *S. aureus*) and said bacteria-directed probe or probes are said to be *"capable of determining a [or "the"] select gram-positive bacteria in a [or "the"] sample"* or more specifically for staphylococcus *aureus; "capable of determining Staphylococcus aureus bacteria in a [or "the"] sample".* In some embodiments, other select bacteria (including as appropriate one or more gram-negative bacteria) may also be selected for determination. In this case, the sample is also contacted with one or more additional bacteria-directed probes for each additional select bacteria sought to be determined by practice of the method. Often, the determination of multiple select bacteria in a sample is accomplished by use of a multiplex assay wherein each different type of bacteria is stained with a unique stain, combination of stains and/or unique combination of stain and cell morphology.

The probe or probes chosen to determine a select bacteria (i.e. the bacteria-directed probe or probes) can be a rRNA-directed probe or probes. Said rRNA-directed probe or probes bind with specificity to a target in the rRNA of the select bacteria. However, the bacteria-directed probe or probes need not be rRNA-directed. Rather, they may, for example, be mRNA-directed. By *"mRNA-directed"we* refer to a probe or probes that bind with specificity to a target in mRNA. The bacteria-directed probe or probes may also be directed to other regulatory RNAs (e.g. small RNA (sRNA) or antisense RNA (aRNA)) that are specific to said bacteria.

Moreover, the bacteria-directed probe or probes need not be hybridization probes. For example, the bacteria-directed probe or probes can be, for example, antibody-based (See for example: US 6,231,857 and US 7,455,985) since it is known that antibodies can be used to distinguish one type of bacteria from another or others.

In the methods of the present invention, the probes chosen to determine a select trait are directed to a target or targets: 1) within the chromosomal DNA; 2) within the mRNA; and/or 3) within a native plasmid of a bacteria that may be present in the sample, wherein said target or targets are associated with the select trait. Therefore, said probes are said to be *"chromosomal DNA-, mRNA-* and/or *native plasmid-directed"* based on the nature of the target or targets. Furthermore, said chromosomal DNA-, mRNA- and/or native plasmid-directed probes are said to be: "capable of *determining chromosomal DNA, mRNA and*/*or plasmid nucleic acid associated with a [or* "the"] *select trait"* because said chromosomal DNA-, mRNA- and/or native plasmid-directed probe or probes selectively bind to a respective target or targets associated with said select trait. Thus, said chromosomal DNA-, mRNA- and/or native plasmid-directed probes are used for determining said select trait of bacteria of said sample. In some embodiments, the select trait is methicillin-resistance.

It is to be understood however that said chromosomal DNA-, mRNA-and/or native plasmid-directed probe or probes are not directed to [binding to] a target protein associated with the trait. Rather, the target or targets for said chromosomal DNA-, mRNA- and/or native plasmid-directed probe or probes typically lie/lies within the nucleic acid sequence of the chromosomal DNA, mRNA and/or DNA of the native plasmid.

As inferred from the language above, there is no requirement that the probe or probes used to determine a select trait be directed to all of: 1) chromosomal DNA; 2) mRNA; and 3) native plasmid. Rather, the probe or probes used to determine a select trait can be directed to only one of, or any combination of two or more of: 1) chromosomal DNA; 2) mRNA; and 3) native plasmid. For example, in some embodiments, the probe or probes used to determine the select trait are chromosomal DNA and/or mRNA-directed. In some embodiments, the probe or probes used to determine the select trait are mRNA-directed.

Moreover, in some embodiments, the methods described herein can be practiced in multiplex mode whereby multiple traits (e.g. two traits, three traits, four traits, etc.) are being determined for bacteria of a single sample. There is no requirement that the probe or probes for different traits be directed to the same target type. Although it is permissible that the probe or probes for different traits are directed to the same target type (e.g. one of 1) chromosomal DNA; 2) mRNA; or 3) native plasmid), it is also permissible that probes for different traits are directed to different target types. It is also permissible that some probes for different traits are directed to the same target type and some probes for different traits are directed to different target types in the same assay. Indeed any possible combination of probes for different target types is permissible.

The chromosomal DNA-, mRNA- and/or native plasmid-directed probe used in the methods of the present invention is a PNA probe. The chromosomal DNA-, mRNA- and/or native plasmid-directed probe or probes can be unlabeled. Probe/target complexes formed using unlabeled probes can be determined as previously described herein. However, in the present invention, the chromosomal DNA-, mRNA- and/or native plasmid-directed probe or probes are labeled with one or two labels. In some embodiments, each chromosomal DNA-, mRNA- and/or native plasmid-directed probe will comprise only one label. In some embodiments, each chromosomal DNA-, mRNA-and/or native plasmid-directed probe can comprise multiple labels. It is also permissible to mix single labeled probes and multi-labeled probes in the same assay.

As noted several times previously, the methods described herein can be practiced in multiplex mode whereby, for example; 1) two or more select bacteria are determined in a single sample; 2) two or more select traits are determined in a single sample; or 3) two or more select bacteria and two or more select traits are determined in a single sample. In general, such multiplex assays are performed by contacting the sample with additional probes as needed to determine the additional select bacteria and/or select trait(s). In some embodiments, said contacting can be done simultaneously so that all the different bacteria and/or traits can be determined at the end of a single procedure. For this embodiment, the probe or probes directed to each different select bacteria and/or different select trait can be independently detectable. In general, the labels of the various probes used in practice of the method are selected to produce different stained bacteria based on the type of bacteria and/or trait(s). In some cases however, it will be possible to have some identically stained bacteria, whereby one or more of the select bacteria and/or traits is determined based on morphology of the bacteria (possibly in combination with a determination of the stain).

Rather than multiplex with different (independently detectable) labels (or uniquely stained bacteria), it is also possible to get multiple results by use of a reprobe cycling method (See: Published US Pat. Application No. 2005/0123959 to Williams et al.). In a reprobe cycling method, a result is obtained and then the sample is reanalyzed for determining a second, third, fourth, fifth, etc. result. Typically, in a reprobe cycling method, the prior result is removed (erased) before the sample is treated to obtain the next result.

With respect to the methods disclosed herein, it is possible to use the same label type (e.g. fluorescein) to determine two or more select bacteria and/or two or more select traits by use of a reprobe cycling method. In some embodiments, it is possible to determine a select bacteria and a select trait in the same reprobing cycle. In some embodiments it is possible to determine a select bacteria and a select trait in a different reprobing cycle. In general, a person of skill in the art can select which select bacteria and/or select trait(s) are to be determined in a particular reprobing cycle by selection of the probe or probes applied to the sample during said reprobing cycle.

### Targets:

In general, a target can be any target molecule (or a portion thereof) that is present within the bacteria (or yeast) during the whole-cell assay that can be determined using a respective probe. Some non-limiting examples of targets include nucleic acid sequences present (e.g. select sequences within rRNA, mRNA, chromosomal DNA or plasmid DNA) within any nucleic acid of the bacteria, an antigen, an antibody, a protein, a peptide and/or a hormone.

The methods of the present invention are practiced with: 1) a bacteria-directed probe or probes capable of determining a select gram-positive bacteria that may be present in a sample; and 2) a trait-directed probe or probes capable of determining chromosomal DNA, mRNA and/or native plasmid associated with a select trait that may be present in a bacteria in the sample (which trait may or may not be present in said select gram-positive bacteria). For the avoidance of doubt however, this invention is not directed to use of a target that is a protein for determining a trait.

It is to be understood that the methods disclosed herein can be used to determine additional target(s) (for example by multiplexing or reprobe cycling) that might be of interest in a sample and determined during practice of the methods disclosed herein. For example, it is possible to obtain additional information from the sample by contacting said sample with one or more additional probes directed to said additional target(s) whose presence within bacteria (or yeast) of the sample is indicative of an another condition of interest (for example another condition of clinical interest for proper diagnosis of a patient). Said additional condition of interest may be the presence of another bacteria (which bacteria may be gram-positive or gram-negative) in the sample. Said additional condition of interest may be the presence of yeast in the sample. Said additional condition of interest may be the presence of a plasmid in the select gram-positive bacteria and/or in other bacteria of the sample. Said additional condition of interest may be the presence of another trait or traits in bacteria (including the select gram-positive bacteria) of the sample. The method disclosed herein can be used in combination with numerous probes for numerous targets. Accordingly, it is possible by practice of methods disclosed herein to determine one or more additional conditions of interest based on a proper selection of targets (and the respective probe or probes for each target) which may, for example, include determining: 1) additional bacteria; 2) plasmids; 3) yeast; 4) traits; or 5) any possible combination of two or more of the foregoing.

Persons of skill in the art will be able to design select suitable targets (and design appropriate probes to said suitable targets) using routine experimentation and commercially available materials and/or information. For example, ISH is commonly used to determine select bacteria (See: Amann, R., "Methodological Aspects of Fluorescence In Situ Hybridization", Bioscience Microflora, 19(2): 85-91 (2000) and Pernthaler et al., "Fluorescence in situ Hybridization (FISH) with rRNA-targeted Oligonucleotide Probes", Methods in Microbiology, 30: 207-226 (2001)) including staphylococcus aureus bacteria (See: US Pat. No. 6,664,045 at Fig. 3 and US Pat. Application No. 2008/0008994; Cerqueira et al., "DNA Mimics for the Rapid Identification of Microorganisms by Fluorescence in situ Hybridization (FISH)", Int. J. Mol. Sci., 9: 1944-1960 (2008); and Forrest et al., "Impact of rapid in situ hybridization testing on coagulase-negative staphylococci positive blood cultures", Journal of Antimicrobial Chemotherapy, 58: 154-158 (2006)). As previously noted (See: the section above entitled "Probes"), targets for such determinations can, for example, be rRNA. This is not intended to be a limitation however, as the target for selecting a bacteria can, for example, be a surface antigen (See: US 7,455,985).

### Forming Probe/Target Complexes:

The select bacteria (e.g. the select gram-positive bacteria) and select trait are determined by determining formation of the appropriate probe/target complexes within the bacteria of the sample. In brief, by contacting the sample with probes chosen for their affinity for their respective targets known to be associated with (and specific for) the select bacteria and/or trait, the appropriate probe/target complexes will form within the bacteria of the sample.

The nature of the probe/target complex is determined by the nature of the probe and its respective target. Various types of probe/target complexes are contemplated. For example, hybridization probes for bacteria determination can be rRNA-directed or mRNA-directed. Thus, each complex formed upon binding of the probe to its target is a probe/rRNA complex or probe/mRNA complex, respectively.

Similarly, hybridization probes for trait determination can be chromosome DNA-directed, mRNA-directed or native plasmid-directed. Hence, each complex formed upon binding of the probe to its respective target is a probe/chromosome DNA complex, probe/mRNA complex or probe/plasmid complex, respectively.

With respect to antibody probes, binding of the antibody to its antigen target produces an antibody/antigen complex.

Those of skill in the art will recognize that the probe/target complexes in the bacteria are formed under suitable binding conditions (or more correctly termed "suitable hybridization conditions" for hybridization probes). Suitable binding conditions for each probe/target complex will be determined based on the nature of the probe and target. It suffices to say that suitable binding conditions are reflected in conditions where the interactions of the probe and its respective target are specific. Moreover, persons of ordinary skill in the art can determine suitable binding conditions for forming many types of probe/target complexes. Indeed, numerous hybridization buffers (See for example: a ready-to-use hybridization solution optimized for in situ hybridization procedures such as: See the worldwide web at: sigmaaldrich.com/catalog/ProductDetail.do?N4=H77821SIGMA&N5=SEARCH_CONCAT_PNO|BRAND_KEY&F= SPEC) and/or binding buffers (See for Example; commercially available ready-to-use antibody binding buffers from ThermoScientific as described at: See the worldwide web at: piercenet.com/ Products/Browse.cfm?fldlD=01010401&WT.mc_id=go_AbPur _Bind_pf&gclid=CNyJ-4uxgJoCFdxM5Qod1Vt5Fw) are commercially available for use in various assay formats. It is to be understood that binding conditions need not be completely optimized but rather that the conditions merely be suitable for specific binding of the probe to its respective target such that the assay produces accurate and reproducible result. Moreover, where different types of probes (e.g. hybridization probes and antibody-based probes) are used in the same contacting step, binding conditions should be suitable for the binding of each type of probe to its respective target. For a more detailed discussion of this issue see the section below entitled: "Harmonizing Binding Conditions In Whole-Cell Assays".

### Determining Probe/Target Complexes:

Once formed, the probe/target complexes can be determined. The probe/target complexes can be determined using a label associated with each different (or different type of) probe/target complex. In some embodiments, all labels associated with different (or different types of) probe/target complexes are the same. In some embodiments, different labels (or combinations of labels) are associated with each different (or different type of) probe/target complex. In some embodiments, there is a mixture of the same label associated with some of the different (or different types of) probe/target complexes (e.g. the bacteria-directed probes where cell morphology can be used to distinguish between bacteria species) and different labels associated with others of the different (or different types of) of probe/target complexes (e.g. probes used to determine different traits).

A probe/target complex can be determined directly or indirectly. By *"directly",* we mean that the probe of the probe/target complex comprises a linked label which label is determined based on its own properties (See the discussion pertaining to determining direct and indirect determination of labels above in the section entitled: "Probes"). By *"indirectly',* we mean that the probe/target complex is determined using a secondary composition (e.g. a labeled antibody) that comprises a label and that binds to (or interacts with) the probe/target complex (or a label linked to the probe/target complexes), wherein said label is determined (*Id**.***) as indicia of the probe/target complex. Regardless, determining the label correlates with determining the probe/target complex.

In whole-cell assays, determining the probe/target complexes can, in some embodiments, be performed by examining how the cells (i.e. the bacteria) are stained. In brief, regardless of whether the labeling is direct or indirect, the cells become stained because the label(s) associated (directly or indirectly) with the probe/target complex or complexes is/are assimilated within (or at least on the surface of) the intact cells (i.e. bacteria). As noted previously, it is possible to use unique labels and/or unique combinations of labels for different bacteria and/or traits. Thus, any method capable of determining the stained bacteria in the sample can be used to determine the select bacteria and/or select traits.

For example, the select bacteria and/or traits can be determined based on their visual appearance under a microscope. In some embodiments, the process can be automated so that the result can be determined using a computer and algorithm.

In some embodiments, the select bacteria and/or traits can be determined using a slide-scanner. Similarly, a slide scanner can be automated so that the result can be determined using a computer and algorithm.

In some embodiments, the select bacteria and/or traits can be determined using a flow-cytometer. Likewise, a flow-cytometer can be automated so that the result can be determined using a computer and algorithm.

Moreover, any other instrument or method suitable for determining stained cells can be used to determine the probe/target complexes formed using the inventive methods disclosed herein.

### Cell Morphology:

It is an advantage of the present invention that various types of bacteria possess a unique morphology. In addition to the labels (e.g. stains) used to mark the bacteria, morphology of the cells can be used to either confirm the identity of bacteria or possibly introduce a second level of differentiation, for example, in a multiplex assay.

For example, bacilli tend to be rod-like whereas streptococci tend to be spherical (See the worldwide web at: en.wikipedia.org/wiki/Bacterial_cell_ structure#Cell_morphology and en.wikipedia.org/wiki/File:Bacterial_morphology _diagram.svg). In some assays, for example, it may be that determining a yellow stained rod-like cell will confirm the presence, location and/or quantity of a select gram-positive bacteria in the sample. In this case, the shape of the bacteria is used to (so to speak) distinguish signal (the select gram-positive bacteria) from noise (other bacteria) in the assay.

In some (e.g. multiplex) assays for example, multiple cell types may be used wherein at least two bacteria of different morphology are stained with, for example, a yellow marker. In this case, the presence, location and/or quantity of the two select bacteria can be determined based, for example, on whether or not they are stained yellow and are rod-like or spherical in shape. Of course an assay using this methodology can be further developed (further multiplexed) using bacteria of other known and distinguishable morphologies.

Associated with morphology (albeit not necessarily a strict example of cell morphology), in some embodiments characteristics of the staining process can also be used to confirm or determine a result. For example, where an antibody probe interacts with a surface antigen to stain the surface of the bacteria (e.g. use of an antibody based bacteria-directed probe) and a second, uniquely labeled target-directed (e.g. a mRNA-directed probe) interacts with a target inside of the bacteria (e.g. in the cytoplasm) to thereby stain the inside of the bacteria, a unique staining pattern can result. For example if the antibody probe is red and the target probe is green, when observed using microscope, the bacteria will appear as a red cover (or halo) surrounding a green body. Thus, bacteria of the sample are confirmed or determined based on whether or not they exhibit this particular staining pattern.

From the foregoing it is clear that cell-morphology (and staining patterns) is feature of the present invention that can be used in determining the select gram-positive bacteria or other select bacteria sought to be determined in any methods disclosed herein. By comparison, cell morphology is not available in cell-free assays since the bacteria are destroyed.

### Traits:

As defined above, a trait (for purposes of this invention) refers to any characteristic or attribute of bacteria that can be determined by analysis of the chromosomal DNA, mRNA and/or native plasmid of said bacteria. A "select trait" is a trait that is selected for determination in a particular assay. An assay may be designed to determine more than one select trait.

Because a trait is based on the genetic makeup of the bacteria, the bacteria are said to possess the trait (i.e. the characteristic or attribute) whether or not it is expressed (e.g. exhibited) by the bacteria (i.e. the trait is an inherent property). Thus, possession of the trait differs from expression of the trait in that bacteria can possess the trait but not exhibit the characteristic or property associated with the trait since expression refers to when the bacteria exhibits the characteristic or attribute (i.e. phenotype) associated with the trait. It is therefore clear that if a bacteria exhibits a trait, it possesses the trait (i.e. it contains the genetic material required to exhibit the trait) but that a bacteria can possess the trait without exhibiting the trait.

There are many bacterial traits that may be determined using the methods disclosed herein. Some non-limiting examples of traits that can be determined include: 1) antibiotic resistance; 2) toxin production; and/or 3) virulence. In some embodiments, examples of a trait or traits can be determined using targets in: 1) the mecA gene or vanA or vanB gene; 2) tcbD gene and/or 3) *Iuk*F and *Iuk*S gene of bacteria, respectively.

Because traits are associated with chromosomal DNA, mRNA and native plasmids, the target molecule(s) for some traits can be produced in very low copy number in the select bacteria. In the scientific literature related to gram-positive bacteria, this has typically been addressed by use of probes comprising multiple labels in combination with signal amplification of indirect labels (See for Example: Hahn et al., Applied and Environmental Microbiology, 59(8): 2753-2757 at page 2754, col. 2; Wagner et al., "In situ detection of a virulence factor mRNA and 16S rRNA in Listeria", FEMS Microbiol. Lett., 160(1): 159-168 (Mar. 1998); and Hönerlage et al., "Detection of mRNA of nprM in Bacillus megaterium ATTC 14581 grown in soil by whole-cell hybridization", Arch. Microbiol., 163: 235-241 (1995): But also see: Coleman et al., J. Microbiological Methods, 71: 246-255 (2007)) with respect to assays for **gram-negative bacteria**. In Coleman et al., a mRNA-directed probe comprising a single label was used provided that said label was a near-infrared fluorescent dye and long camera exposure times were employed). However, Applicants have found that is it possible to determine traits associated with, for example, the determination of low copy number mRNA targets in gram-positive bacteria in a whole-cell assay format by using only single labeled probes (wherein the label is not a near infrared fluorescent dye) and without the use of amplification techniques (e.g. signal amplification or nucleic acid amplification). In some cases, this has been accomplished with an associated induction of mRNA production in the (live) bacteria prior to performing the whole-cell assay (See more below under the heading: "Inducing mRNA Production").

### Specificity:

As noted above, probe/target complexes are formed under conditions that permit specificity of binding. Specificity of hybridization (i.e. the sequence specific binding of a hybridization probe to a nucleic acid target) is a function of various factors related to stringency and/or blocking strategy(ies). Specificity of binding also applies to antibody binding or the binding of members of any other type of ligand-ligand pair. Like hybridization specificity, specificity of binding of antibodies to antigens (or binding of one member of a ligand pair to another member) is also condition dependent. In principle, conditions are selected to optimize specificity so that non-specific binding is minimized or eliminated. Nevertheless, it is to be understood that specificity of binding is a relative term which also depends on many factors, including the nature (e.g. affinity) of the compositions forming the binding complex. Below is a non-limiting discussion of various conditions/considerations. Using no more than routine experimentation in combination with the disclosure provided herein, persons of skill in the art will be able to achieve suitable conditions so that binding (or hybridization) of specific probes to their respective targets is specific (such that practice of the method produces an accurate and reproducible result). In many cases, this can be accomplished using commercially available buffers.

### Blocking probes:

In hybridization reactions, blocking probes (made of nucleic acids, nucleic analogs, nucleic acid mimics or chimeras) can be used to suppress the binding of probes to a non-target and thereby improve specificity of the formation of probe/target complexes. Especially effective blocking probes are PNA oligomers (See: Coull et al., U.S. Pat. No. 6,110,676 and Fiandaca et al. "PNA Blocker Probes Enhance Specificity In Probe Assays", Peptide Nucleic Acids: Protocols and Applications, pp. 129-141, Horizon Scientific Press, Wymondham, UK, 1999)).

### Hybridization Conditions/Stringency:

Persons of ordinary skill in the art will recognize that factors commonly used to impose or control stringency of hybridization include formamide concentration (or other chemical denaturant reagent), salt concentration (i.e., ionic strength), hybridization temperature, detergent concentration, pH and the presence or absence of chaotropes. Blocking probes (See the section immediately above for a discussion of blocking probes) may also be used as a means to improve discrimination beyond the limits possible by mere optimization of stringency factors. Optimal stringency for forming a probe/target complex is often found by the well-known technique of fixing several of the aforementioned stringency factors and then determining the effect of varying a single stringency factor. The same stringency factors can be modulated to thereby control the stringency of hybridization of a nucleic acid mimic, nucleic acid analog or chimera to a nucleic acid target (e.g. a sequence within rRNA, mRNA or chromosomal DNA), except that for some of these modified oligomers (e.g. PNA) the hybridization may be fairly independent of ionic strength. Optimal or suitable stringency for an assay may be experimentally determined by examination of each stringency factor until the desired degree of discrimination is achieved. Nevertheless, optimal stringency is not required. Rather, all that is required is that the non-specific binding of probes to other than their respective targets is minimized in the assay to the extent necessary to achieve an accurate and reproducible result.

In the Examples provided below hybridization was performed using a hybridization buffer. As noted, various hybridization buffers are commercially available. Such buffers, in combination with temperature control, often provide suitable hybridization conditions.

As time to result can be an important factor particularly for clinical samples, the hybridization reactions performed in the examples provided below differ significantly from those of Hahn et al., Wagner et al. and Hönerlage et al., *inter alia,* in that they were performed in 2 hours rather than 5-16 hours (for the mRNA-directed probes).

### Suitable Antibody Binding Conditions

Suitable antibody binding conditions comprise conditions suitable for specifically binding an antibody to its antigen. Factors effecting antibody binding to its antigen (or for the binding of the ligands of a ligand-ligand complex) are substantially similar to those described above for hybridization and can be optimized in a similar manner. Suitable antibody binding conditions for various antibodies are known to persons of skill in the art. For those that are not, they can be determined. As noted above, suitable binding buffers are also commercially available.

Therefore, using the disclosure provided herein; with or without additional routine experimentation, persons of skill in the art can determine suitable antibody binding conditions. By way of additional general guidance to the practitioner, methods for preparing and using antibodies can be found in numerous references including: Molecular Probes Of The Nervous System, Volume 1, "Selected Methods For Antibody and Nucleic Acid Probes", Cold Spring Harbor Laboratory Press, 1993 by S. Hockfield et al.

### Harmonizing Binding Conditions In Whole-Cell Assays:

When practicing the methods disclosed herein, persons of skill in the art may find it useful to harmonize the hybridization conditions, antibody binding conditions and other assay conditions (e.g. conditions for ligand-ligand binding). For example, in some embodiments, the staining of cells with one or more hybridization probes may be performed simultaneously with, prior to, or subsequent to, an antibody binding event. Because adjustment of the same variables (pH, salt concentration etc.) is commonly involved, aided by no more than routine experimentation, those of skill in the art will easily be able to harmonize conditions so that the assay produces a satisfactory result. A discussion of some of the problems and related solutions for harmonizing conditions for using antibody probes and hybridization probes in a single assay can be found in Goldbard et al. (US 6,524,798) and Aßmus et al., "Improved In Situ Tracking of Rhizosphere Bacteria Using Dual Staining with Fluorescence-Labeled Antibodies and rRNA-Targeted Oligonucleotides", Microb. Ecol., 33: 32-40 (1997). It is also worth noting that the use of non-nucleic acid, and preferably PNA probes, can simplify the harmonization process because PNA probes bind to complementary nucleic acid (as compared with nucleic acid/nucleic acid interactions) under a wide range of conditions, thereby permitting one to tailor the conditions more closely to those suitable for the antibody-antigen and/or other ligand-ligand binding.

### RNase-free Reagents:

RNases are enzymes found in nature that degrade RNA. Bacteria contain RNase enzymes. These RNase enzymes can remain active long after the bacteria are dead, for example, by fixation. When the target used to determine a select bacteria or select trait is an RNA molecule, residual RNase activity in the bacteria examined in a whole-cell assay can actively degrade the target molecule(s). If the target molecule(s) is/are low copy number molecules, any destruction of the target molecule(s) can significantly decrease signal of an assay.

There are various reagents commercially available which inactive RNase enzymes. These reagents are commonly referred to RNase inhibitors. One such commercially available RNase inhibitor is Tris(2-carboxyethyl)phosphine hydrochloride (TCEP - Product # 77720 from Thermo Scientific, Rockford, II).

RNase inhibitors can be used to treat samples so that RNase activity in the bacteria cells is inhibited so that degradation of RNA targets is forestalled. RNase inhibitors can be added to any reagent, mixture, formulation and/or solution used in the practice of this invention to inhibit RNase activity in said reagent, mixture, formulation and/or solution and to further inhibit any degradation or target molecules in the bacteria if the bacteria are contacted with said reagent, mixture, formulation and/or solution. Said reagents are said to be RNase-free. It is to be understood however that use of RNase inhibitors is not an absolute requirement of practice of the disclosed methods.

### Inducing mRNA Production:

The literature has suggested that as a target, mRNA is difficult to determine within bacteria (See for example: Coleman et al., "mRNA-targeted fluorescent in-situ hybridization (FISH) of Gram-negative bacteria without template amplification or tyramide signal amplification", J. Microbiological Methods, 71: 246-255 (2007))). This seems to partially result from low copy number within bacteria and partially result from mRNAs inherent instability. One way to increase copy number of target mRNA molecules is to induce mRNA production in live bacteria.

Thus, in some embodiments, mRNA production is induced within bacteria by treatment of live bacteria with a mRNA inducing reagent or reagents for a period of time before they are treated with a mRNA-directed probe or probes. The treatment with the mRNA inducing reagent or reagents can be performed before the bacteria are fixed. The treatment with the mRNA inducing reagent or reagents can be combined with other procedures (such a use of RNase free reagents) so that mRNA targets in the bacteria are not substantially degraded before the bacteria and/or traits are determined according to methods disclosed herein. It is to be understood that in some cases, bacteria will produce enough mRNA to be detectable (without induction). Thus, mRNA induction is not an absolute requirement of practice of the disclosed methods.

### mRNA Stabilizing:

It is also possible to use mRNA stabilizing reagents to stabilize cellular mRNA. Thus, a mRNA stabilizing reagent or reagents can be used in the practice of the methods disclosed herein. A mRNA stabilizing reagent differs from a mRNA inducing reagent in that an mRNA stabilizing reagent preserves mRNA present in the cell whereas a mRNA inducing reagent causes the living cell to produce more mRNA. It is to be understood that these roles are not mutually exclusive however. That is, it is possible for a reagent to be both a mRNA inducing reagent as well as a mRNA stabilizing reagent. For example, some antibiotics can be both a mRNA inducing reagent and a mRNA stabilizing reagent. It is to be understood however that use of a mRNA stabilizing reagent or reagents is not an absolute requirement of practice of the disclosed methods.

### Fixing Bacteria:

Whole cell assays can be performed using fixed bacteria. Fixing bacteria is the process of treating bacterial cells to thereby preserve and/or prepare said bacterial cells for microscopic analysis. Fixed bacteria can be stored for a period time before they are analyzed.

A commonly used fixative reagent is paraformaldehyde. Other commonly used fixative reagents include glyoxal, glutaraldehyde, zinc salts, heat, alcohols (methanol and ethanol), acidic solutions and combinations of any two or more of these. In some embodiments, methods disclosed herein can be practiced by contacting the sample with a fixative reagent or reagents. A commonly used process for fixing cells is referred to as flame fixation; which process may (or may also not) be accompanied by contacting the bacteria with a reagent or reagents. Thus, the methods disclosed herein can be practiced with a fixation step which may (or may not) include contacting the sample with a reagent or reagents.

Any fixative reagent or reagents may contain other compositions not strictly related to fixation. For example, in some embodiments one or more probes may be added to a fixation reagent or reagents. In this way, fixation and probe/target formation can be performed simultaneously. Any combination of reagents is permissible so long as the combination operates for its intended purpose much in the way that the individual reagent or reagents would if not combined.

### Permeabilizing Bacteria:

Permeabilization of bacteria is the process by which the cell membrane/cell wall is modified so that reagents required to perform an assay can pass into (and out of) the bacteria. Cell permeabilization differs from fixation and for many species of bacteria, cell permeabilization is not required.

Some non-limiting examples of cell permeabilizing reagents include solutions/formulations comprising one or more enzymes such as lysostaphin, lysozyme, and proteinases (e.g. proteinase-K and/or achromopeptidase). To permeabilize the bacteria, said enzymes can be contacted with the sample and thereby partially digest the cell membrane and/or cell wall. In some embodiments, the cell permeabilizing reagent or reagents are chemicals, mixtures of chemicals and enzymes or sequential treatment with chemical(s) and enzyme(s) in any order.

Thus, in some embodiments, gram-positive bacteria (e.g. staphylococci bacteria) can be contacted with the cell permeabilizing reagent or reagents in a manner that permits reagents that normally are excluded from (or that pass slowly into) the bacteria to pass more freely into the bacteria and thereby facilitate the whole-cell assays described herein. The degree of permeabilization depends on the nature of the reagents that must penetrate into the cell for practice of the particular assay. Generally, as the size of the molecule that must pass through the cell membrane/cell wall increases, a greater the degree of permeabilization must be performed. Cell permeability that is too low can lead to false-negative or false-positive results (See: Pernthaler et al., "Simultaneous Fluorescence In Situ Hybridization of mRNA and rRNA in Environmental Bacteria", Applied and Environmental Microbiology, 70(9): 5426-5433 (Sept. 2004) at page 5429, col. 2). However, extensive treatment with the cell permeabilizing reagent or reagents can result in destruction of the bacteria cells (See: Furukawa et al., *Microbes Environ.* at page 231, col. 1-2). Various protocols for permeabilizing bacterial cells are discussed in several of the references listed in Section 8, below. Persons using routine experimentation in combination with the disclosure provided herein can determine appropriate conditions for permeabilizing bacteria for any particular assay.

### Washing:

In whole-cell assays, washing steps are commonly performed between one or more steps (or substeps) of a method to remove one or more of the components (or excess components) applied to a sample in a previous step (or substep) to thereby prepare the sample for the next method step (or substep). Washing reagents often are buffered solutions comprising a salt and/or a detergent. In practice, a washing reagent is commonly referred to as a wash(ing) buffer or wash(ing) solution. Numerous washing reagents are commercially available.

A washing step is often practiced after a sample is contacted with probes so that excess probe that does not selectively bind to its respective target is washed away. However, there are reports of no wash ISH-based assays (See: US Pat. No 6,905,824). Whether or not a washing step is required will depend in part on the nature of the fixative reagent or reagents as well as the probe or probes used in the assay and the means by which the determinations are made.

### Amplification Techniques:

As used herein "amplification techniques" refers to methods/techniques used to improve methods of detection either by increasing the number of target molecules that can be determined in an assay or by increasing the signal output from a label. These particular amplification techniques are therefore referred to as target amplification or signal amplification, respectively.

### Target Amplification:

As noted, in target amplification, the number of target molecules is increased. A commonly performed target amplification technique is polymerase chain reaction (PCR) whereby a target nucleic acid (or portion thereof) is copied in an exponential manner, for example by, use of a pair of primers, a thermostable polymerase, nucleotide triphosphates and a process for performing thermal cycles which denature and anneal the target molecule (and copies thereof). Other non-limiting examples of target amplification methods include: Ligase Chain Reaction (LCR), Strand Displacement Amplification (SDA) and Transcription-Mediated Amplification (TMA).

### Signal Amplification:

Signal amplification of a label can be used to improve upon the limits of detection of a method. In brief, signal amplification is typically used where a cell (i.e. bacteria) possesses a low copy number of a particular target and thus, a resulting small number of the respective probe/target complexes. Particularly where a determination (e.g. of the select bacteria or trait) is based on bacteria staining, there may not be enough signal generated if the number of probe/target complexes in the bacteria are sufficiently low. However, if the signal of a single label associated with a probe/target complex can be multiplied or amplified many times, it becomes possible to make a determination even for low copy number targets in a bacterial cell.

There are several types of signal amplification techniques available. Signal amplification can be applied to both direct and indirect labeling techniques. Some non-limiting examples of signal amplification include tyramide signal amplification (TSA, also known as catalyzed reporter deposition (CARD)), Enzyme Labeled Fluorescence (ELF-97 - product and information available from Invitrogen, Carlsbad, CA), Branched DNA (bDNA) Signal Amplification, and rolling-circle amplification (RCA). Specific methods for using these signal amplification techniques to detect low copy number targets within bacteria are discussed in more detail in several of the references listed in Section 8, below.

### 5. Various Embodiments of the Invention

*It should be understood that the order of steps or order for performing certain actions is immaterial so long* as *the present teachings remain operable or unless otherwise specified. Moreover, in some embodiments, two or more steps or actions can be conducted simultaneously so long as the present teachings remain operable or unless otherwise specified.*

This invention pertains to methods for determining select gram-positive bacteria and select traits of gram-positive bacteria of the sample. Generally the select trait(s) will typically be one that is commonly associated with the select gram-positive bacteria (though it may also be found in gram-negative bacteria). Also, it is to be understood that the methods described herein are not limited to determining one select bacteria and one select trait per sample. Rather, the methods can be used to determine multiple bacteria in a sample and/or multiple traits associated with bacteria of the sample. In some embodiments, the multiple bacteria and/or multiple traits will be determined using a multiplex assay. The multiplex assay can involve the use of differential staining of the bacteria whereby the different stain or stains a bacteria exhibits is used to determine the bacteria type and/or trait(s).

Therefore, in some embodiments, this invention pertains to a method comprising: a) contacting a sample with: i) a bacteria-directed probe or probes capable of determining a select gram-positive bacteria in the sample; and ii) a chromosomal DNA-, mRNA- and/or native plasmid-directed labeled PNA probes capable of determining chromosomal DNA, mRNA and/or plasmid nucleic acid associated with a select trait that may be possessed by the select gram-positive bacteria of the sample. Often the sample will be suspected of comprising one or more gram-positive bacteria. It is to be understood that said contacting of the sample with the components identified in substeps i) and ii) can be practiced in any order or the contacting can occur simultaneously as the order of the contacting is not intended to be a limitation. Said method further comprises: b) determining one or more of the select gram-positive bacteria in the sample; and c) determining bacteria of the sample that possess the select trait. The method: i) is practiced on whole-cells (i.e. intact cells); ii) steps (b) and (c) are carried out (i.e. practiced) in either order or simultaneously; and iii) the chromosomal DNA-, mRNA- and/or native plasmid-directed labeled PNA probes each comprise a single label or two labels (i.e. each probe is a single labeled or dual labeled probe).

In some embodiments, the focus is on determining bacteria of the sample that possess the select trait. Thus, in some embodiments, this invention pertains to a method comprising contacting a sample comprising bacteria with a chromosomal DNA, mRNA- and/or native plasmid-directed labeled probes capable of determining chromosomal DNA, mRNA and/or plasmid nucleic acid associated with a select trait that may be possessed by a select gram-positive bacteria and/or in other bacteria of said sample. The method further comprises determining bacteria of said sample that possess said select trait wherein; i) said method is practiced on whole-cells; and ii) said chromosomal DNA-, mRNA- and/or native plasmid-directed labeled probes each comprise a single label or two labels. Said method may further comprise contacting the sample with a bacteria-directed probe or probes capable of determining a select gram-positive bacteria in said sample and determining one or more of said select gram-positive bacteria in said sample.

According to these various methods, determination of the select gram-positive bacteria and/or select trait involves determining the formation of probe/target complexes for the bacteria-directed probe or probes and chromosomal DNA-, mRNA-and/or native plasmid-directed labeled probes, respectively. The formation of the probe/target complexes is accomplished under suitable binding conditions (or suitable hybridization conditions as appropriate). In some embodiments, formation of the respective probe/target complex or complexes will be evident based on the nature of the staining of the bacteria. Thus, for these embodiments, the select bacteria and/or select trait can be determined by analysis of the staining of individual bacteria. The staining of individual bacteria can, for example, be monitored (determined) using a microscope, slide scanner or flow cytometer.

These methods are practiced without use of signal amplification of the label or labels linked to the chromosomal DNA-, mRNA-and/or native plasmid-directed labeled PNA probes and without use of in-situ PCR. These methods can be practiced without contacting the sample with a cell permeabilizing reagent or reagents. In some embodiments, said single label (linked to each of the chromosomal DNA-, mRNA- and/or native plasmid-directed labeled probe or probes) comprises a fluorescent label or labels that exhibit(s) an emission maximum of less than 650 nm.

In some embodiments, these methods can be practiced using only mRNA-directed probes, wherein probes are capable of determining the select trait. In some aspects only a single mRNA-directed probe is used to determine a trait or a single mRNA-directed probe is used to determine each of the traits of interest (i.e. one probe per trait such that if you have three traits of interest, three probes would be used). In the methods of the invention, these methods are practiced with a mixture of mRNA-directed labeled PNA probes.

Each of the mRNA-directed probes comprises a single label or two labels (i.e. each probe is a single labeled or dual labeled probe). In some embodiments, said label or labels is/are a fluorescent label or labels that exhibit(s) an emission maximum of less than 650 nm.

Each chromosomal DNA-, mRNA- and/or native plasmid-directed labeled probe comprises a single label or two labels (i.e. each probe is a single labeled or dual labeled probe). The method is practiced without signal amplification of a label or labels of said chromosomal DNA-, mRNA-and/or native plasmid-directed labeled PNA probes. In .some embodiments, each chromosomal DNA-, mRNA- and/or native plasmid-directed labeled probe comprises a single label and the method is practiced without signal amplification of said single label of said chromosomal DNA-, mRNA- and/or native plasmid-directed labeled probes.

In some embodiments, bacteria-directed probe or probes is/are antibody-based. As such, the target for each probe is an antigen found on the surface of, or within, the select gram-positive bacteria.

In some embodiments, the bacteria-directed probe or probes is/are mRNA-directed. In some embodiments, the bacteria-directed probe or probes is/are directed to a regulatory RNA (e.g. sRNA or aRNA). As such, the target for each probe is a nucleobase sequence of (or within) mRNA or regulatory RNA (e.g. sRNA or aRNA), respectively.

Each bacteria-directed probe is labeled with a single label or two labels (i.e. each probe is a single labeled or dual labeled probe). In some embodiments, said label or labels are fluorescent and exhibit an emission maximum of less than 650 nm. In some embodiments, one or more of said label or labels are fluorescent and exhibits an emission maximum of 650 nm or more.

In some embodiments, practice of the first described method above further comprises determining any select gram-positive bacteria of the sample that also possess the select trait based on analysis of steps (b) and (c). By "analysis of steps (b) and (c)" we refer to analyzing the determination(s) made in steps (b) and (c), which determinations can, by application of reasoning, lead one to recognize, in this case, which select gram-positive bacteria of the sample that also possess the select trait.

It is to be understood that not all of the select gram-positive bacteria of the sample will possess the select trait (in fact it may be that none of the select gram-positive bacteria possess the select trait). For example, the sample may be a mixed population and thereby comprise both select gram-positive bacteria that do possess the select trait as well as select gram-positive bacteria that do not possess the select trait. It some embodiments, all or substantially all of the select gram-positive bacteria will possess the select trait.

In some embodiments, these methods can be practiced with or without various additional steps and/or reagents. For example, one or more washing steps maybe conducted by contacting the sample with one or more washing reagents. In some embodiments, the sample is contacted with a fixative reagent or reagents. In some embodiments, the sample is contacted with a cell permeabilizing reagent or reagents. In some embodiments, the sample is contacted with a mRNA inducing reagent or reagents. In some embodiments, the mRNA inducing reagent or reagents can induce the production of non-surface protein associated with the select trait, which can increase the sensitivity and/or accuracy of an assay for the select trait. It is to be understood that in some embodiments, two or more of the forgoing reagents can be applied to the same sample, each reagent contacting the sample one or more times. Contacting of the sample with the various reagents can be performed in any order (or simultaneously) that permits accurate determination of the select bacteria and/or traits.

In some embodiments, theses methods may be conducted as an RNase-free assay. Typically, this involves treating all the reagents that are used to contact the sample with a reagent or reagents that inhibits RNase activity. Similarly, the sample itself can be contacted with the same or a different reagent or reagents that inhibit RNase activity.

In some embodiments, one or more steps that are commonly performed are omitted. For example, in hybridization assays, it is common to perform a pre-hybridization step prior to contacting the sample with the hybridization probe or probes. In some embodiments of this invention where one or more hybridization probes are used, the method is performed with no pre-hybridization step. When an antibody probe or probe is used, a blocking step is often performed (or not) before the sample is contacted with said antibody probe or probes but this step may be omitted. In some embodiments, the cell permeabilization step is omitted. In some embodiments, a wash step or steps is/are omitted. Indeed any commonly performed step can be omitted where said omission does not cause the method to fail to produce an accurate result.

In some embodiments, all probes are labeled. In some embodiments, all labels are fluorescent labels. In some embodiments, these methods are conducted as an in-situ hybridization (ISH) assay because all probes are hybridization probes (i.e. they hybridized to their respective targets). In some embodiments, all probes are hybridization probes and all labels are fluorescent labels. In this case the method is conducted as a fluorescence in-situ hybridization (FISH) assay.

In some embodiments, the select trait can be associated with 1) antibiotic resistance; 2) toxin production; and/or 3) virulence. For example, the select trait can be associated with the presence of the: 1) the mecA gene or *vanA* or *vanB* gene; 2) *tcdB* gene and/or 3) *lukF* and *lukS* genes of bacteria, respectively. Thus, the select trait can be determined by determining the presence of the: 1) the mecA gene or *vanA* or *vanB* gene; 2) *tcdB* gene and/or 3) *lukF* and *lukS* genes of bacteria, respectively, in the select gram-positive bacteria (or other bacteria of the sample).

In some embodiments, more than one select gram-positive bacteria and/or select trait can be determined. In some embodiments, this can be accomplished by multiplexing. In some embodiments, this can be accomplished by reprobe cycling the sample. In some embodiments, this can be accomplished by both multiplex and reprobe cycling the sample. Thus, in some embodiments, these methods further comprises contacting the sample with; 1) a second bacteria-directed probe or probes capable of determining a second select gram-positive bacteria in the sample; and/or 2) a second chromosomal DNA, mRNA- and/or native plasmid-directed labeled probe or probes capable of determining chromosomal DNA, mRNA and/or plasmid nucleic acid associated with a second select trait that may be possessed by any bacteria of the sample (including the (first) select gram-positive bacteria and/or the second select gram-positive bacteria). It is to be understood that the method can also be practiced by contacting the sample with additional probes or probe sets to one or more additional select bacteria and/or select traits.

In some embodiments, this invention is more specifically directed to determining one or more methicillin-resistant staphylococcus aureus (MRSA) bacteria, methicillin-resistant coagulase-negative staphylococci (MR-CNS) and/or methicillin-sensitive staphylococcus aureus (MSSA) in a sample. As suggested in the "Introduction", above, being able to efficiently determine methicillin-resistant staphylococcus aureus (MRSA) bacteria in particular, and optionally other methicillin-resistant bacteria (such as methicillin-resistant coagulase-negative staphylococci (MR-CNS) and/or methicillin-sensitive staphylococcus aureus (MSSA)), in clinical samples is critical in many areas of patient care.

Thus, in some embodiments, this invention is directed to a method or methods comprising: a) contacting a sample with: i) a bacteria-directed probe or probes capable of determining *S*. *aureus* bacteria in the sample; and ii) a chromosomal DNA and/or mRNA-directed labeled probe or probes capable of determining methicillin-resistance in bacteria of the sample. Often the sample will be suspected of comprising one or more methicillin-resistant staphylococcus aureus (MRSA) bacteria. It is to be understood that said contacting of the sample with the components identified in step a), substeps i) and ii), can be practiced in any order or the contacting can occur simultaneously as the order of the contacting is not intended to be a limitation. Said method further comprises: b) determining one or more staphylococcus aureus bacteria (i.e. a select gram-positive bacteria) in the sample; and c) determining one or more bacteria of the sample that possess methicillin-resistance (i.e. a trait). Said determinations are made by determining formation of probe/target complexes form between the probes and their respective targets within the bacteria under suitable binding conditions (or suitable hybridization conditions, as appropriate).

The method: i) is practiced on whole-cells (i.e. intact cells); and ii) steps (b) and (c) are carried out in either order or simultaneously. The chromosomal DNA- and/or mRNA-directed labeled probe or probes each comprise a single label or dual label (i.e. each probe is a single labeled or dual labeled probe).

In some embodiments, the focus is on determining bacteria of the sample that possess the select trait (i.e. methicillin-resistance). Thus, in some embodiments, this invention pertains to a method comprising contacting a sample with a chromosomal DNA and/or mRNA-directed labeled probe or probes capable of determining methicillin-resistance in bacteria of said sample; and determining one or more bacteria of said sample that possess methicillin-resistance wherein, said method is practiced on whole-cells. The bacteria can be gram-positive bacteria. The method can further comprise contacting the sample with a bacteria-directed probe or probes capable of determining *S. aureus* bacteria in said sample and determining one or more S. *aureus* bacteria in said sample.

These methods are practiced without use of signal amplification of the label or labels linked to the chromosomal DNA- and/or mRNA-directed labeled probe or probes. If however, the chromosomal DNA- and/or mRNA-directed labeled probe or probes each comprise a single label or two labels, said label or labels can be fluorescent and have an emission maximum of less than, equal to or more than 650 nm. In some embodiments, these methods can be practiced without use of any amplification techniques. In some embodiments, these methods can be practiced without contacting the sample with a cell permeabilizing reagent or reagents.

In some embodiments, these methods can be practiced using only mRNA-directed probe or probes wherein said probe or probes are capable of determining mRNA associated with methicillin-resistance. In some embodiments, only a single mRNA-directed probe is used to determine methicillin-resistance. In some embodiments, two or more mRNA-directed probes are used to determine methicillin-resistance (i.e. a mixture of mRNA-directed probes which probes can each be labeled with one or two labels).

Each of the mRNA-directed probe or probes comprises a single label or two labels (i.e. each probe is a single labeled or dual labeled probe). In some embodiments, said label or labels is/are fluorescent and exhibit(s) an emission maximum of less than, equal to or more than 650 nm. Each chromosomal DNA-and/or mRNA-directed labeled probe comprises a single label or two labels (i.e. each probe is a single labeled or dual labeled probe) and the method is practiced without signal amplification of said single label of said chromosomal and/or DNA-, mRNA-directed labeled probe or probes.

In some embodiments, bacteria-directed probe or probes is/are antibody-based. As such, the target for each probe is an antigen found on the surface of, or within, the select gram-postlive bacteria.

Also disclosed herein are bacteria-directed probe or probes that is/are rRNA-directed. As such, the target for each probe is a nucleobase sequence found within rRNA of the select gram-positive bacteria. As suitable rRNA-directed probe for determining S. aureus bacteria in clinical samples is commercially available and a study describing its use is described in: Forrest et al., "Impact of rapid in situ hybridization testing on coagulase-negative staphylococci positive blood cultures", Journal of Antimicrobial Chemotherapy, 58: 154-158 (2006). In some embodiments, the bacteria-directed probe or probes is/are mRNA-directed or directed to other regulatory RNA (e.g. sRNA or aRNA). As such, the target for each probe is a nucleobase sequence of (or within) mRNA or regulatory RNA (e.g. sRNA or aRNA), respectively.

Each bacteria-directed probe is labeled with a single label or two labels (i.e. each probe is a single labeled or dual labeled probe). In some embodiments, said label or labels is/are fluorescent and exhibit(s) an emission maximum of less than 650 nm. In some embodiments, one or more of said label or labels is/are fluorescent and exhibit(s) an emission maximum of 650 nm or more.

In some embodiments, practice of the first disclosed method specifically related to methicillin resistance determination further comprises determining any methicillin-resistant staphylococcus aureus bacteria of the sample based on analysis of steps (b) and (c). By "analysis of steps (b) and (c)" we refer to analyzing the determination(s) made in steps (b) and (c), which determinations can, by application of reasoning, lead one to recognize, in this case, which *S*. *aureus* bacteria of the sample are methicillin-resistant staphylococcus aureus (MRSA) bacteria.

It is to be understood that in some samples not all of the S. *aureus* bacteria of the sample will be methicillin-resistant. For example, the sample may be a mixed population and thereby comprise methicillin-resistant staphylococcus aureus (MRSA) bacteria, methicillin-resistant coagulase-negative staphylococci (MR-CNS) and/or methicillin-susceptible staphylococcus aureus (MSSA) bacteria. As noted by Gröbner et al. at page 1691, col. 1, the BD GeneOhm™ StaphSR assay cannot distinguish samples that comprise mixed populations of MRSA and MSSA. It is an advantage of the present invention that, because the types and traits of individual bacteria can be determined, it is possible to properly characterize mixed populations using the whole-cell methods disclosed herein (See for example: Example 10).

In some embodiments, all, or substantially all, of the bacteria of the sample will be methicillin-resistant staphylococcus aureus (MRSA) bacteria. In some embodiments, none of the bacteria of the sample with be methicillin-resistant staphylococcus aureus (MRSA) bacteria, in which case the treatment regime of a patient (from which the sample may have been taken) could be altered so as to reduce hospital costs (Again see: Forrest et al.).

In some embodiments, these methods can be practiced with or without various additional steps and/or reagents. For example, one or more washing steps maybe conducted by contacting the sample with one or more washing reagents. In some embodiments, the sample is contacted with a fixative reagent or reagents. In some embodiments, the sample is contacted with a cell permeabilizing reagent or reagents. In some embodiments, the sample is contacted with a mRNA inducing reagent or reagents. In some embodiments, the mRNA inducing reagent or reagents can induce the production of non-surface protein associated with the select trait, which can increase the sensitivity and/or accuracy of an assay for the select trait. It is to be understood that in some embodiments, two or more of the forgoing reagents can be applied to the same sample, each reagent contacting the sample one or more times. Contacting of the sample with the various reagents can be performed in any order (or simultaneously) that permits accurate determination of the select bacteria and/or traits.

In some embodiments, the method is conducted as an RNase-free assay. Typically, this involves treating all the reagents that are used to contact the sample with a reagent or reagents that inhibits RNase activity. Similarly, the sample itself can be contacted with the same or a different reagent or reagents that inhibit RNase activity.

In some embodiments, one or more steps that are commonly performed are omitted. For example, in hybridization assays, it is common to perform a pre-hybridization step prior to contacting the sample with the hybridization probe or probes. In some embodiments of this invention where one or more hybridization probes are used, the method is performed with no pre-hybridization step. When an antibody probe or probe is used, a blocking step is often performed (or not) before the sample is contacted with said antibody probe or probes but this step may be omitted. In some embodiments, the cell permeabilization step is omitted. In some embodiments, a wash step or steps is/are omitted. Indeed any step commonly performed can be omitted where said omission does not cause the method to fail to produce an accurate result.

In some embodiments, all probes are labeled. In some embodiments, all labels are fluorescent labels. In some embodiments, these methods can be conducted as an in-situ hybridization (ISH) assay because all probes are hybridization probes (i.e. they hybridized to their respective targets). In some embodiments, all probes are hybridization probes and all labels are fluorescent labels. In this case, theses methods can be conducted as a fluorescence in-situ hybridization (FISH) assay.

In some embodiments, practice of the first disclosed method specifically related to methicillin resistance determination further comprises: α) contacting the sample with a second bacteria-directed probe or probes capable of determining coagulase-negative staphylococci (CNS) bacteria in the sample wherein the said second bacteria-directed probe or probes is/are independently detectable from said bacteria-directed labeled probe or probes capable of identifying staphylococcus aureus bacteria in the sample; β) determining coagulase-negative staphylococci (CNS) bacteria in the sample (e.g. S. epidermidis which is a common skin bacteria that is a staphylococci other than staphylococcus aureus); and χ) determining methicillin-resistant coagulase-negative staphylococci (MR-CNS) bacteria in the sample based on analysis of steps (β) and (c) and optionally step (b). By "analysis of step (β) and (c) and optionally step (b)" we refer to analyzing the determination(s) made in steps (β) and (c) and optionally step (b), which determinations can, by application of reasoning, lead one to recognize, in this case, which bacteria in the sample are methicillin-resistant coagulase-negative staphylococci (MR-CNS).

In some embodiments, both methicillin-resistant staphylococcus aureus (MRSA) and methicillin-resistant coagulase-negative staphylococci (MR-CNS) bacteria are determined in the same sample. In some embodiments, methicillin-resistant staphylococcus aureus (MRSA) methicillin-resistant coagulase-negative staphylococci (MR-CNS) bacteria or methicillin-sensitive staphylococcus aureus (MSSA) are determined in the same sample. In some embodiments, a mixed population of methicillin-resistant staphylococcus aureus (MRSA), methicillin-resistant coagulase-negative staphylococci (MR-CNS) bacteria and/or methicillin-sensitive staphylococcus aureus (MSSA) of the sample are determined (See Example 10). In some embodiments, this determination can be made simultaneously. An example of such an assay (performed in a multiplex format) can be found in Example 3. It should be apparent to those of ordinary skill in the art that the determination of bacteria and traits using the images in Fig. 3 (as discussed in Example 3) can be automated.

In some embodiments, the method further comprises characterizing the sample as heterogeneous or homogeneous for methicillin-resistant staphylococcus aureus (MRSA) and/or methicillin-resistant coagulase-negative staphylococci (MR-CNS) bacteria based on analysis of steps (b), (β), (c) and (χ). By "analysis of steps (b), (β), (c) and (χ)" we refer to analyzing the determination(s) made in steps (b), (β), (c) and (χ), which determinations can, by application of reasoning, lead one to recognize, in this case, whether or not the methicillin-resistant bacteria of the sample are heterogeneous or homogeneous for the methicillin-resistance trait.

In practice, this may be possible by determining the amount (e.g. intensity) of staining exhibited by select bacteria in a sample with respect to the select trait as compared with other of the select bacteria in the sample. If the intensity of staining with respect to the select trait for various select bacteria is substantially the same, expression of the trait in the various bacteria of the sample is homogeneous. However, if the intensity of staining with respect to the select trait for various select bacteria differs among various bacteria of the sample, expression of the trait in the various bacteria of the sample is heterogeneous.

Moreover, in some embodiments, determining whether or not a sample is heterogeneous or homogeneous for MRSA may require (or at least it may be preferable to make) reference to additional testing such as, for example, growing bacteria of the sample in culture in different media, wherein each different media comprises a different concentration of antibiotic or antibiotics. In this way it is possible to determine that the select bacteria of the sample exhibit different levels of expression of the methicillin-resistance trait based on the colony count at the different levels of antibiotic(s) in the media.

In some embodiments, theses methods may further comprise contacting said sample with a mRNA inducing reagent or reagents. In some embodiments, these methods may further comprise treating said sample with an RNase inhibitor. In some embodiments, no pre-hybridization step is performed.

In some embodiments, all labels are fluorescent labels and said method is a fluorescent in-situ hybridization (FISH) assay. In some embodiments, a label or labels of said chromosomal DNA and/or mRNA-directed labeled probe or probes is/are determined directly.

In the methods of the invention, chromosomal DNA- and/or mRNA-directed labeled probes are PNA. In some embodiments, the chromosomal DNA-and/or mRNA-directed labeled probe or probes is/are 10 to 20 nucleobase subunits in length.

Applicants have surprisingly determined that the various methods disclosed herein can be performed without performing a cell permeabilization step. Accordingly, in some embodiments, this invention is directed to a method comprising: a) contacting a sample with: i) a bacteria-directed probe or probes capable of determining a select gram-positive bacteria in said sample; and ii) a chromosomal DNA-, mRNA-and/or native plasmid-directed labeled probe or probes capable of determining chromosomal DNA, mRNA and/or plasmid nucleic acid associated with a select trait that may be possessed by said select gram-positive bacteria and/or in other bacteria of said sample. Said method further comprises: b) determining one or more of said select gram-positive bacteria in said sample; and c) determining bacteria of said sample that possess said select trait; wherein, i) said method is practiced on whole-cells; ii) steps (b) and (c) are carried out in either order or simultaneously; and iii) said method is practiced without treating the sample with a cell permeabilizing reagent or reagents. The method is further practiced without performing any signal amplification. In some embodiments, the bacteria-directed probe or probes and the chromosomal DNA-, mRNA- and/or native plasmid-directed labeled probe or probes each comprise a single or double label and said determinations are made by direct detection of said labels.

In some embodiments, the method can be practiced to determine *S*. *aureus* bacteria and methicillin-resistance. Accordingly, step (a) will more specifically be directed to: a) contacting a sample with: i) a bacteria-directed probe or probes capable of determining *S. aureus* bacteria in said sample; and ii) a chromosomal DNA and/or mRNA-directed labeled probe or probes capable of determining methicillin-resistance in bacteria of said sample. Similarly, steps (b) and (c) will more specifically be directed to determining one or more *S*. *aureus* bacteria in said sample; and c) determining one or more bacteria of said sample that possess methicillin-resistance.

In some embodiments, the method is directed to focusing on determining a trait or traits of bacteria in the sample. Thus, in some embodiments, the method comprises contacting a sample comprising bacteria with a chromosomal DNA-, mRNA-and/or native plasmid-directed labeled probe or probes capable of determining chromosomal DNA, mRNA and/or plasmid nucleic acid associated with a select trait that may be possessed by a select gram-positive bacteria and/or in other bacteria of said sample; and determining bacteria of said sample that possess said select trait wherein, said method is practiced on whole-cells; and ii) said method is practiced without treating the sample with a cell permeabilizing reagent or reagents.

This disclosure also pertains to probe mixtures, compositions and/or formulations useful for determining select traits and/or select gram-positive bacteria. In some aspects, each of said mRNA-directed probe of said mixture is a single labeled probe or a dual labeled probe. For example, in some aspects this disclosure pertains to a mixture, composition and/or formulation comprising two or more mRNA-directed probes capable of determining a select trait known to exist in select bacteria, select gram-positive bacteria and/or other bacteria of a sample. Said mRNA-directed probe or probes can bind with specificity to a target, associated with said select trait, within a molecule or molecules of mRNA of said bacteria. Said mixtures can further comprise one or more bacteria-directed probes (e.g. a rRNA-directed bacteria-directed probe capable of determining a select bacteria in a sample). The mRNA-directed probe or probes and/or bacteria-directed probe or probes can be PNA probes. Said mixtures, compositions and/or formulations can, for example, be used as in the hybridization (contacting) step of the methods disclosed herein such that contacting a sample with said mixture, composition or formulation produces probe/target complexes that can be determined to thereby indicate bacteria types and traits, as appropriate.

### 6. Other Advantages Of Embodiments Of The Present Invention

It is an advantage of some embodiments of the present invention that it is possible to efficiently determine both select gram-positive bacteria and select trait(s) of bacteria (whether or not the select trait is associated with the select gram-positive bacteria) in a single sample using a whole-cell assay format. By using a whole-cell assay format, it is, *inter alia,* possible to: 1) maintain information about cell morphology and thereby further confirm information such as bacteria species (i.e. confirm that the select bacteria determined possess the expected cell morphology); 2) determine whether or not the sample comprises a mixed population of bacteria or interest, 3) determine if a sample is heterogeneous or homogeneous for a select bacteria or select trait; and/or 4) quantify (absolutely or with respect to other bacteria in the sample) bacteria of various types in a sample. All this can, for example, be accomplished in a multiplex format by differential staining of the bacteria based on characteristics sought to be determined in the assay. The methods also permit automation (including automation of the multiplex mode of practice) of the determination step(s) whereby results can be provided according to execution of an algorithm.

Applicants have demonstrated that, in some embodiments, it is possible to determine mRNA in intact gram-positive bacteria using single labeled and/or dual labeled PNA probes (which probes can be short (10-20 subunits in length) probes produced by denovo methods) without use of: 1) cell permeabilization by enzymatic treatment; 2) amplification techniques (e.g. signal amplification or target amplification) and/or 3) fluorescent labels that exhibits an emission maximum of at least 650 nm. In some embodiments, mixtures of mRNA-directed probes can used to increase signal where the number of mRNA targets in a bacteria are expected to be very low.

Additionally, it is an advantage that, in some embodiments, it is possible to determine mRNA targets within bacteria using short probes that are prepared by denovo synthesis (as compared with transcript probes which are prepared by enzymatic methods). Moreover, in some embodiments, it is possible to reduce the probe hybridization step to less than 2 hours as compared with the 5-16 hours described in various references listed below.

Indeed, the scientific literature has acknowledged the difficulty in detecting mRNA in gram-positive bacteria (See in the "Introduction" section above the associated discussion of: Hahn et al., "Detection of mRNA in Streptomyces Cells by Whole-Cell Hvbridization with Diaoxiaenin-Labeled Probes", Applied and Environmental Microbiology, 59(8): 2753-2757 (Aug. 1993); Wagner et al., "In situ detection of a virulence factor mRNA and 16S rRNA in Listeria", FEMS Microbiol. Lett., 160(1): 159-168 (Mar. 1998); and Hönerlage et al., "Detection of mRNA of nprM in Bacillus megaterium ATTC 14581 grown in soil by whole-cell hybridization", Arch. Microbiol., 163:235-241 (1995)).

While the literature does contain various reports related to the difficulty of performing whole-cell assays on gram-positive bacteria in general (and despite the clinical significance of determining methicillin-resistant bacteria (e.g. MRSA)), there does not appear to be any example of whole-cell assays for determining MRSA and/or MR-CNS. It may be that it is very difficult to determine evidence of the methicillin-resistance trait in intact staphylococcus aureus bacteria. Anyway, it is an advantage of the present invention that it is possible to determine the trait of methicillin-resistance in select gram-positive bacteria of a sample (including as appropriate MRSA and MR-CNS) using chromosomal DNA- and/or mRNA-directed labeled probe or probes (in combination with a probe or probes for the select-bacteria) in a whole-cell assay format. This method may find great utility as a clinical assay given the concerns related to the spread and treatment of patients with MRSA in hospitals.

It is an advantage of the whole-cell format that the heterogeneity/homogeneity of bacteria of the sample can be determined based on visual analysis of the sample. Thus, it is an advantage of the present invention that the heterogeneity/homogeneity of a trait or traits of the bacteria of a sample can be determined by practice of the methods disclosed herein. For its clinical significance with respect to patient care, this advantage may prove to be particularly useful with respect to determining the heterogeneity/homogeneity of methicillin-resistance of bacteria of a sample.

### 7. Examples

*Aspects of the present teachings can be further understood in light of the following examples, which should not be construed* as *limiting the scope of the present teachings in any way*, *which is only limited by the appended claims.*

### Example 1: Determination of MRSA

Unless otherwise noted, all procedures were performed at room temperature (RT). The composition of Hybridization Buffer (HB), Wash Buffer (WB), paraformaldehyde (PAF), RNase-free Permeabilizing Solution (PS), Mounting Media (MM) as well as a description of various bacterial strains (e.g. *S*. *aureus* and other staphylococcus strains), PNA probes and fluorescent microscopy (FM) used in these Examples 1-4 is provided in Appendix I (below).

### Cell growth, fixation and permeabilization:

S. *aureus* bacteria were inoculated into Tryptic Soy Broth (TSB) and grown overnight (16 hours) at 37°C. MecA mRNA production was then induced with 0.5 µg/mL oxacillin in overnight culture diluted (10-fold) with TSB. Oxacillin-induced cells were diluted (20-fold) in sterile 2% glucose solution and deposited on glass slides by cytocentrifugation. Deposited cells were fixed with 10% paraformaldehyde (PAF) for 30 minutes (min). Free aldehyde groups were then blocked for 30 min with Phosphate Buffered Saline (PBS, 10 mM phosphate pH=7.4, 0.9% NaCl) containing 0.1 M glycine. Slides were then rinsed with PBS containing 0.05 % Tween 20 (PBS-Tween) and air dried. 0.075 mL of RNase-free Permeabilizing Solution (PS) was then applied on deposited cells and incubated for 30 min. After the incubation, the cells were rinsed with 70% methanol and air dried.

### Fluorescence in situ hybridization and fluorescence microscopy:

25 µL of Hybridization Buffer (HB) containing PNA Probes (See below for probe concentrations) was then applied on slide and covered with a cover slip. The cells were then hybridized for 2 hours at 55°C in a hybridization chamber. After the hybridization, the slides were washed with Wash Buffer (WB) for 30 min at 55°C. A drop of Mounting Media (MM) containing 1 µg/mL 4'-6-Diamindo-2-phenylindole (DAPI) and a cover slip was applied on the dried specimen and slide-deposited cells were then examined by FM.

### Probe concentrations:

1) A mixture of five fluorescein-labeled PNA probes (probe 1, 2, 3, 4 and 5 (See Table 1, below) at concentrations of 220, 280, 120, 95 and 79 nM, respectiveiy). Combined concentration of probes was 794 nM. (Slides A & C)

2) A single fluorescein-labeled E. coli specific rRNA-directed probe (probe 6 concentration 750 nM). This was used as a control probe. (Slide B)

3) A single fluorescein-labeled PNA probe (probe 2, concentration 750 nM). (Slide D)

4) A single fluorescein-labeled PNA probe (probe 2, concentration 300 nM) combined with biotin-labeled* PNA probe (probe 7, concentration 5000 nM). 16.6-fold excess of non-labeled probe was used in control experiment to prove specificity of hybridization. (Slide E)

* In this case the probe was incidentally labeled with biotin. Because the assay was performed as a FISH assay and biotin is non-fluorescent, this probe is the functional equivalent of a non-labeled probe.

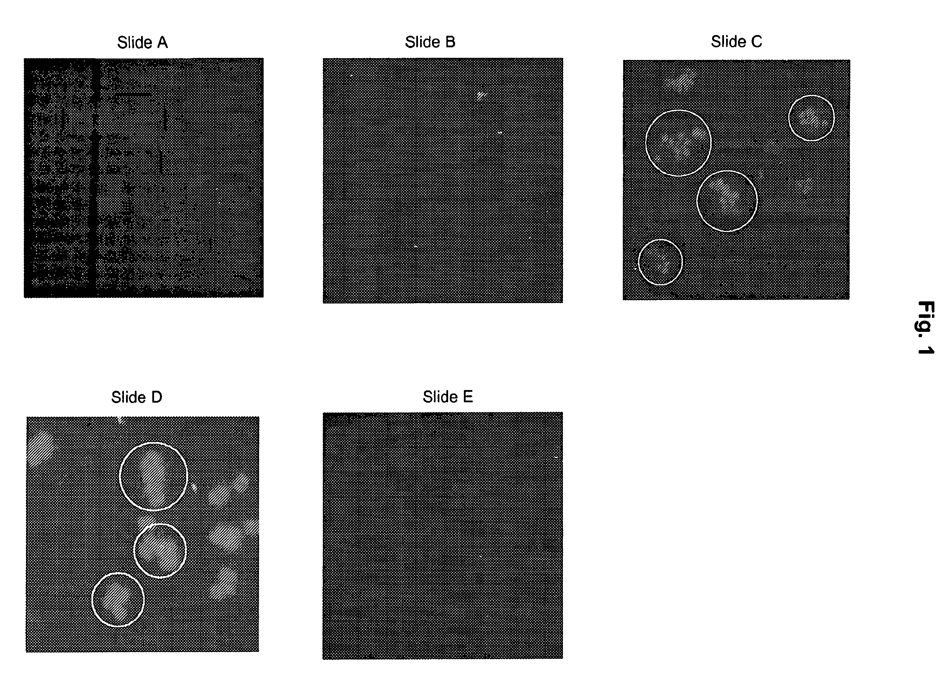

Images obtained from the FM analysis are presented in Fig. 1, which Figure contains the images obtained from 5 slides (Slides A-E). Some (but not all) of the visible colonies in the slides are circled (See circled sections of Slides C & D). This should assist in analysis of the slides where black and white copies of the Figures are provided in lieu of color copies. The conditions for each of the slides examined in Fig. 1 are as follows:

A) MSSA 29213 probed with 5 PNA probes (Probes 1-5)

B) MRSA 43300 probed with E. coli-directed probe (Probe 6)

C) MRSA 43300 probed with 5 PNA probes (Probes 1-5)

D) MRSA 43300 probed with a single PNA probe (Probe 2)

E) MRSA 43300 probed with a single PNA probe (Probe 2) in the presence of 16.6-fold excess of biotin labeled Probe 2 (i.e. Probe 7).

### Results:

The results of this Example are discussed with reference to Fig. 1. Green fluorescent bacteria were observed in slides C and D. This result was consistent with the presence of MRSA bacteria treated (contacted) with a PNA probe (Slide D) or probes (Slide C). No bacteria were observed in slide A where the bacterial strain (MSSA) of staphylococci does not contain the MecA gene (Slide A). No signal was observed with MRSA bacteria treated with a probe directed to a rRNA target known to be associated with E. *coli* bacteria (Slide B). No signal was observed with MRSA bacteria treated with both a labeled mRNA-directed probe (Probe 2) and a large excess of the same probe in biotin labeled (functionally unlabeled) form (Probe 7) - See Slide E.

### Example 2: PNA FISH of S. aureus.

Cell growth, fixation and cell permeabilization were performed as described in Example 1 except as noted below.

### Fluorescence in situ hybridization and fluorescence microscopy:

25 µL of HB containing five fluorescein-labeled PNA probes (Probes 1-5 at concentrations described in Example 1) in addition to one tetramethylrhodamine (TAMRA)-labeled, *S. aureus*-specific, rRNA-directed probe (Probe 8 at a concentration of 500 nM) was applied on slide and covered with a cover slip. The remainder of the procedure was performed as described in Example 1. Images obtained from the FM analysis are presented in Fig. 2, Slides A1, A2, B1 and B2. Some (but not all) of the visible colonies in the slides are surrounded by a circle (slide A) or a square (Slide B). This should assist in analysis of the slides where black and white copies of the Figures are provided in lieu of color copies. In Image A-1 and A-2, the same colonies are encircled to simplify the comparison. The conditions for each of the slides examined in Fig. 2, are as follows:

A-1) MRSA 43300, Dual band filter

B-1) MSSA 29213, Dual band filter

A-2) MRSA 43300, FITC filter

B-2) MSSA 29213, FITC filter

### Results:

The results of this Example are discussed with reference to Fig. 2. Red fluorescent stained bacteria in Slides A-1 and B-1 confirms *S*. *aureus* is present based on hybridization of the TAMRA labeled Probe 8 to its rRNA target. This result is expected since both MRSA and MSSA are staphylococci bacteria. Green fluorescent stained bacteria in Slide A-2 suggest MRSA is present based on hybridization of Probes 1-5 to their respective targets. The Images in A-1 and A-2 are of the same slide (using a different filter set, i.e. red or green) and nearly the same section of the slide as can be seen by alignment of the visible colonies. The absence of green fluorescent stained bacteria in Slide B-2 indicates that the bacteria are not methicillin-resistant. This (negative) result is consistent with the nature of the MSSA bacteria used in the assay; which bacteria are not methicillin-resistant. Images B-1 and B-2 are different sections of the same slide using a different filter set (red and green, respectively).

### Example 3: Simultaneous Dual (Multiplex) Determination by PNA FISH in S. aureus and S. epidermidis.

In this example a mixture of MRSA 43300 and MRSE 51625 was prepared and examined using three fluorescent filters as described below. The growth and oxacillin induction of *S*. *aureus* cells was performed as described in Example 1. *S*. *epidermidis* cells were however not induced by oxacillin, because MecA mRNA in these cells mRNA was expressed constitutively. *S*. *epidermidis* was grown essentially as described for *S. aureus* in Example 1. Cell fixation and permeabilization of both MRSA 43300 and MRSE 51625 was performed as described in Example 1.

### Fluorescence in situ hybridization and fluorescence microscopy

Hybridization Buffer containing five fluorescein-labeled PNA probes (Probes 1-5 at concentrations described in Example 1), one TAMRA-labeled, S. aureus-specific, rRNA-directed probe (Probe 8 at concentration described in Example 2) and one Pacific Blue-labeled, *S. epidermidis*-specific, rRNA-directed probe (Probe 9, concentration 500 nM) was then applied on slide containing a mixture of MRSA 43300 and MRSE 51625 and covered with a cover slip. The remainder of the procedure was performed as described in Example 1. Images obtained from the FM analysis are presented in Fig. 3, Images A-C. These images are of the same section of the slide. In each case, only the fluorescence filter was changed. This is an example of a multiplex assay as two different select bacteria are independently determined and one trait (methicillin-resistance) is also determined for the bacteria of a single sample.

In these images some (but not necessarily all) of the visible colonies are surrounded by a circle (MRSA bacteria) or a rectangle (MRSE), as appropriate. This should assist in analysis of the slides where black and white copies of the Figures are provided in lieu of color copies. In Image A the MRSA bacteria (in circles) are orange in color and the MRSE bacteria (in rectangles) are green. In Image B, no MRSA bacteria are visible but the MRSE bacteria (in rectangles) are blue. In Image C, both MRSA (in circles) and MRSE bacteria (in rectangles) are green in color.

### Results:

The results of this Example are discussed with reference to Fig. 3. In Image A (dual band filter) both orange stained bacteria and green stained bacteria are visible. In this image, the MRSA appear orange due to combined green fluorescence of Probes 1-5 (Probes 1-5 labeled with Flu) and red fluorescence of Probe 8 (rRNA-directed S. aureus-specific probes labeled with TAMRA). The MRSE bacteria are only green because they are not *S. aureus* (i.e. no TAMRA signal). This image (in combination with Image C) suggests that all visible bacteria are methicillin-resistant.

In Image B, a DAPI (blue) filter was used. Blue stained MRSE bacteria are visible in this image because Probe 9 is labeled with Pacific Blue and the probes target rRNA of *S. epidermidis.*

In Image C, a FITC (green) filter was used. Both MRSA and MRSE bacteria appeared in this image with green fluorescent stain. Both species of bacteria were stained green.

As noted above, Images A-C were of the same sample and approximately the same viewing field. Thus, it is possible to compare bacteria in each Image to directly determine whether or not they are visible in another image. This permits easy (and potentially automated) determinations of the bacteria and their traits based on simple visual analysis.

### Example 4: Enhanced Determination of MRSA using PNA FISH and Tyramide Signal Amplification (TSA).

### Fluorescence in situ hybridization and fluorescence microscopy

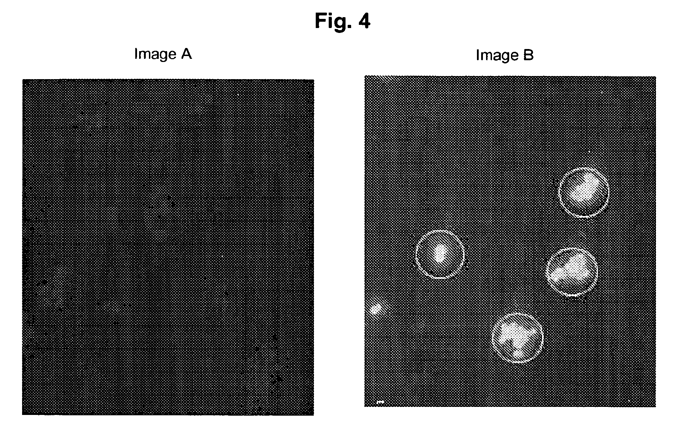

The growth and oxacillin induction of MRSA 33591 cells was performed as described in Example 1. Cell fixation and permeabilization was also performed as described in Example 1 except that prior to hybridization the slides were treated at 80°C for 5 minutes with 75 µL of TE buffer (Tris-HCl 100mM, EDTA, 10 mM, pH 8.0). For hybridization, 25 µL of HB** containing five biotin-labeled PNA probes (Probes 11, 12, 13, 14 and 15 (concentration of each probe was 100 nM for a total concentration of 500 nM) was applied on one slide. Another slide was probed with one biotin-labeled, *C*. *albicans*-specific rRNA-directed probe (probe 10, concentration 500 nM) in control experiment. Hybridization and washing were performed as described in Example 1. Slides were then processed with reagents commercially available from Molecular Probes (TSA Kit #25, Eugene, OR) according to manufacturer's protocol. Specifically, slide-deposited cells were first incubated 30 min with 0.075 mL of Blocking Buffer (BB) then 30 min with 0.1 mL of streptavidin-HRP diluted in BB. After washing (3x5 min wash) with PBS-Tween, the cells were incubated 10 min with 0.075 mL of AlexaFluor 594-labeled tyramide solution and washed (3x5 min wash) again. A drop of MM and cover slip was then applied on air dried slides and the cells were examined by FM. Specific conditions for each of the slides examined in Fig. 4, are as follows:

A) MRSA 33591 with 1 rRNA-directed *C. albicans*-specific biotin labeled PNA probe (Probe 10)

B) MRSA 33591 with 5 biotin labeled PNA probes (Probes 11-15)

** For this example only, the Sigma P/N R5636 (1 ml), ribonucleic acid, transfer, from bakers yeast and Trevigen, P/N 9600-5-D, calf thymus DNA were diluted (20x) into the hybridization buffer.

### Results:

The results of this Example are discussed with reference to Fig. 4. Some (but not all) of the visible colonies in the images are surrounded by a circle (Image B). This should assist in analysis of the slides where black and white copies of the Figures are provided in lieu of color copies.

The intense red fluorescent stained bacteria in Image B demonstrate that indirect determination of the biotin label coupled with signal amplification can be used with Probes 11-15. The sample in Image A was a control using a rRNA-directed probe to a bacteria not present in the sample. Thus, the absence of visible bacteria in Image A (by comparison with the result of Image B) suggests that MecA detection was specific and the signal amplified properly.

### Appendix I

***Description of bacterial* strains:**
S. aureus ATCC 43300 (MRSA 43300); a methicillin-resistant strain
S. aureus ATCC 29213 (MSSA 29213); a methicillin-sensitive strain
S. epidermidis ATCC 51625 (MRSE 51625); a methicillin-resistant strain
S. aureus ATCC 33591 (MRSA 33591); a methicillin-resistant strain

[ATCC stands for American Type Culture Collections and is a source for various organisms - See: See the worldwide web at: atcc.org/CulturesandProducts/Microbiology/BacteriaandPhages/tabid/176/Default.aspx]

### Composition of buffers/solutions:

**Hybridization buffer (HB):** 50 mM Tris-HCl pH=7.6; 0.1% (w/v) Sodium Pyrophosphate; 10 mM NaCl; 5 mM ethylenediaminetetraacetic acid (EDTA), 10% Dextran Sulfate MW 500,000; 0.2% (w/v) Ficoll 400 K; 30% Formamide; 1 % (v/v) Triton X-100; 0.2% (w/v) Polyvinyl Pyrrolidone MW 360 000; water adjust to 100%.

**Wash Buffer (WB):** 5 mM Tris-HCl pH=10.0; 15 mM NaCl; 0.1% (v/v) Triton X-100; water adjust to 100%.

**RNase-free Permeabilization Solution (PS):** 50 mM Tris-HCl pH=7.6; 5 mM MgSO₄; 0.1 mg/ml lysostaphin; 5 mM TCEP (Product # 77720 from Thermo Scientific, Rockford, II). Solution was prepared fresh. Before use, it was heated for 15 min at 65 °C and cooled to RT.

**10% Paraformaldehyde (PAF):** 5 grams of paraformaldehyde was dispersed in 25 ml of deionized water. After addition of 2 ml of 2M NaOH, the solution was incubated at 55 °C until all paraformaldehyde powder was dissolved (with occasional manual agitation). Solution was then made 1 x PBS by adding 10 ml of 10x PBS concentrate (Product of Sigma-Aldrich, St. Louis, MO, P/N P7059) and quantity sufficient to bring total volume up to 50 ml. The pH of the solution was adjusted to 3.5 with 2M HCl.

**Blocking Buffer (BB):** As provided in TSA Kit #25 from Molecular Probes, Eugene, OR.

**Mounting Media (MM):** AdvanDx, Inc., Woburn, MA; product number CP 0023.

### PNA Probes:

PNA Probes were obtained from Panagene, Daejeon, Korea. All probes (except Probe 7) comprise a single label (see Table 1 for the label type). Table 1 lists attributes of the PNA probes used. All PNA probes (including additional PNA probes listed in other Tables disclosed herein) were prepared by chemical de novo methods (not by transcription).

**Table 1**

| **SEQ ID NO: Probe No.:** | **Label** | **Nucleobase Sequence** | **Select Bacteria or Select Trait** |
|---|---|---|---|
| 1 | Flu | GTATTTCTGAAGACTA | MR |
| 2 | Flu | GCTATCGTGTCACAA | MR |
| 3 | Flu | GCTCCAACATGAAGAT | MR |
| 4 | Flu | GATGATGCAGTTATTG | MR |
| 5 | Flu | GATGATACCTTCGTT | MR |
| 6 | Flu | TCAATGAGCAAAGGT | E. coli |
| 7 | Biotin | GCTATCGTGTCACAA | MR |
| 8 | TAMRA | GCTTCTCGTCCGTTC | S. aureus |
| 9 | Pacific Blue | TCCTCGTCTGTTCGC | S. epidermidis |
| 10 | Biotin | AGAGAGCAGCATCCA | C. albicans |
| 11 | Biotin | GTATTTCTGAAGACTA | MR |
| 12 | Biotin | GCTATCGTGTCACAA | MR |
| 13 | Biotin | GCTCCAACATGAAGAT | MR |
| 14 | Biotin | GATGATGCAGTTATTG | MR |
| 15 | Biotin | GATGATACCTTCGTT | MR |

Abbreviations used: Flu = fluorescein, MR = methicillin-resistance. All labels were linked to the N-terminus of the probe through a 8-amino-3,6-dioxaoctanoic acid linker (sometimes referred to in the scientific literature as the O-linker or simply as "O"). Pacific Blue is available as a reactive N-succinimidyl ester from Invitrogen, Carlsbad, CA.

### Fluorescence Microscopy (FM):

Olympus System Microscope BX 51 equipped with DP 70 Microscope Digital Camera was used for all microscopical examinations. The camera uses Windows XP/2000/NT 4.0 operating system for running Olympus DP 70 software. Images were taken with a 60x (UPlanFI) oil immersion objective. Omega Optical filters XF53, XF202, XF06 and XF102-2 were used for visualizing fluorescent signals of FITC/Texas Red (dual band), FITC for fluorescein (single band), DAPI (single band) and Texas red or TAMRA (single band), respectively. One-two second and ∼1/10 second exposures were required for visualization of other probes and rRNA-directed signals, respectively.

**Note:** Examples 5-10 were all performed without enzymatic treatments intended to permeabilize the gram-positive bacterial cells (i.e. without a cell permeabilization step)

### Example 5: Determination of MRSA-Specific MecA mRNA by PNA FISH in S. aureus

Unless otherwise noted, all procedures were performed at room temperature (RT). The composition of Fixation Solution (FS), Hybridization Buffer (HB2), and Wash Buffer (WB), Mounting Media (MM) as well as a description of various bacterial strains (e.g. staphylococcal chromosome cassette *mec* (SCC*mec*) type I-V and other staphylococcus strain), PNA probes and fluorescent microscopy (FM) used in this Example 5 is provided in the Appendix II (below) or in Appendix I (above) for reagents that are common to all of Examples 1-5.

### Cell growth and fixation:

S. aureus bacteria were inoculated into Tryptic Soy Broth (TSB) or blood culture bottles and grown overnight (16-18 hours) at 35-37 °C with shaking, diluted 1:9 in prewarmed TSB and grown for another 1.5 hr (OD_{600 nm} = 0.5). This preculture was induced with cefoxitin 3 µg/mL with shaking at 35-37 °C for another 40 min. Cefoxitin-induced cells were added on glass slides (20 µL) and heat fixed with Fixation Solution (FS) for 20 minutes (min) at 80 °C. After heat fixation the slides were immersed in 100% methanol for 5 min and left to air-dry for approximately 5 min.

### Fluorescence in situ hybridization and fluorescence microscopy:

25 µL of Hybridization Buffer 2 (HB2) containing PNA Probes (See below for probe concentration) was then applied on slide in a PNA FISH Workstation (AdvanDx, Part No: AC005) and covered with a cover slip. The cells were then hybridized for 2 hours at 55°C. After the hybridization, the slides were washed with Wash Buffer (WB) for 30 min at 55°C. A drop of Mounting Media (MM) and a cover slip was applied on the dried specimen and slide-deposited cells were then examined by according to the procedure labeled Fluorescence Microscopy 2 (FM2).

### Probe concentrations:

A mixture of six fluorescein-labeled, mecA mRNA-directed PNA probes (Probes 16, 17, 18, 19, 20 and 21 (See: Table 3, below)) at concentrations of 500 nM for each of the probes; combined concentration of probes was 3000 nM.

### Results:

The results for this Example are summarized in Table 2. For the PNA FISH assay using m-RNA-directed Probes 16-21, microscope analysis showed green fluorescent bacteria (Positive) for methicillin-resistant *Staphylococcus aureus* (MRSA). These were recorded as positive (if green bacteria were observed) or negative. MRSA strains comprise target mRNA associated with the mecA gene as this gene is known to be associated with methicillin-resistance. Methicillin-susceptible S.aureus (MSSA) which does not contain the mecA gene and had no signal observed (Negative). The mecA gene, is located on a 21- to 67-kb genomic island called staphylococcal chromosome cassette (*SCCmec*). *SCCmec* type I, type II and type III (hospital-acquired MRSA) and CA-MRSA (community acquired MRSA and SCCmec types IV and V) were represented. Strains known to be *SCCmec* types I, II, I II, IV and V were all positive by this procedure. Strains known to be hetero- or homogeneous, i.e. S. *aureus* ATCC 43300 (heterogeneous oxacillin resistance), *SCCmec* II) & S. *aureus* ATCC 33591 (homogeneous oxacillin resistance), *SCCmec* III) were both detected by this procedure. The data obtained using the commercially available *mecA* EVIGENE^{®} product (a cell-free assay) were consistent with both: 1) the known properties of the isolates; and 2) the results obtained with the whole-cell FISH assay performed as discussed above.

**Table 2**

| **Specie** | **ID** | ***SCCmec*** | **EVIGENE *¹mecA*** | **²MIC (µg/mL)** | **PNA FISH** mecA |
|---|---|---|---|---|---|
| *S.aureus* (MRSA) | Reference (Clinical COL) | type I | Positive | ≥256 | Positive |
| | Reference (Clinical EU) | type Ia | Positive | ≥256 | Positive |
| | Reference (NCTC10422) | type Ib | Positive | ≥256 | Positive |
| | Reference (ATCC 43300) | type II | Positive | 128 | Positive |
| | Reference (ATCC 33591) | type III | Positive | ≥256 | Positive |
| | | | | ≥256 | |
| | Reference | type IIIa | Positive | | Positive |
| | (DK, SSI) | | | | |
| | Reference (USA500) | type IV | Positive | 48 | Positive |
| | Reference (USA300) | type IV | Positive | 48 | Positive |
| | Reference (DK, SSI) | type V | Positive | 12 | Positive |
| *S.aureus* (MSSA) | Reference (ATCC 11632) | NA | Negative | 3 | Negative |
| | Reference (ATCC 25923) | NA | Negative | 3 | Negative |
| | Reference (ATCC 29213) | NA | Negative | 4 | Negative |

| | | | | | |
|---|---|---|---|---|---|
| The strains were tested for the present of the mecA *gene* in mecA EVIGENE^{®} (AdvanDx, Part No KT102-96). This assay is intended for identification of methicillin-resistant staphylococci by detection of the mecA gene in a cell-free assay. ² Susceptibility testing was done with cefoxitin Etest® strips (bioMerieux, Part No 541000658). Breakpoints for categorization of the susceptibility of S. *aureus* (S: ≤ 4 µg/ml and R: ≥ 8 µg/ml) according to Clinical and Laboratory Standards Institute (CLSI). NA = Not applicable. | | | | | |

### Appendix II

***Description of bacterial strains:***
*S. aureus* COL (MRSA COL); SCCmec I; a methicillin-resistant strain
*S. aureus* EU (MRSA EU); SCCmec la; a methicillin-resistant strain
*S. aureus* NCTC 10422 (MRSA 10422); SCCmec Ib; a methicillin-resistant strain
*S*. *aureus* ATCC 43300 (MRSA 43300); SCCmec II; a methicillin-resistant strain
*S*. *aureus* ATCC 33591 (MRSA 33591); SCCmec III; a methicillin-resistant strain
*S. aureus* DK (MRSA DK); SCCmec IIIa; a methicillin-resistant strain
S. aureus USA500 (MRSA USA500); SCCmec IV; a methicillin-resistant strain
S. aureus USA300 (MRSA USA300); SCCmec IV; a methicillin-resistant strain
*S*. aureus DK (MRSA DK); SCCmec V; a methicillin-resistant strain
*S*. *aureus* ATCC 11632 (MSSA 11632); a methicillin-sensitive strain
*S*. *aureus* ATCC 25923 (MSSA 25923); a methicillin-sensitive strain
*S*. *aureus* ATCC 29213 (MSSA 29213); a methicillin-sensitive strain

[ATCC stands for American Type Culture Collections and is a source for various organisms - See the worldwide web at: atcc.org/CulturesandProducts/Microbiology /BacteriaandPhages/tabid/176/Default.aspx. NCTC stands for National collection of Type Cultures and is a source for various organisms - See the worldwide web at: ukncc.co.uk/index.htm]

### Composition of buffers:

**Hybridization Buffer 2 (HB2)**: 50 mM Tris-HCl pH=7.5; 0.1% (w/v) Sodium Pyrophosphate; 10 mM NaCl; 5 mM ethylenediaminetetraacetic acid (EDTA), 10% Dextran Sulfate MW 500,000; 0.2% (w/v) Ficoll 400 K; 30% Formamide; 1% (v/v) Triton X-100; 0.2% (w/v) Polyvinyl Pyrrolidone MW 360 000; water adjust to 100%.

**Fixation Solution (FS)**: An aqueous solution of: 7 mM Na₂HPO₄, 7 mM NaH₂PO₄, 130nM NaCl, 1% (v/v) Triton X-100 and 0.05% (v/v) ProClin 300.

### PNA Probes:

PNA Probes were obtained from Panagene, Daejeon, Korea. All probes comprise a double label (see Table 3 for the label type). Table 3 lists attributes of the PNA probes used in Example 5.

**Table 3**

| **SEQ ID NO:/Probe No.** | **Probe Configuration & Nucleobase Sequence** | **Target** | **Select Trait** |
|---|---|---|---|
| 16 | Flu-OO-CACATTGTTTCGGTCT-OO-Lys(Flu) | mRNA) | mecA |
| 17 | Flu-OO-CATTAGTTGTAAGATG-OO-Lys(Flu) | mRNA | mecA |
| 18 | Flu-OO-TCTTCAGAGTTAATGG-OO-Lys(Flu) | mRNA | mecA |
| 19 | Flu-OO-GCTATTATCGTCAACG-OO-Lys(Flu) | mRNA | mecA |
| 20 | Flu-OO-AGCATCAA TAGTTAG-OO-Lys(Flu) | mRNA | mecA |
| 21 | Flu-OO-TGTGCTTACAAGTGC-OO-Lys(Flu) | mRNA | mecA |

| | | | |
|---|---|---|---|
| Abbreviations used: Flu = fluorescein, Lys = Lysine. All labels were linked to the C-terminus and the N-terminus of the probe through two 8-amino-3,6-dioxaoctanoic acid linker (sometimes referred to in the scientific literature as the O-linker). The Flu label was attached to the C-terminus of the probe via the amine group of the lysine side chain. | | | |

### Fluorescence Microscopy 2 (FM2):

Olympus System Microscope BX 51 equipped with DP 70 Microscope Digital Camera was used for all microscopical examinations. The camera uses Windows XP/2000/NT 4.0 operating system for running Olympus DP 70 software. Images were taken with a 60x (UPlanSApo) oil immersion objective. Omega Optical filters XF53 was used for visualizing fluorescent signals of FITC/Texas Red (dual band).

### Example 6: Determination of mecA mRNA Expression bv mRNA-Directed PNA FISH Using Bacteria Isolates Spiked into Blood Culture

An evaluation of mecA detection was performed on 172 reference and clinical isolates (Listed in Table 15, below) that were mixed with/spiked into blood culture. The study included 127 MRSA strains (reference and clinical isolates). Among the reference strains were strains with **S**taphylococcal **C**hromosome **C**assette (SCCmec) Ia, Ib, II and type III, IIIa (Hospital-acquired MRSA), CA-MRSA (community acquired MRSA, SCCmec IV and V). There were also 15 methicillin-susceptible *S.aureus* (MSSA) (reference and clinical isolates), 25 methicillin-resistant coagulase-negative staphylococci (MR-CNS) and five methicillin-susceptible CNS (MS-CNS, S. *epidermidis, S.warneri, S.capitis, S. haemolyticus and S.hominis*) included in the study. All isolates were tested for the presence of the mecA gene using mecA EVIGENE^{®} (AdvanDx, Part No: KT102-96). The product, mecA EVIGENE^{®}, is intended for identification of methicillin-resistant staphylococci by detection of the mecA gene in bacteria of samples of interest (but not in a whole-cell assay format). Susceptibility testing was done with cefoxitin Etest® strips (bioMérieux, Part No 541000658) for 57 isolates. Testing using these commercial products was performed according to the vendors instructions. Breakpoints for categorization of the susceptibility of *S*. *aureus* (S: ≤ 4 µg/ml and R: ≥ 8 µg/ml) according to CLSI. Unless otherwise noted, all procedures were performed at room temperature (RT). The composition of Fixation Solution (FS), Hybridization Buffer (HB2), and Wash Buffer (WB), Mounting Media (MM) as well as PNA probes and fluorescent microscopy (FM) used in this Example 6 is provided in Appendix, I, Appendix II or in Appendix III (below).

### Cell growth and fixation:

1-2 colonies from each strain were inoculated into negative blood culture bottles, grown overnight (16-18 hours) at 35±2°C with shaking, diluted 1:9 in prewarmed TSB and grown for another 1.5 hr. This preculture was induced with cefoxitin 3 µg/mL with shaking at 35±2°C for another 40 min. Cefoxitin-induced cultures were added on glass slides (20 µL) and heat fixed with Fixation Solution (FS) for 20 minutes (min) at 80 °C. After heat fixation the slides were immersed in 100% methanol for 5 min and left to air-dry for approximately 5 min.

### Fluorescence in situ hybridization and fluorescence microscopy:

For samples that weren't treated with commercial products, 25 µL of Hybridization Buffer (HB2) containing PNA Probes (See below for probe concentration) was applied on the slide in a hybridization chamber and covered with a cover slip. The cells were hybridized for 1.5 hours at 55°C. After hybridization, the slides were washed with Wash Buffer (WB) for 30 min at 55°C. A drop of Mounting Media (MM) and a cover slip was applied on the dried specimen and slide-deposited cells were examined according to the procedure labeled Fluorescence Microscopy 2 (FM2).

### Probes and Probe concentrations:

A mixture of 8 to 11 fluorescein-labeled, mecA mRNA-directed PNA probes (See Table 4, below) at concentrations of 500 nM for each of the probes was used in the hybridization buffer. PNA probes used in this Example 6 are listed in Table 4. PNA Probes were obtained from Panagene, Daejeon, Korea. Table 4 lists attributes of the PNA probes used in this Example 6.

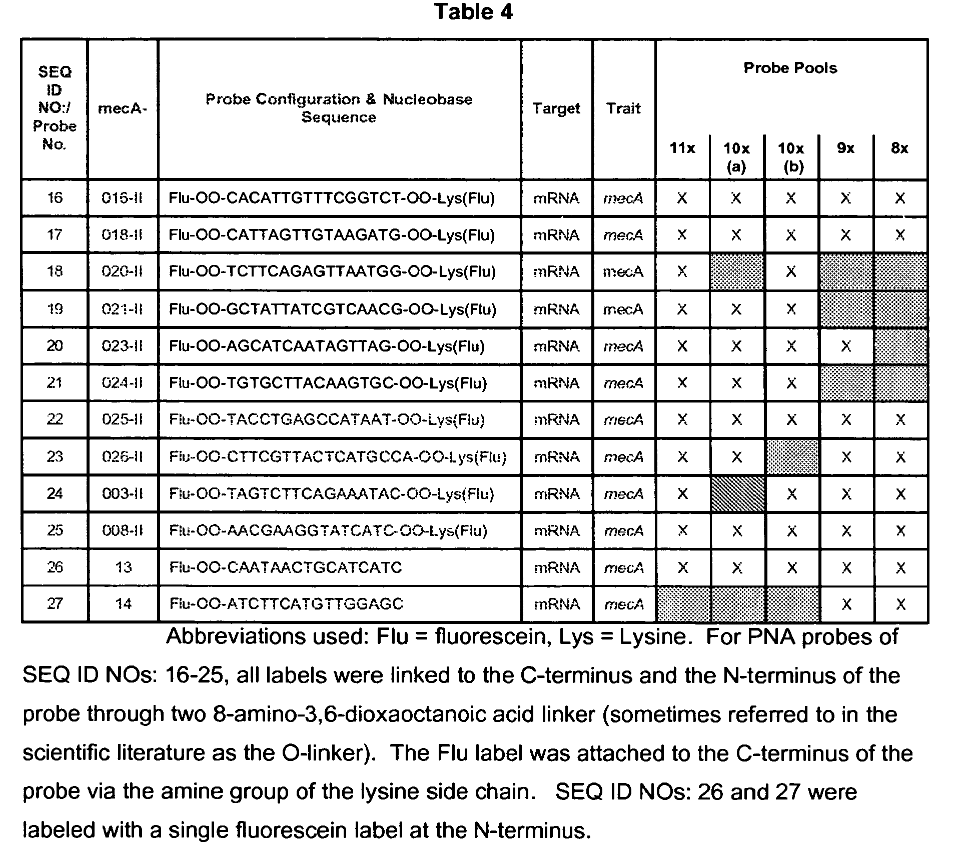

### Probe Pool Compositions:

8x Pool contained Probes 16, 17 & 22-27

9x Pool contained Probes 16, 17, 20 & 22-27

10x(a) Pool contained Probes 16, 17, 19-23 & 25-26

10x(b) Pool contained Probes 16-22 & 24-26

11x Pool contained Probes 16-26

### Results:

Table 5 summarizes the data obtained by performing this Example. Detection of *mecA* gene expression by FISH using m-RNA-directed PNA Probes 16-27 in spiked blood culture bottles were compared to results obtained by a phenotypic method (Etest® strips, bioMérieux) and detection of the mecA gene by mecA EVIGENE® (a cell-free assay; AdvanDx, Inc., Woburn, MA). The FISH assay demonstrated 100% sensitivity (127/127) for MRSA and 88% (22/25) sensitivity for MR-CNS; as three samples (out of 25 MR-CNS tested strains) were not detected. No false positive was detected as all MSSA and MS-CNS strains tested had no signal observed (Negative). Determination of cefoxitin minimal inhibitory concentration (MIC) by Etest® strips showed that the tested MRSA strains had MIC between 12 to ≥ 256 and were all detected.

The signal intensity with 8 PNA probes (Probes 16-17, 22-27) was enough to detect most MRSA strains (73 out of 127) however for some MRSA strains and particularly most MR-CNS strains a set of all 11 probes was required for mecA mRNA determination. A total of 73 MRSA strains tested positive with a pool of 8 mecA mRNA-directed PNA probes, 6 MRSA strains with a pool of 9 mecA mRNA-directed PNA probes, 7 MRSA strains with a pool of 10(a) mecA mRNA-directed PNA probes and 41 MRSA strains with a 11-probe pool. For the MR-CNS stains tested, only seven strains were positive with a 8-probe pool, one strain was positive with a 9-probe pool and 17 strains were positive with a 11-probe pool. MSSA strains were all negative with the 8-, 9- and 11-probe pools. The MS-CNS strains tested were negative with a 10(b)-probe pool of mecA mRNA-directed PNA probes. These data demonstrate that determination can be strain dependent and it is possible to adjust the m-RNA-directed probe components of the assay to thereby produced an accurate result across various strains or bacteria.

**Table 5**

| **Sample type** | **MIC (µg/ml) Detest®** | **EVIGENE®** *mecA* | **PNA FISH** *mecA* **(Probes 16-27)** |
|---|---|---|---|
| MSSA *(n* = 15) | 3 to 4 | Negative (15/15) | Negative (15/15) |
| MRSA (*n* = 127) | 12 to ≥ 256 | Positive (127/127) | Positive (127/127) |
| MR-CNS (*n* = 25) | 32 to ≥ 256 | Positive (25/25) | Positive (22/25) |
| MS-CNS (*n* = 5) | 1.5 | Negative (5/5) | Negative (5/5) |

| | | | |
|---|---|---|---|
| Note: Etest® is a registered trademark of AB bioMérieux, Dalvägen, Sweden EVIGENE® is a registered trademark of Statens Serum Institute, Copenhagen, Denmark | | | |

### Example 7: Analysis of Isolates Giving Discrepant Results in the BD GeneOhm™ StaphSR Assay

In this study, we examined five isolates which, according to a literature report (Gröbner *et al*. (2009)), gave discrepant results when using the commercially available BD GeneOhm™ StaphSR assay due to the presence of methicillin-susceptible, revertant MRSA strains (*n* = 3) and MRSA strains that were not detected by this BD GeneOhm StaphSR assay (*n* = 2) when compared to other real-time PCR-based molecular approaches and to conventional standard laboratory methods (Vitek2, bioMérieux). The BD GeneOhm StaphSR assay determines an *S. aureus*-specific target sequence and a specific target near the staphylococcal cassette chromosome *mec* (*SCCmec*) insertion site and the *orfX* junction in MRSA to thereby differentiate between MSSA and MRSA (Gröbner *et al.* 2009).

The five strains were also tested for the presence of *S*. *aureus* (16S rRNA) with *S. aureus*/CNS PNA FISH^{®} (AdvanDx, Part No: KT005) according to the manufactures instructions. Furthermore, a determination of the presence of the mecA gene was confirmed by mecA EVIGENE^{®} (AdvanDx, Part No: KT102-96). Unless otherwise noted, all procedures were performed at room temperature (RT). The composition of Fixation Solution (FS), Hybridization Buffer (HB2), and Wash Buffer (WB), Mounting Media (MM) as well as a description of various bacterial strains, PNA probes and fluorescent microscopy (FM) used in this Example 7 is provided in Appendix I, Appendix II or Appendix III.

Note: BD GeneOhm™ is a trademark of BD Worldwide.
PNA FISH® is a registered trademark of AdvanDx, Inc., Woburn, MA

### Cell growth and fixation:

*S. aureus* bacteria were inoculated into negative blood culture, grown overnight (16-18 hours) at 35-±2°C in BacT/ALERT (bioMérieux), diluted 1:2 in prewarmed TSB with cefoxitin (end conc. 3 µg/mL) and incubated with shaking at 35-±2°C for 40 min. Cefoxitin-induced cells were added (5 µL) on glass slides and heat fixed with Fixation Solution (FS) for 2 minutes at 80 °C. After heat fixation, the slides were immersed in 100% methanol for 5 min and left to air-dry for approximately 5 min.

### Fluorescence in situ hybridization and fluorescence microscopy:

For samples not treated with commercial products, 25 µL of Hybridization Buffer 2 (HB2) containing PNA Probes (See below for probe concentration) was applied on the slide in a PNA FISH Workstation and covered with a cover slip. The cells were hybridized for 0.5 hours at 55°C. After hybridization, the slides were washed with Wash Buffer (WB) for 30 min at 55°C. A drop of Mounting Media (MM) with DAPI (3.3 ng/mL) and a cover slip was applied on the dried specimen and slide-deposited cells were examined according to the procedure labeled Fluorescence Microscopy 2 (FM2).

### PNA Probes & Probe concentrations:

PNA Probes used in this Example 7 were obtained from Panagene, Daejeon, Korea. Table 6 lists attributes of the PNA probes used in this Example 7.

**Table 6**

| **SEQ ID NO:/Probe No.** | **mecA-** | **Probe Configuration & Nucleobase Sequence** | **Target** | **Select Bacteria or Select Trait** |
|---|---|---|---|---|
| 16 | 016-II | Flu-OO-CACATTGTTTCGGTCT-OO-Lys(Flu) | mRNA | *mecA* |
| 17 | 018-II | Flu-OO-CATTAGTTGTAAGATG-OO-Lys(Flu) | mRNA | *mecA* |
| 18 | 020-II | Flu-OO-TCTTCAGAGTTAATGG430-Lys(Flu) | mRNA | *mecA* |
| 19 | 021-II | Flu-OO-GCTATTATCGTCAACG-OO-Lys(Flu) | mRNA | *mecA* |
| 20 | 023-II | Flu-OO-AGCATCAATAGTTAG-OO-Lys(Flu) | mRNA | *mecA* |
| 21 | 024-II | Flu-OO-TGTGCTTACAAGTGC-OO-Lys(Flu) | mRNA | *mecA* |
| 22 | 025-II | Flu-OO-TACCTGAGCCATAAT-OO-Lys(Flu) | mRNA | *mecA* |
| 24 | 003-II | Flu-OO-TAGTCTTCAGAAATAC-OO-Lys(Flu) | mRNA | *mecA* |
| 25 | 008-II | Flu-OO-AACGAAGGTATCATC-OO-Lys(Flu) | mRNA | *mecA* |
| 26 | 13 | Flu-OO-CAATAACTGCATCATC | mRNA | *mecA* |
| 28 | 014-II | Flu-OO-ATCTTCATGTTGGAGC-OO-Lys(Flu) | mRNA | *mecA* |
| 29 | 017-II | Flu-OO-ACGATGCCTATCTCAT-OO-Lys(Flu) | mRNA | *mecA* |
| 30 | 19 | Flu-OO-GATAGTTACGACTTTC | mRNA | *mecA* |
| 31 | 022-II | Flu-OO-ATGTATGTGCGATTGT-OO-Lys(Flu) | mRNA | *meCA* |
| 32 | 028-II | Flu-OO-GATCAATGTTACCGTA-OO-Lys(Flu) | mRNA | *mecA* |
| 33 | 029-II | Flu-OO-CGCTATGATCCCAATC-OO-Lys(Flu) | mRNA | *mecA* |
| 8 | Sta16S03 | TAM-GCTTCTCGTCCGTTC | rRNA | *S.aureus* |

All abbreviations used in this Table have previously been defined. For Probes of SEQ ID NOs 16-25, 28-29 and 31-33 all labels were linked to the C-terminus and the N-terminus of the probe through two 8-amino-3,6-dioxaoctanoic acid linker (sometimes referred to in the scientific literature as the O-linker). The Flu label was attached to the C-terminus of the probe via the amine group of the lysine side chain. Probes of SEQ ID NOs: 26 and 30, a single Flu label was used. For the Probe of SEQ ID NO: 8, a single TAMRA label was used (No O-linkers were used to link the TAMRA label to the probe).

A mixture of 16 fluorescein-labeled, mecA mRNA-directed PNA probes (See Table 6, SEQ ID NOs: 16-22, 24-26, 28-33) at concentrations of 500 nM for each of the probes in HB2 was used to determine whether or not the bacteria in the sample were methicillin-resistant. To determine *S. aureus* bacteria in the sample, one TAMRA-labeled, *S. aureus*-specific, rRNA-directed probe (SEQ ID NO: 8) was used at a concentration of 14 nM in HB2.

### Results:

With reference to Table 7, when using the 16 mRNA-directed mecA PNA probes (i.e. SEQ ID NOs: 16-22, 24-26, 28-33), in combination with the *S. aureus* rRNA-directed probe (SEQ ID NO: 8), in a FISH format on revertant MRSA strains (in which a part including the mecA gene has been deleted from the *SCCmec* gene cassette) and SCCmec types reported to be missed in rapid molecular diagnostic tests (Gröbner *et al.* 2009), the results with our assay showed 100% accuracy. The results with our new assay were also consistent with independent results obtained using the AdvanDx' mecA EVIGENE^{®} commercial product and the *S. aureus*/CNS PNA FISH^{®} commercial product. Thus, the data in this Example demonstrates that a mixture of the mRNA-directed PNA probes, in combination with a rRNA-directed probe that determines *S*. *aureus* bacteria (comprising a "bacteria-directed" probe), can be used to accurately distinguish MRSA and MSSA bacteria that are not distinguishable using certain other commercial products.

**Table 7**

| **Strain No.** | **Isolate type** | **Real-time PCR result** (*Gröbner et al*. *2009*) | | **PNA FISH®** | **EVIGENE®** | **PNA FISH##** |
|---|---|---|---|---|---|---|
| | | BD GeneOhm *S.aureus*/*SCCmec* | PCR *sa442*/*mecA* | *S.aureus*/ *CNS* | *mecA* | *S.aureus*/ *mecA* |
| 1481 | MSSA | +/+ (MRSA) | +/(MSSA) | +/(*S.aureus*) | Negative | +/(MSSA) |
| 1482 | MSSA | +/+ (MRSA) | +/(MSSA) | +/(*S.aureus*) | Negative | +/(MSSA) |
| 1483 | MSSA | +/+ (MRSA) | +/(MSSA) | +/(*S.aureus*) | Negative | +/(MSSA) |
| 1484 | MRSA | -/+ (*S. aureus*) | +/+ (MRSA) | +/*(S. aureus)* | Positive | +/+ (MRSA) |
| 1485 | MRSA | -/+ (*S. aureus*) | +/+ (MRSA) | +/(*S. aureus*) | Positive | +/+ (MRSA) |

| | | | | | | |
|---|---|---|---|---|---|---|
| ## with mixture of Probes 16-22, 24-26, 28-30 and 8 | | | | | | |

### Appendix III

***Description of bacterial strains*:**
*# 1481, S*. *aureus,* Gröbner *et al*. 2009 strain #13; (MSSA); a methicillin-sensitive strain
*# 1482, S*. *aureus,* Gröbner *et al.* 2009 strain #30; (MSSA); a methicillin-sensitive strain
#*1483, S. aureus,* Gröbner *et al.* 2009 strain #114; (MSSA); a methicillin-sensitive strain
*#1484, S*. *aureus,* Gröbner *et al*. 2009 strain #35; (MRSA) SCCmec II; a methicillin-resistant strain
#1485, *S. aureus,* Gröbner *et al.* 2009 strain #126; (MRSA) SCCmec II; a methicillin-resistant strain

### Example 8: Analysis of Isolates in TSB with or without Cefoxitin Induction

The induction and detection of mecA expression were performed at log-phase cultures optical density at 600 nm (OD_{600 nm} = 0.5) on uninduced and cefoxitin-induced cells. Induced and uninduced samples were likewise examined with AdvanDx' commercial *S*. *aureus*/*CNS* PNA FISH^{®} product and mecA EVIGENE^{®} product as discussed in Example 7, above. Susceptibility testing was performed with cefoxitin Etest® strips (bioMérieux, Part No 541000658) according to the manufacturers instructions.

Unless otherwise noted, all procedures were performed at room temperature (RT). The composition of Fixation Solution (FS), Hybridization Buffer (HB2), and Wash Buffer (WB), Mounting Media (MM) as well as a description of various bacterial strains, PNA probes and fluorescent microscopy (FM) used in this Example 8 is provided in Appendix I, Appendix II or Appendix IV (below).

### Cell growth and fixation:

*S. aureus* bacteria were inoculated into Tryptic Soy Broth (TSB) grown overnight (16-18 hours) at 35-37 °C with shaking, diluted to OD₆₀₀ₙₘ = 0.25 in prewarmed TSB and grown to OD_{600 nm} = 0.5. The preculture was split in two cultures; an induced (with cefoxitin 3 µg/mL) and an uninduced (with cefoxitin 0 µg/mL) culture. Both cultures were grown for another 40 min with shaking at 35-37 °C before preparation of smears. Cefoxitin-induced, and -uninduced cells, were added on glass slides (20 µL) and heat fixed with Fixation Solution (FS) for 20 min at 80 °C. After heat fixation, the slides were immersed in 100% methanol for 5 min and left to air-dry for approximately 5 min.

### Fluorescence in situ hybridization and fluorescence microscopy:

This procedure was performed on both the Cefoxitin-induced, and - uninduced cells as described in Example 7, above.

### PNA Probes & Probe concentrations:

PNA Probes were obtained from Panagene, Daejeon, Korea. The sequence and other aspects of probes used in this Example 8 are listed in Table 9.

**Table 9**

| **SEQ ID NO:/Probe No.** | **Name** | **Probe Configuration & Nucleobase Sequence** | **Target Type** |
|---|---|---|---|
| 16 | 016-II | Flu-OO-CACATTGTTTCGGTCT-OO-Lys(Flu) | mRNA |
| 17 | 018-II | Flu-OO-CATTAGTTGTAAGATG-OO-Lys(Flu) | mRNA |
| 18 | 020-II | Flu-OO-TCTTCAGAGTTAATGG-OO-Lys(Flu) | mRNA |
| 19 | 021-II | Flu-OO-GCTATTATCGTCAACG-OO-Lys(Flu) | mRNA |
| 20 | 023-II | Flu-OO-AGCATCAATAGTTAG-OO-Lys(Flu) | mRNA |
| 21 | 024-II | Flu-OO-TGTGCTTACAAGTGC-OO-Lys(Flu) | mRNA |
| 22 | 025-II | Flu-OO-TACCTGAGCCATAAT-OO-Lys(Flu) | mRNA |
| 23 | 026-II | Flu-OO-CTTCGTTACTCATGCCA-OO-Lys(Flu) | mRNA |
| 24 | 003-II | Flu-OO-TAGTCTTCAGAAATAC-OO-Lys(Flu) | mRNA |
| 25 | 008-II | Flu-OO-AACGAAGGTATCATC-OO-lys(Flu) | mRNA |
| 26 | 13 | Flu-OO-CAATAACTGCATCATC | mRNA |

All abbreviations used in this Table have previously been defined. A mixture of the 11 fluorescein-labeled mecA mRNA-directed PNA probes listed in Table 9, at concentrations of 500 nM for each of the probes, was used in HB2. The combined PNA probe concentration was therefore 5500 mM.

### Results:

The results of this Example 8 are summarized in Table 10. The induction of mecA expression was strain dependent as some strains were only positive for mecA gene signal using the mixture of mRNA-directed PNA probes after induction with cefoxitin. Cefoxitin induction improved results for the SCCmec type II and III strains. However, for some strains of SCC*mec* type I and IV, induction was not required as these strains were positive with and without cefoxitin induction. The tested SCCmec type V strain was not positive even after cefoxitin induction in TSB. However, the same strain was detected positive when spiked into blood culture. This strain has a very low cefoxitin MIC (MIC = 12 µg/mL); which is close to the susceptibility breakpoint (S: ≤4 µg/ml and R: ≥ 8 µg/mL) according to Clinical and Laboratory Standards Institute (CLSI) which could indicate a low level of mecA expression. As Example 6, above suggests, variation of the probe mixture (e.g. increasing the number of m-RNA probes in the mixture or the concentration of the probe used) might lead to improved results for strain 306.

All strains were tested to confirm active *S*. *aureus* (16S rRNA) after cefoxitin induction with *S*. *aureus*/*CNS* PNA FISH^{®} (AdvanDx) and the presence of the mecA gene were tested with mecA EVIGENE^{®} (AdvanDx). The *S*. *aureus*/*CNS* PNA FISH^{®} results were positive for all MRSA and MSSA strains after cefoxitin induction suggesting that the MSSA strains are still active after 40 min cefoxitin induction.

**Table 10**

| **Strain ID.** | **Specie** | **SCCmec type** | **MIC (µg/ml)** | **EVIGENE®** | **PNA FISH (Probes From Table 9) *mecA*** | |
|---|---|---|---|---|---|---|
| | | | **Etest®** | ***mecA*** | ***No induction*** | ***With induction*** |
| 9 | **MRSA** | I | ≥256 | Positive | Positive | Positive |
| 1468 | | I | ≥256 | Positive | Positive | Positive |
| 39 | | II | 128 | Positive | Negative | Positive |
| 11 | | II | ≥256 | Positive | Negative | Positive |
| 2 | | III | ≥256 | Positive | Negative | Positive |
| 13 | | IIIa | ≥256 | Positive | Negative | Positive |
| 320 | | IV | ≥256 | Positive | Negative | Positive |
| 886 | | IV | 48 | Positive | Positive | Positive |
| 306 | | V | 12 | Positive | Negative | Negative |
| 155 | **MSSA** | NA | 3 | Negative | Negative | Negative |
| 156 | | NA | 4 | Negative | Negative | Negative |

### Appendix IV

***Description of bacterial strains:***
# *9*, *S*. *aureus* EU (MRSA EU); SCCmec I; a methicillin-resistant strain
*# 1468, S*. *aureus* COL (MRSA COL); SCCmec I; a methicillin-resistant strain
#*39*, *S*. *aureus* ATCC 43300 (MRSA 43300); SCCmec II; a methicillin-resistant strain
#*11, S*. *aureus* EU (MRSA EU); SCCmec II; a methicillin-resistant strain
#*2*, *S*. *aureus* ATCC 33591 (MRSA 33591); SCCmec III; a methicillin-resistant strain
#*13, S*. *aureus* DK (MRSA EU); SCCmec IIIa; a methicillin-resistant strain
#*320, S*. *aureus* DK (clinical DK); SCCmec IV; a methicillin-resistant strain
#*886*, *S*. *aureus* USA300 (MRSA USA300); SCCmec IV; a methicillin-resistant strain
#*306*, *S*. *aureus* DK (MRSA DK); SCCmec V; a methicillin-resistant strain
#*155*, *S*. *aureus* ATCC 25923 (MSSA 25923); a methicillin-sensitive strain
#*156*, *S*. *aureus* ATCC 29213 (MSSA 29213); a methicillin-sensitive strain

[ATCC stands for American Type Culture Collections and is a source for various organisms - See the worldwide web at: atcc.org/CulturesandProducts/Microbiology /BacteriaandPhages/tabid/176/Default.aspx. NCTC stands for National collection of Type Cultures and is a source for various organisms - See the worldwide web at: ukncc.co.uk/index.htm]

### Example 9: Detection of mecA mRNA by Solution Hybridization

An experiment was performed to simulate the work flow in which a clinical sample might be processed. This experiment included a combined fixative/hybridization solution (FLOW Hybridization Buffer) which also contained mRNA-directed PNA probes for detection of mecA mRNA in the whole-cells. The induction and detection of mecA expression were performed at log-phase cultures (OD₆₀₀ₙₘ ∼ 0.5) on uninduced and Cefoxitin-induced cells. Unless otherwise noted, all procedures were performed at room temperature (RT). The composition of FLOW Hybridization Buffer (FHB), FLOW Wash Buffer (FWB) and Mounting Medium (MM) as well as a description of various bacterial strains (e.g. staphylococcal chromosome cassette *mec* (*SCCmec*) type I-V and other staphylococcus strain), PNA probes and Fluorescent Microscopy 2 (FM2) used in this Example 9 is provided in the Appendix I, Appendix II or in Appendix V (below).

### Cell growth:

*S. aureus* bacteria were inoculated into Tryptic Soy Broth (TSB) and grown overnight (16-18 hours) at 35±2°C with shaking, diluted to OD₆₀₀ₙₘ ∼ 0.25 in prewarmed TSB and grown to OD₆₀₀ₙₘ ∼ 0.5. This pre-culture was split in two cultures; an induced (with cefoxitin 3 µg/mL) and an uninduced (with 0 µg/mL Cefoxitin) culture. Both cultures were grown for another 40 min with shake at 35±2°C before being subjected to probe hybridization.

### Combined fixation and hybridization in aqueous alcohol solution containing PNA probe:

200 µL of FLOW Hybridization Buffer (FHB) containing PNA Probes (See below for probe concentration) was mixed with either 20 µL of induced, or uninduced, culture in a tube and hybridized for 3 hours at 55 °C with shaking. After the hybridization, the cells were washed three times with 500 µL FLOW Wash Buffer (FWB). 30 µL of cells were then applied to a slide and heat fixed for 20 min at 55 °C.

### Fluorescence microscopy:

One drop of Mounting Medium (MM) and a cover slip was applied on to the dried specimen and slide-deposited cells were examined according to the procedure labeled Fluorescence Microscopy 2 (FM2)

### PNA Probes and Probe concentrations:

PNA Probes were obtained from Panagene, Daejeon, Korea. Table 11 lists attributes of the PNA probes used in this Example 9.

**Table 11**

| **SEQ ID NO:/Probe No.** | **Name** | **Probe Configuration & Nucleobase Sequence** | **Target** | **Select Trait** |
|---|---|---|---|---|
| 16 | 016-II | Flu-OO-CACATTGTTTCGGTCT-OO-Lys(Flu) | mRNA | *mecA* |
| 17 | 018-II | Flu-OO-CATTAGTTGTAAGATG-OO-Lys(Flu) | mRNA | *mecA* |
| 18 | 020-II | Flu-OO-TCTTCAGAGTTAATGG-OO-Lys(Flu) | mRNA | *mecA* |
| 19 | 021-II | Flu-O-GCTATTATCGTCAACG-OO-Lys(Flu) | mRNA | *mecA* |
| 20 | 023-II | Flu-OO-AGCATCAATAGTTAG-OO-Lys(Flu) | mRNA | *mecA* |
| 21 | 024-II | Flu-OO-TGTGCTTACAAGTGC-OO-Lys(Flu) | mRNA | *mecA* |
| 22 | 025-II | Flu-OO-TACCTGAGCCATAAT-OO-Lys(Flu) | mRNA | *mecA* |
| 24 | 003-II | Flu-OO-TAGTCTTCAGAAATAC-OO-Lys(Flu) | mRNA | *mecA* |
| 25 | 008-II | Flu-OO-AACGAAGGTATCATC-OO-Lys(Flu) | mRNA | *mecA* |
| 26 | 13 | Flu-OO-CAATAACTGCATCATC | mRNA | *mecA* |

| | | | | |
|---|---|---|---|---|
| All abbreviations used in this Table have previously been defined. | | | | |

A mixture of the ten fluorescein-labeled, mecA mRNA-directed PNA probes listed in Table 11 were used at concentrations of 250 nM for each of the probes. Thus, the combined concentration of probes was 2500 nM.

### Results:

Analysis of uninduced and cefoxitin-induced *S.aureus* isolates with mecA PNA FISH^{Flow} are summarized in Table 12. The induction of mecA expression was found to be strain dependent, as some strains did not need induction to obtain a mecA signal. These results are similar to those found for the slide based assay described in Example 8 and Table 10, above. SCCmec types I, II, III and IV all resulted in mecA signal, when induced with cefoxitin. Of two *SCCmec* type I strains, one strain did not result in mecA signal when not induced, however, the other did. The *SCCmec* type II and III strains did not result in any mecA signal when not induced. Of two *SCCmec* type IV strains, one strain resulted in no signal or very low mecA signal when not induced, and the other one resulted in mecA signal when not induced. The *SCCmec* V strain did not result in any signal regardless of induction with cefoxitin. However, the same strain was detected positive when spiked into blood culture. This strain has a very low cefoxitin MIC (MIC = 12 µg/mL) close to the susceptibility breakpoint (S: ≤ 4 µg/ml and R: ≥ 8 µg/mL) according to CLSI; which again could indicate a low level of mecA expression.

With reference to Example 8 and Table 10 by comparison with this Example 9 and Table 12, The mecA PNA FISH Flow results agreed 100% with the slide-based results of Example 8 except for one strain (a SCCmec type I) which required induction when tested in PNA FISH ^{Flow} (compare this result those of Table 8 for Strain ID 9). The results indicate that some MRSA strains may need induction.

**Table 12**

| **Strain ID.** | **Specie** | **SCCmec type** | **MIC (µg/ml)** | **PNA FISH^{Flow} *mecA*** | |
|---|---|---|---|---|---|
| | | | **Etest®** | ***No induction*** | ***With induction*** |
| 9 | **MRSA** | I | ≥256 | Negative | Positive |
| 1468 | | I | ≥256 | Positive | Positive |
| 39 | | II | 128 | Negative | Positive |
| 11 | | II | ≥256 | Negative | Positive |
| 2 | | III | ≥256 | Negative | Positive |
| 13 | | IIIa | ≥256 | Negative | Positive |
| 320 | | IV | ≥256 | Negative | Positive |
| 886 | | IV | 48 | Positive | Positive |
| 306 | | V | 12 | Negative | Negative |
| 155 | **MSSA** | NA | 3 | Negative | Negative |
| 156 | | NA | 4 | Negative | Negative |

### Appendix V

***Description of bacterial strains:*** All strains are listed in Appendix IV.

### Composition of buffers:

**FLOW Hybridization Buffer (FHB):** 25 mM Tris-HCl pH = 9; 0.1% (w/v) sodium dodecyl sulfate; 100 mM NaCl; 50% methanol; water adjust to 100%.

**FLOW Wash Buffer (FWB):** 5 mM Tris-HCl pH = 9; 0.1% (v/v) Triton-X 100; 25 mM NaCl; 0.05% (v/v) proclin 300; water adjust to 100%.

### Example 10: Analysis of Isolates Spiked in Blood Culture

Some commercial molecular diagnostic tests for the determination of S. *aureus* and mecA can, in blood cultures containing a mixture of MSSA (negative for mecA gene) and methicillin-resistant CNS (MR-CNS; negative for S. *aureus* but positive for mecA gene), lead to an incorrect identification of the sample as being MRSA. The study in this Example 10 involves the simultaneous determination of S. *aureus* (S. *aureus* rRNA-directed probe (SEQ I D NO: 8)) and mecA expression (mRNA-directed PNA probes (i.e. SEQ ID NOs: 16-22, 24-26)) for mixed spiked blood culture (MSSA + MRSA, MSSA + MR-CNS, MRSA + MR-CNS and MSSA + MR-CNS + MRSA) by using a PNA FISH assay.

In addition, all cultures were tested for the presence of S. *aureus* (16S rRNA) with S. *aureus*/*CNS* PNA FISH^{®} (AdvanDx, Part No: KT005) and with S. *aureus* EVIGENE^{®} (AdvanDx, Part No: KT106-96) according to manufacturers instructions. The presence of the mecA gene was independently confirmed with mecA EVIGENE^{®} (AdvanDx, Part No: KT102-96) according to the manufacturers instructions. Unless otherwise noted, all procedures were performed at room temperature (RT). The composition of Fixation Solution (FS), Hybridization Buffer (HB2), and Wash Buffer (WB), Mounting Media (MM) as well as a description of various bacterial strains, PNA probes and fluorescent microscopy (FM) used in this Example 10 is provided in Appendix I, Appendix II or Appendix VI.

### Cell growth and fixation:

MSSA, MRSA, MR-CNS *(S. epidermidis)* and MS-CNS *(S. epidermidis)* bacteria (See: Appendix VI) were inoculated into negative blood culture and grown overnight (16-18 hours) at 35-37 °C with shaking. All strains were mixed 1:1 (150 µL + 150 µL) or 1:2 (150 µL + 300 µL). After mixing the culture was diluted 1:9 in prewarmed TSB and grown for another 1.5 hr. This preculture was induced with cefoxitin 3 µg/mL with shaking at 35±2°C for another 40 min. Cefoxitin-induced cells were added (20 µL) on glass slides and heat fixed with Fixation Solution (FS) for 2 minutes (min) at 80 °C. After heat fixation the slides were immersed in 100% methanol for 5 min and left to air-dry for approximately 5 min.

### Fluorescence in situ hybridization and fluorescence microscopy:

For samples that weren't treated with commercial products, 25 µL of Hybridization Buffer (HB2) containing PNA Probes (See below for probe concentration) was applied on the slide in a hybridization chamber and covered with a cover slip. The cells were hybridized for 0.5 hours at 55°C. After hybridization, the slides were washed with Wash Buffer (WB) for 30 min at 55°C. A drop of Mounting Media (MM) and a cover slip was applied on the dried specimen and slide-deposited cells were examined according to the procedure labeled Fluorescence Microscopy 2 (FM2).

### PNA Probes and Probe concentrations:

PNA Probes were obtained from Panagene, Daejeon, Korea. Table 13 lists attributes of the PNA probes used in this Example 10.

**Table 13**

| **SEQ ID NO:/Probe No.** | **mecA-** | **Probe Configuration & Nucleobase Sequence** | **Target** | **Select Bacteria or Select Trait** |
|---|---|---|---|---|
| 16 | 016-II | Flu-OO-CACATTGTTTCGGTCT-OO-Lys(Flu) | mRNA | *mecA* |
| 17 | 018-II | Flu-OO-CATTAGTTGTAAGATG-OO-Lys(Flu) | mRNA | *mecA* |
| 18 | 020-II | Flu-OO-TCTTCAGAGTTAATGG-OO-Lys(Flu) | mRNA | *mecA* |
| 19 | O21-II | Flu-OO-GCTATTATCGTCAACG-OO-Lys(Flu) | mRNA | *mecA* |
| 20 | 023-II | Flu-OO-AGCATCAATAGTTAG-OO-Lys(Flu) | mRNA | *mecA* |
| 21 | 02.4-II | Flu-OO-TGTGCTTACAAGTGC-OO-Lys(Flu) | mRNA | *mecA* |
| 22 | 025-II | Flu-OO-TACCTGAGCCATAAT-OO-LyS(Flu) | mRNA | *mecA* |
| 24 | 003-II | Flu-OO-TAGTTCAGAAATAC-OO-Lys(Flu) | mRNA | *mecA* |
| 25 | 008-II | Flu-OO-AACGAAGGTATCATC-OO-lys(Flu) | mRNA | *mecA* |
| 26 | 13 | Flu-OO-CAATAACTGCATCATC | mRNA | *mecA* |
| 8 | Sta16S03 | TAM-GCTTCTCGTCCGTTC | rRNA | *S.aureus* |

| | | | | |
|---|---|---|---|---|
| All abbreviations used in this Table have previously been defined. | | | | |

A mixture of the 10 fluorescein-labeled, mecA mRNA-directed PNA probes listed as SEQ ID NOs: 16-22 and 24-26 in Table 13 were used at concentrations of 500 nM for each of the probes. The total concentration of mRNA-directed PNA probes was 5000 nM. Also added to HB2 was the one rRNA-directed S. *aureus* PNA probe (SEQ ID NO: 8) at concentration of 12 nM.

### Results:

MSSA, MRSA, MR-CNS *(S. epidermidis)* and MS-CNS *(S. epidermidis)* bacteria were detected when spiked into blood culture. The bacteria were characterized by a red fluorescence signal for the MSSA, a yellow signal for the MRSA due to combined green fluorescence of Probes 16-22 and 24-26 (labeled with Flu) and red fluorescence of Probe 8 (rRNA-directed *S. aureus*-specific probes labeled with TAMRA), a green signal for the MR-CNS (These bacteria were only green because they are not S. aureus (i.e. no TAMRA signal) and no signal for the MS-CNS as there are no probes directed for this bacteria in the assay. All results are summarized in Table 14. Identification of *S.aureus* and mecA expression in mixed populations in blood culture was possible for MSSA+MRSA, MSSA + MR-CNS, MRSA + MR-CNS and MSSA + MR-CNS + MRSA.

When testing the same mixed populations in EVIGENE® product, the result for MSSA + MR-CNS mixed samples was MRSA. Using the *S.aureus*/CNS PNA FISH product in combination with EVIGENE®, the MSSA + MR-CNS mixed sample was shown to be mixed was not able to identify if it is the *S.aureus* or the CNS that is positive for the mecA gene. The PNA FISH assay performed with the probes listed in Table 13 provides unequivocal identification of MRSA, MSSA and MR-CNS by providing both *S. aureus* identification and a independent determination of mecA expression in individual bacteria cells. Consequently, this whole-cell assay is capable of accurate determination of complex samples containing mixed populations.

**Table 14**

| **Strain ID.** | **Sample type** | **EVIGENE** | **PNA FISH (Commercial Product)** | **PNA FISH (mRNA-Directed & rRNA-Directed Probes)** |
|---|---|---|---|---|
| | | ***S.aureus*/** *mecA* | ***S.aureus*/ CNS** | ***S.aureus*/ *mecA*** |
| 155 | MSSA | MSSA | *S.aureus* | MSSA |
| 320 | MRSA | MRSA | *S.aureus* | MRSA |
| 350 | MR-CNS | MR-CNS | CNS | MR-CNS |
| 629 | MS-CNS | negative | CNS | negative |
| 155 + 320 | MSSA + MRSA | MRSA | *S.aureus* | MSSA + MRSA |
| 155 + 350 | MSSA + MR-CNS | MRSA | *S.aureus* + CNS | MSSA + MR-CNS |
| 320 + 350 | MRSA + MR-CNS | MRSA | *S.aureus* + CNS | MRSA + MR-CNS |
| 155 + 320 + 350 | MSSA + MR-CNS + MRSA | MRSA | *S.aureus* & CNS | MSSA + MR-CNS + MRSA |

### Appendix VI

***Description of bacterial strains:***
#*155*, *S*. *aureus* ATCC 25923 (MSSA 25923); a methicillin-sensitive strain
#*320, S*. *aureus* DK (MRSA, clinical DK); SCCmec IV; a methicillin-resistant strain
#*350, S*. *epidermidis* (MR-CNS, clinical); a methicillin-resistant strain
#*629*, *S. epidermidis* ATCC 14990 (MS-CNS); a methicillin-sensitive strain

**Table 15**

| **Strain ID** | **Specie** | **ID** | ***SCCmec* /*ST type*** | **MIC** |
|---|---|---|---|---|
| 306 | MRSA | Reference DK | *type V* | 12 |
| 460 | MRSA | Reference, Tokyo | *type V* | 12 |
| 927 | MRSA | Clinical DK | *type IV* | 24 |
| 15 | MRSA | Clinical DK | *type IV*/*ST80* | 48 |
| 297 | MRSA | Reference DK | *type IA, EVIGENE IV* | 48 |
| 304 | MRSA | Reference DK | *type IV* | 48 |
| 307 | MRSA | Reference DK | *type IV* | 48 |
| 560 | MRSA | Reference (USA500) | *type IV* | 48 |
| 886 | MRSA | Reference (FPR3757, USA300) | *type IV* | 48 |
| 926 | MRSA | Clinical DK | *type IV (EVIGENE)* | 48 |
| 797 | MRSA | Clinical DK | *type 11 (EVIGENE)* | 64 |
| 557 | MRSA | Reference (USA300) | *type IV* | 64 |
| 321 | MRSA | Clinical DK | *type V (EVIGENE)* | 96 |
| 924 | MRSA | Clinical DK | *type IV (EVIGENE)* | 96 |
| 1184 | MRSA | Reference (ATCC BAA-1708) | *type II (EVIGENE)* | 96 |
| 39 | MRSA | Reference (ATCC 43300) | *type II (EVIGENE)* | 128 |
| 2 | MRSA | Reference (ATCC 33591) | *type III* | ≥256 |
| 10 | MRSA | Reference EU | *type Ia* | ≥256 |
| 12 | MRSA | Reference EU | *type III* | ≥256 |
| 13 | MRSA | Reference EU | *type IIIa* | ≥256 |
| 295 | MRSA | Reference DK | *type IA* | ≥256 |
| 299 | MRSA | Reference DK | *type II* | ≥256 |
| 300 | MRSA | Reference DK | *type II* | ≥256 |
| 302 | MRSA | Reference DK | *type IIIA* | ≥256 |
| 303 | MRSA | Reference DK | *type III* | ≥256 |
| 305 | MRSA | Reference DK | *type III* | ≥256 |
| 559 | MRSA | Reference (USA300) | *type IV* | ≥256 |
| 801 | MRSA | Clinical DK | *type II (EVIGENE)* | ≥256 |
| 812 | MRSA | Clinical DK | *type II (EVIGENE)* | ≥256 |
| 920 | MRSA | Clinical DK | *type I (EVIGENE)* | ≥256 |
| 931 | MRSA | Clinical DK | *type III (EVIGENE)* | ≥256 |
| 1468 | MRSA | Reference (COL) | *type I* | ≥256 |
| 9 | MRSA | Reference EU | *type I* | ≥256 |
| 11 | MRSA | Reference EU | *type II* | ≥256 |
| 298 | MRSA | Reference DK | *type II* | ≥256 |
| 320 | MRSA | Clinical DK | *type IV (EVIGENE)* | ≥256 |
| 463 | MRSA | Reference (NCTC10422) | *type Ib* | ≥256 |
| 792 | MRSA | Clinical DK | *type IV* | NA |
| 807 | MRSA | Clinical DK | *type IV* | NA |
| 810 | MRSA | Clinical DK | *NA* | NA |
| 814 | MRSA | Clinical DK | *type IV* | NA |
| 816 | MRSA | Clinical DK | *type IV* | NA |
| 820 | MRSA | Clinical DK | *NA* | NA |
| 827 | MRSA | Clinical DK | *NA* | NA |
| 830 | MRSA | Clinical DK | *NA* | NA |
| 831 | MRSA | Clinical US | *NA* | NA |
| 834 | MRSA | Clinical US | *NA* | NA |
| 835 | MRSA | Clinical US | *NA* | NA |
| 836 | MRSA | Clinical US | *NA* | NA |
| 837 | MRSA | Clinical US | *NA* | NA |
| 838 | MRSA | Clinical US | *NA* | NA |
| 839 | MRSA | Clinical US | *NA* | NA |
| 840 | MRSA | Clinical US | *NA* | NA |
| 841 | MRSA | Clinical US | *NA* | NA |
| 842 | MRSA | Clinical US | *NA* | NA |
| 843 | MRSA | Clinical US | *NA* | NA |
| 845 | MRSA | Clinical US | *NA* | NA |
| 846 | MRSA | Clinical US | *NA* | NA |
| 847 | MRSA | Clinical US | *NA* | NA |
| 848 | MRSA | Clinical US | *NA* | NA |
| 849 | MRSA | Clinical US | *NA* | NA |
| 850 | MRSA | Clinical US | *NA* | NA |
| 851 | MRSA | Clinical US | *NA* | NA |
| 852 | MRSA | Clinical US | *NA* | NA |
| 853 | MRSA | Clinical US | *NA* | NA |
| 854 | MRSA | Clinical US | *NA* | NA |
| 855 | MRSA | Clinical US | *NA* | NA |
| 856 | MRSA | Clinical US | *NA* | NA |
| 857 | MRSA | Clinical US | *NA* | NA |
| 858 | MRSA | Clinical US | *NA* | NA |
| 859 | MRSA | Clinical US | *NA* | NA |
| 860 | MRSA | Clinical US | *NA* | NA |
| 861 | MRSA | Clinical US | *NA* | NA |
| 862 | MRSA | Clinical US | *NA* | NA |
| 863 | MRSA | Clinical US | *NA* | NA |
| 864 | MRSA | Clinical US | *NA* | NA |
| 865 | MRSA | Clinical US | *NA* | NA |
| 866 | MRSA | Clinical US | *NA* | NA |
| 867 | MRSA | Clinical US | *NA* | NA |
| 868 | MRSA | Clinical US | *NA* | NA |
| 869 | MRSA | Clinical US | *NA* | NA |
| 870 | MRSA | Clinical US | *NA* | NA |
| 871 | MRSA | Clinical US | *NA* | NA |
| 872 | MRSA | Clinical US | *NA* | NA |
| 873 | MRSA | Clinical US | *NA* | NA |
| 874 | MRSA | Clinical US | *NA* | NA |
| 875 | MRSA | Clinical US | *NA* | NA |
| 876 | MRSA | Clinical US | *NA* | NA |
| 877 | MRSA | Clinical US | *NA* | NA |
| 878 | MRSA | Clinical US | *NA* | NA |
| 879 | MRSA | Clinical US | *NA* | NA |
| 880 | MRSA | Clinical US | *NA* | NA |
| 881 | MRSA | Clinical US | *NA* | NA |
| 882 | MRSA | Clinical US | *NA* | NA |
| 883 | MRSA | Clinical US | *NA* | NA |
| 884 | MRSA | Clinical US | *NA* | NA |
| 885 | MRSA | Clinical US | *NA* | NA |
| 896 | MRSA | Clinical DK | *type IV* | NA |
| 904 | MRSA | Clinical DK | *type IV* | NA |
| 913 | MRSA | Clinical DK | *type IV (EVIGENE)* | NA |
| 916 | MRSA | Clinical DK | *type IV (EVIGENE)* | NA |
| 917 | MRSA | Clinical DK | *NA* | NA |
| 919 | MRSA | Clinical DK | *NA* | NA |
| 921 | MRSA | Clinical DK | *NA* | NA |
| 923 | MRSA | Clinical DK | *I* | NA |
| 929 | MRSA | Clinical DK | *type IV (EVIGENE)* | NA |
| 930 | MRSA | Clinical DK | *type II (EVIGENE)* | NA |
| 935 | MRSA | Clinical DK | *type IV (EVIGENE)* | NA |
| 984 | MRSA | Clinical DK | *NA* | NA |
| 1169 | MRSA | Clinical DK | *type I, IV and V* | NA |
| 1170 | MRSA | Clinical DK | *type IV* | NA |
| 1171 | MRSA | Clinical DK | *type IV* | NA |
| 1172 | MRSA | Clinical DK | *NA* | NA |
| 1173 | MRSA | Clinical DK | *NA* | NA |
| 1174 | MRSA | Clinical DK | *NA* | NA |
| 1175 | MRSA | Clinical DK | *NA* | NA |
| 1177 | MRSA | Clinical DK | *NA* | NA |
| 1180 | MRSA | Clinical DK | *type V* | NA |
| 1181 | MRSA | Clinical DK | *NA* | NA |
| 1373 | MRSA | Clinical US | *NA* | NA |
| 1376 | MRSA | Clinical US | *NA* | NA |
| 1484 | MRSA | Clinical EU | *type II (EVIGENE)* | NA |
| 1485 | MRSA | Clinical EU | *type II (EVIGENE)* | NA |
| 823 | MRSA | Clinical DK | *type IV* | NA |
| *888* | MRSA | Clinical DK | *type IV* | NA |
| *891* | MRSA | Clinical DK | *type IV* | NA |
| 900 | MRSA | Clinical DK | *type IV (EVIGENE)* | NA |
| 4 | MR-CNS, S. epidermidis | Reference (ATCC 51625) | *NA* | 32 |
| 353 | MR-CNS, S. hominis | Reference DK | *NA* | 64 |
| 351 | MR-CNS, S. warneri | Reference DK | *NA* | 192 |
| 352 | MR-CNS, S. epidermidis | Reference DK | *NA* | 256 |
| 414 | MR-CNS | Clinical DK | *NA* | 256 |
| 154 | MR-CNS, S. saccharolyticus | Reference (ATCC 14953) | *NA* | ≥256 |
| 350 | MR-CNS, S. epidermidis | Reference | *NA* | ≥256 |
| 408 | MR-CNS | Clinical DK | *NA* | ≥256 |
| 412 | MR-CNS | Clinical DK | *NA* | ≥256 |
| 432 | MR-CNS | Clinical DK | *NA* | NA |
| 444 | MR-CNS | Clinical DK | *NA* | NA |
| 428 | MR-CNS | Clinical DK | *NA* | NA |
| 429 | MR-CNS | Clinical DK | *NA* | NA |
| 430 | MR-CNS | Clinical DK | *NA* | NA |
| 431 | MR-CNS | Clinical DK | *NA* | NA |
| 433 | MR-CNS | Clinical DK | *NA* | NA |
| 434 | MR-CNS | Clinical DK | *NA* | NA |
| 435 | MR-CNS | Clinical DK | *NA* | NA |
| 438 | MR-CNS | Clinical DK | *NA* | NA |
| 440 | MR-CNS | Clinical DK | *NA* | NA |
| 442 | MR-CNS | Clinical DK | *NA* | NA |
| 443 | MR-CNS | Clinical DK | *NA* | NA |
| 445 | MR-CNS | Clinical DK | *NA* | NA |
| 447 | MR-CNS | Clinical DK | *NA* | NA |
| 448 | MR-CNS | Clinical DK , | *NA* | NA |
| 91 | MS-CNS, S. Warneri | Reference (ATCC 49454) | *NA* | NA |
| 92 | MS-CNS, S. Capitis | Reference (ATCC 35661) | *NA* | NA |
| 120 | MS-CNS, S. Haemolyticus | Reference (ATCC 29970) | *NA* | NA |
| 143 | MS-CNS, S. Hominis | Reference (ATCC 27844) | *NA* | NA |
| 629 | MS-CNS, S. epidermidis | Reference (ATCC 14990) | *NA* | 1.5 |
| 1 | MSSA | Reference (ATCC 6538) | *NA* | 3 |
| 23 | MSSA | Clinical DK | *NA* | 3 |
| 117 | MSSA | Reference (ATCC 11632) | *NA* | 3 |
| 155 | MSSA | Reference (ATCC 25923) | *NA* | 3 |
| 21 | MSSA | Clinical EU | *NA* | 4 |
| 22 | MSSA | Clinical DK | *NA* | 4 |
| 24 | MSSA | Clinical DK | *NA* | 4 |
| 26 | MSSA | Clinical DK | *NA* | 4 |
| 54 | MSSA | Clinical EU | *NA* | 4 |
| 156 | MSSA | Reference (ATCC 29213) | *NA* | 4 |
| 1482 | MSSA | Clinical EU | *NA* | NA |
| 1176 | MSSA | Clinical DK | *NA* | NA |
| 1178 | MSSA | Clinical DK | *NA* | NA |
| 1481 | MSSA | Clinical EU | *NA* | NA |
| 1483 | MSSA | Clinical EU | *NA* | NA |

| | | | | |
|---|---|---|---|---|
| NA = Not applicable | | | | |

*While the present teachings are described in conjunction with various embodiments, it is not intended that the present teachings be limited to such embodiments. On the contrary, the present teachings encompass various alternatives, modifications and equivalents, as will be appreciated by those of skill in the art.*

### 8. References

**US Patent and Published Patent Applications:**
1. US Pat. No. 5,225,326 to Bresser et al. issued July 6, 1993.
2. US Pat. No. 5,612,458 to Hyldig-Nielsen issued March 18, 1997.
3. US Pat. No. 5,702,895 to Matsunaga et al. issued December 30, 1997.
4. US Pat. No. 6,156,507 to Hiramatsu et al. issued on December 5, 2000.
5. US Pat. No. 6,231,857 to Wenyuan et al. issued on May 15, 2001.
6. US Pat. No. 6,524,798 to Goldbard et al. issued February 25, 2003.
7. US Pat. No. 6,656,687 to Hyldig-Nielsen, J.J. issued on December 2, 2003.
8. US Pat. No. 6,664,045 to Hyldig-Nielsen et al. issued on December 16, 2003.
9. US Pat. No. 6,841,154 to Foster et al. issued on January 11, 2005.
10. US Pat. No. 6,905,824 to Rigby et al. issued on June 14, 2005.
11. US Pat. No. 7,074,599 to Uhl et al. issued Ju!y 11, 2006.
12. US Pat. No. 7,455,985 to Stuart et al. issued Nov. 25, 2008.
13. US Pat. Application No. 2002/0142326 to Coull et al. published on October 3, 2002.
14. US Pat. Application No. 2005/0123959 to Williams et al. published on June 9, 2005.
15. US Pat. Application No. 2008/0008994 to Stender et al. published on January 10, 2008.

**Scientific Publications:**
1. Hahn et al., "Detection of mRNA in Streptomyces Cells by Whole-Cell Hybridization with Digoxigenin-Labeled Probes", Applied and Environmental Microbiology, 59(8): 2753-2757 (Aug. 1993).
2. Hönerlage et al., "Detection of mRNA of nprM in Bacillus megaterium ATTC 14581 grown in soil by whole-cell hybridization", Arch. Microbiol., 163: 235-241 (1995).
3. Aßmus et al., "Improved In Situ Tracking of Rhizosphere Bacteria Using Dual Staining with Fluorescence-Labeled Antibodies and rRNA-Targeted Oligonucleotides", Microb. Ecol., 33: 32-40 (1997).
4. Wagner et al., "In situ detection of a virulence factor mRNA and 16S rRNA in Listeria", FEMS Microbiol. Lett., 160(1): 159-168 (Mar. 1998).
5. Cavassini et al., "Evaluation of MRSA-Screen, a Simple Anti-PBP 2a Slide Latex Agglutination Kit, for Rapid Detection of Methicillin Resistance in Staphylococcus aureus", J. Clinical Microbiology, 37(5): 1591-1594 (May, 1999).
6. Amann, R., "Methodological Aspects of Fluorescence In Situ Hybridization", Bioscience Microflora, 19(2): 85-91 (2000).
7. Pernthaler et al., "Fluorescence in situ Hybridization (FISH) with rRNA-targeted Oligonucleotide Probes", Methods in Microbiology, 30: 207-226 (2001).
8. Pinho et al., "An acquired and a native penicillin-binding protein cooperate in building the cell wall of drug-resistant staphylococci", PNAS, 98(19): 10886-10891 (Sept. 2001).
9. Pucci et al., "Direct Quantitation of the Numbers of Individual Penicillin-Binding Proteins per Cell in Staphylococcus aureus", J. Bacteriology, 184(2): 588-591 (Jan. 2002).
10. Sekar et al., "An Improved Protocol for Quantification of Freshwater Actinobacteria by Fluorescence In Situ Hybridization", Applied and Environmental Microbiology, 69(5): 2928-2935 (May, 2003).
11. Pernthaler et al., "Simultaneous Fluorescence In Situ Hybridization of mRNA and rRNA in Environmental Bacteria", Applied and Environmental Microbiology, 70(9): 5426-5433 (Sept. 2004).
12. Zwirgimaler et al., "Recognition of individual genes in a single bacterial cell by fluorescence in situ hybridization - RING-FISH", Molecular Microbiology, 51(1): 89-96 (2004).
13. Maruyama et al., "Visualization and Enumeration of Bacteria Carrying a Specific Gene Sequence by In Situ Rolling Circle Amplification", Applied and Environmental Microbiology, 71(12): 7933-7940 (Dec. 2005).
14. Furakawa et al., "Comprehensive Analysis of Cell Wall-Permeabilizing Conditions for Highly Sensitive Fluorescence In Situ Hybridization", Microbes Environ., 21(4): 227-234 (2006).
15. Forrest et al., "Impact of rapid in situ hybridization testing on coagulase-negative staphylococci positive blood cultures", Journal of Antimicrobial Chemotherapy, 58: 154-158 (2006).
16. Coleman et al., "mRNA-targeted fluorescent in-situ hybridization (FISH) of Gram-negative bacteria without template amplification or tyramide signal amplification", J. Microbiological Methods, 71: 246-255 (2007).
17. Smolin et al., "Detection of Low-Copy-Number Genomic DNA Sequences in Individual Bacterial Cells by Using Peptide Nucleic Acid-Assisted Rolling-Circle Amplification and Fluorescence In Situ Hybridization", Applied and Environmental Microbiology, 73(7): 2324-2328 (2007).
18. Hoshino et al., "Quantification of Target Molecules Needed To Detect Microorganisms by Fluorescence In Situ Hybridization (FISH) and Catalyzed Reporter Deposition-FISH", Applied and Environmental Microbiology, 74(16): 5068-5077 (2008).
19. Cerqueira et al., "DNA Mimics for the Rapid Identification of Microorganisms by Fluorescence in situ Hybridization (FISH)", Int. J. Mol. Sci., 9: 1944-1960 (2008).
20. Ender et al., "A novel DNA-binding protein modulating methicillin resistance in Stapyloccocus aureus", BMC Microbiology, 9:15 (2009).
21. Gröbner et al., "Evaluation of the BD GeneOhm StaphSR Assay for Detection of Methicillin-Resistant and Methicillin-Susceptible Staphylococcus aureus Isolates from Spiked Positive Blood Culture Bottles", J. Clin. Microbiol., 47(6): 1689-1694 (2009).

## Claims

1. A method comprising:
a) contacting a sample comprising bacteria with a mixture of first labeled PNA probes, the first labeled PNA probes being chromosomal DNA-, mRNA- and/or native plasmid-directed and capable of determining chromosomale DNA, mRNA and/or plasmid nucleic acid associated with a select trait that may be possessed by a select gram-positive bacteria of said sample; and
b) determining gram-positive bacteria of said sample that possess said select trait; wherein,
i) said method is practiced on whole cells;
ii) said first labeled PNA probes are each labeled with a single label or with two labels; and
iii) the method is practiced without use of in situ PCR and without signal amplification of said label of said first labeled PNA probes.

2. The method of claim 1,
wherein said first labeled PNA probes are chromosomal DNA and/or mRNA-directed and capable of determining methicillin-resistance in bacteria of said sample, and
wherein gram-positive bacteria that possess methicillin-resistance are determined in step b).

3. The method of claim 1, in step a) further comprising contacting the sample with a second probe or probes, said second probe or probes being bacteria-directed and capable of determining the select gram-positive bacteria in said sample, and further comprising
c) determining one or more of said select gram-positive bacteria in said sample; wherein steps (b) and (c) are carried out in either order or simultaneously, preferably wherein
aa) said first labeled PNA probes are chromosomal DNA and/or mRNA.-directed and capable of determining methicillin-resistance in bacteria of said sample,
bb) wherein said second probe or probes are capable of determining *S*. *aureus* bacteria in said sample,
cc) wherein gram-positive bacteria that possess methicillin-resistance are determined in step b), and
dd) wherein *S. aureus* bacteria are determined in step c).

4. The method of any of claims 1 to 3, wherein the first labeled PNA probes are only mRNA-directed labeled probes.

5. The method of any of claims 1 to 3, wherein no pre-hybridization step is performed.

6. The method of any one of claims 3 to 5, wherein said second probe or probes is/are rRNA-directed or antibody-based or mRNA-directed, and/or wherein said second probe or probes is/are labeled with a label or labels, preferably with a single label or with two labels.

7. The method of any one of claims 3 to 6, further comprising determining select gram-positive bacteria of said sample that also possess said select trait.

8. The method of any one of claims 1 to 7, further comprising, prior to performing step (a), contacting said sample with a mRNA inducing reagent or reagents, and/or treating said sample with an RNase inhibitor.

9. The method of any one of claims 1 to 8, wherein all labels are fluorescent labels and said method is a fluorescent in-situ hybridization (FISH) assay.

10. The method of any one of claims 1 to 9, wherein said select trait is associated with 1) antibiotic resistance; 2) toxin production; and/or 3) virulence, preferably wherein said select trait is determined by determining the bacteria that possess: 1) a mecA gene or *vanA* or *vanB* gene; 2) a tcdB gene; and/or 3) a *lukF* or *lukS* gene, respectively.

11. The method of any one of claims 1 to 10 wherein the method is practiced without contacting the sample with an enzyme-based cell permeabilizing reagent or reagents.

12. The method of any of claims 1 to 11, wherein said method further comprises contacting said sample with; 1) a bacteria-directed third probe or probes capable of determining a second select gram-positive bacteria in said sample; and/or 2) a mixture of chromosomal DNA, mRNA-directed and/or native plasmid-directed fourth labeled PNA probes capable of determining chromosomal DNA, mRNA and/or plasmid nucleic acid associated with a second select trait that may be present in any bacteria of said sample.

13. The method of any one of claims 1 to 12, wherein said label or labels of said first and/or fourth labeled PNA probes is/are determined directly.

## Patentansprüche

1. Ein Verfahren umfassend:
a) Kontaktieren einer Probe umfassend Bakterien mit einem Gemisch aus ersten markierten PNA-Sonden, wobei die ersten markierten PNA-Sonden chromosomale DNA-, mRNA- und/oder nativ Plasmid-gerichtet sind und geeignet sind zur Bestimmung chromosomaler DNA, mRNA und/oder Plasmidnukleinsäuren, die mit einem ausgewählten Merkmal verbunden sind, welches im Besitz ausgewählter Gram-positiver Bakterien des besagten Gemisches sein kann; und
b) Bestimmung Gram-positiver Bakterien der genannten Probe, welche das genannte ausgewählte Merkmal besitzen;
wobei,
i) genanntes Verfahren an ganzen Zellen ausgeführt wird;
ii) jede der genannten ersten markierten PNA-Sonden mit einer einzigen Markierung oder mit zwei Markierungen markiert ist; und
iii) das Verfahren genutzt wird ohne die Verwendung von in-situ PCR und ohne Signalverstärkung der genannten ersten markierten PNA-Sonden.

2. Das Verfahren von Anspruch 1,
wobei besagte erste markierte PNA-Sonden chomosomale DNA- und/oder mRNA-gerichtet sind und geeignet sind zur Bestimmung von Methicillin-Resistenz in Bakterien der genannten Probe, und
wobei Gram-positive Bakterien, die Methicillin-Resistenz besitzen in Schritt b) bestimmt werden.

3. Das Verfahren von Anspruch 1, in Schritt a) weiterhin umfassend die Kontaktierung der Probe mit einer zweiten Sonde oder Sonden, wobei genannte zweite Sonde oder Sonden Bakterien-gerichtet sind und geeignet sind zur Bestimmung der ausgewählten Gram-positiven Bakterien in genannter Probe, und weiterhin umfassen
c) Bestimmung eines oder mehrerer genannter ausgewählten Gram-positiver Bakterien in genannter Probe,
wobei Schritte (b) und (c) in jeglicher Reihenfolge oder gleichzeitig durchgeführt werden,
vorzugsweise wobei,
aa) genannte erste markierte PNA-Sonden chromosomale DNA- und/oder mRNA-gerichtet sind und geeignet sind zur Bestimmung von Methicillin-Resistenz in Bakterien der genannte Probe,
bb) wobei genannte zweite Sonde oder Sonden geeignet sind zur Bestimmung von *S. aureus* Bakterien in genannter Probe,
cc) wobei Gram-positive Bakterien, welche Methicillin-Resistenz besitzen, in Schritt b) bestimmt werden, und
dd) wobei *S. aureus)* Bakterien in Schritt c) bestimmt werden.

4. Das Verfahren irgendeines der Ansprüche 1 bis 3, wobei die ersten markierten PNA-Sonden nur mRNA-gerichtete markierte Sonden sind.

5. Das Verfahren irgendeines der Ansprüche 1 bis 3, wobei kein Vorhybridisierung-Schritt erfolgt.

6. Das Verfahren irgendeines der Ansprüche 3 bis 5, wobei genannte zweite Sonde oder Sonden rRNA-gerichtet oder auf Antikörpern basiert oder mRNA-gerichtet ist/sind, und wobei genannte zweite Sonde oder Sonden markiert ist/sind mit einer Markierung oder Markierungen, vorzugsweise mit einer einzelnen Markierung oder mit zwei Markierungen.

7. Das Verfahren irgendeines der Ansprüche 3 bis 6, weiterhin umfassend die Bestimmung ausgewählter Gram-positiver Bakterien der genannte Probe, welche auch das genannte ausgewählte Merkmal besitzen.

8. Das Verfahren irgendeines der Ansprüche 1 bis 7, weiterhin umfassend, vorausgehend der Durchführung von Schritt (a), Kontaktieren der genannten Probe mit einem mRNA induzierenden Wirkstoff oder Wirkstoffen, und/oder Behandlung der genannten Probe mit einem RNase Inhibitor.

9. Das Verfahren irgendeines der Ansprüche 1 bis 8, wobei alle Markierungen fluoreszierende Markierungen sind und genanntes Verfahren ein Fluoreszenz-in-.ritu-Hybridisierungs (FISH)-Assay ist.

10. Das Verfahren irgendeines der Ansprüche 1 bis 9, wobei genanntes ausgewähltes Merkmal assozüert ist mit 1) Antibiotikaresistenz; 2) Toxinproduktion; und/oder 3) Virulenz, vorzugsweise wobei genanntes ausgewähltes Merkmal bestimmt wird durch Bestimmung der Bakterien welche entsprechend besitzen: 1) ein mecA Gen oder *vanA* oder *vanB* Gen; 2) ein *tcdB* Gen; und/oder 3) ein *lukF* oder *lukS* Gen.

11. Das Verfahren irgendeines der Ansprüche 1 bis 10, wobei das Verfahren ohne Kontaktierung der Probe mit einem Enzym basierten Zellpermeabilisations-Wirkstoff oder -Wirkstoffen ausgeführt wird.

12. Das Verfahren irgendeines der Ansprüche 1 bis 11, wobei genanntes Verfahren weiterhin Kontaktierung der genannten Probe umfasst mit; 1) einer Bakteriengerichteten dritten Sonde oder Sonden, welche geeignet ist/sind zur Bestimmung eines zweiten ausgewählten Gram-positiven Bakteriums in genannter Probe; und/oder 2) einem Gemisch aus chromosomale DNA-, mRNA-gerichtet und/oder nativer Plasmid-gerichteten vierten markierten PNA-Sonden, welche geeignet sind zur Bestimmung chromosomaler DNA, mRNA und/oder Plasmidnukleinsäure, die mit einem zweiten ausgewählten Merkmal assozüert sind, welches in jedem Bakterium in genannter Probe vorhanden sein kann.

13. Das Verfahren irgendeines der Ansprüche 1 bis 12, wobei genannte Markierung oder Markierungen der genannten ersten und/oder vierten markierten PNA-Sonden direkt bestimmt wird/werden.

## Revendications

1. Procédé comprenant :
a) la mise en contact d'un échantillon comprenant des bactéries avec un mélange de premières sondes d'APN marquées, les premières sondes d'APN marquées étant dirigées contre un ADN chromosomique et/ou un ARNm et/ou un plasmide natif et capables de déterminer l'ADN chromosomique, l'ARNm et/ou l'acide nucléique plasmidique associés à une caractéristique choisie qui peut être possédée par des bactéries gram positif choisies dudit échantillon ; et
b) la détermination des bactéries gram positif qui possèdent ladite caractéristique choisie dans ledit échantillon ;
dans lequel,
i) ledit procédé est réalisé sur des cellules totales ;
ii) lesdites premières sondes d'APN marquées sont chacune marquées avec un seul marqueur ou avec deux marqueurs ; et
iii) le procédé est réalisé sans utiliser de PCR in situ et sans amplification du signal dudit marqueur desdites premières sondes d'APN marquées.

2. Procédé selon la revendication 1,
dans lequel lesdites premières sondes d'APN marquées sont dirigées contre un ADN chromosomique et/ou un ARNm et capables de déterminer la résistance à la méthicilline chez les bactéries dudit échantillon, et
dans lequel les bactéries gram positif qui possèdent une résistance à la méthicilline sont déterminées dans l'étape b).

3. Procédé selon la revendication 1, comprenant en outre dans l'étape a) la mise en contact d'un échantillon avec une seconde sonde ou des secondes sondes, ladite ou lesdites secondes sondes étant dirigées contre des bactéries et capables de déterminer les bactéries gram positif choisies dans ledit échantillon, ledit procédé comprenant en outre
c) la détermination d'une ou de plusieurs bactéries gram positif choisies dans ledit échantillon ;
dans lequel les étapes (b) et (c) sont réalisées dans l'un ou l'autre ordre ou simultanément,
de préférence dans lequel
aa) lesdites premières sondes d'APN marquées sont dirigées contre un ADN chromosomique et/ou un ARNm et capables de déterminer la résistance à la méthicilline chez les bactéries dudit échantillon,
bb) dans lequel ladite ou lesdites secondes sondes sont capables de déterminer les bactéries *S. aureus* dans ledit échantillon,
cc) dans lequel les bactéries gram positif qui possèdent une résistance à la méthicilline sont déterminées dans l'étape b), et
dd) dans lequel les bactéries *S. aureus)* sont déterminées dans l'étape c).

4. Procédé selon l'une quelconque des revendications 1 à 3, dans lequel les premières sondes d'APN marquées sont uniquement des sondes marquées dirigées contre l'ARNm.

5. Procédé selon l'une quelconque des revendications 1 à 3, dans lequel aucune étape de pré-hybridation n'est réalisée.

6. Procédé selon l'une quelconque des revendications 3 à 5, dans lequel ladite ou lesdites secondes sondes est/sont dirigées contre un ARNr ou à base d'anticorps ou dirigées contre un ARNm, et/ou dans lequel ladite ou lesdites secondes sondes est/sont marquées avec un marqueur ou des marqueurs, de préférence avec un seul marqueur ou avec deux marqueurs.

7. Procédé selon l'une quelconque des revendications 3 à 6, comprenant en outre la détermination des bactéries gram positif qui possèdent également ladite caractéristique choisie dans ledit échantillon.

8. Procédé selon l'une quelconque des revendications 1 à 7, comprenant en outre, avant la réalisation de l'étape a), la mise en contact d'un échantillon avec un réactif ou des réactifs d'induction d'un ARNm, et/ou le traitement dudit échantillon avec un inhibiteur de la RNase.

9. Procédé selon l'une quelconque des revendications 1 à 8, dans lequel tous les marqueurs sont des marqueurs fluorescents et ledit procédé est un dosage d'hybridation *in situ* fluorescente (FISH).

10. Procédé selon l'une quelconque des revendications 1 à 9, dans lequel ladite caractéristique choisie est associée à 1) la résistance aux antibiotiques ; 2) la production de toxines ; et/ou 3) la virulence, de préférence dans lequel ladite caractéristique choisie est déterminée par détermination des bactéries qui possèdent : 1) un gène mecA ou un gène *vanA* ou vanB ; 2) un gène tcdB ; et/ ou 3) un gène *lukF* ou lukS, respectivement.

11. Procédé selon l'une quelconque des revendications 1 à 10, dans lequel ledit procédé est réalisé sans mettre en contact l'échantillon avec un réactif ou des réactifs de perméabilisation cellulaire à base d'enzymes.

12. Procédé selon l'une quelconque des revendications 1 à 11, dans lequel ledit procédé comprend en outre la mise en contact dudit échantillon avec ; 1) une ou des troisièmes sondes dirigées contre des bactéries et capables de déterminer des secondes bactéries gram positif choisies dans ledit échantillon ; et/ou 2) un mélange de quatrièmes sondes d'APN marquées dirigées contre un ADN chromosomique et/ou un ARNm et/ou un plasmide natif et capables de déterminer l'ADN chromosomique, l'ARNm et/ou l'acide nucléique plasmidique associés à une seconde caractéristique choisie qui peut être présente dans quelconque bactérie dudit échantillon.

13. Procédé selon l'une quelconque des revendications 1 à 12, dans lequel ledit marqueur ou lesdits marqueurs desdites premières et/ou quatrièmes sondes d'APN marqué est/sont directement déterminés .


### SEQUENCE LISTING

<110> AdvanDx, Inc.
   Stender, Henrik
   Trnovsky, Jan
   Klimas, Lisa L
   Rasmussen, Anne K
<120> Methods For Whole-Cell Analysis Of Gram-Positive Bacteria
<130> ADXP001-PCT
<150> US 61/179,900
   <151> 2009-05-20
<150> US 61/293,674
   <151> 2010-01-10
<160> 33
<170> PatentIn version 3.5
<210> 1
   <211> 16
   <212> DNA
   <213> Staphylococcus aureus
<400> 1
   gtatttctga agacta 16
<210> 2
   <211> 15
   <212> DNA
   <213> Staphylococcus aureus
<400> 2
   gctatcgtgt cacaa 15
<210> 3
   <211> 16
   <212> DNA
   <213> Staphylococcus aureus
<400> 3
   gctccaacat gaagat 16
<210> 4
   <211> 16
   <212> DNA
   <213> Staphylococcus aureus
<400> 4
   gatgatgcag ttattg 16
<210> 5
   <211> 15
   <212> DNA
   <213> Staphylococcus aureus
<400> 5
   gatgatacct tcgtt 15
<210> 6
   <211> 15
   <212> DNA
   <213> Escherichia coli
<400> 6
   tcaatgagca aaggt 15
<210> 7
   <211> 15
   <212> DNA
   <213> Staphylococcus aureus
<400> 7
   gctatcgtgt cacaa 15
<210> 8
   <211> 15
   <212> DNA
   <213> Staphylococcus aureus
<400> 8
   gcttctcgtc cgttc 15
<210> 9
   <211> 15
   <212> DNA
   <213> Staphylococcus epidermidis
<400> 9
   tcctcgtctg ttcgc 15
<210> 10
   <211> 15
   <212> DNA
   <213> Candida albicans
<400> 10
   agagagcagc atcca 15
<210> 11
   <211> 16
   <212> DNA
   <213> Staphylococcus aureus
<400> 11
   gtatttctga agacta 16
<210> 12
   <211> 15
   <212> DNA
   <213> Staphylococcus aureus
<400> 12
   gctatcgtgt cacaa 15
<210> 13
   <211> 16
   <212> DNA
   <213> Staphylococcus aureus
<400> 13
   gctccaacat gaagat 16
<210> 14
   <211> 16
   <212> DNA
   <213> Staphylococcus aureus
<400> 14
   gatgatgcag ttattg 16
<210> 15
   <211> 15
   <212> DNA
   <213> Staphylococcus aureus
<400> 15
   gatgatacct tcgtt 15
<210> 16
   <211> 16
   <212> DNA
   <213> Staphylococcus aureus
<400> 16
   cacattgttt cggtct 16
<210> 17
   <211> 16
   <212> DNA
   <213> Staphylococcus aureus
<400> 17
   cattagttgt aagatg 16
<210> 18
   <211> 16
   <212> DNA
   <213> Staphylococcus aureus
<400> 18
   tcttcagagt taatgg 16
<210> 19
   <211> 16
   <212> DNA
   <213> Staphylococcus aureus
<400> 19
   gctattatcg tcaacg 16
<210> 20
   <211> 15
   <212> DNA
   <213> Staphylococcus aureus
<400> 20
   agcatcaata gttag 15
<210> 21
   <211> 15
   <212> DNA
   <213> Staphylococcus aureus
<400> 21
   tgtgcttaca agtgc 15
<210> 22
   <211> 15
   <212> DNA
   <213> Staphylococcus aureus
<400> 22
   tacctgagcc ataat 15
<210> 23
   <211> 17
   <212> DNA
   <213> Staphylococcus aureus
<400> 23
   cttcgttact catgcca 17
<210> 24
   <211> 16
   <212> DNA
   <213> Staphylococcus aureus
<400> 24
   tagtcttcag aaatac 16
<210> 25
   <211> 15
   <212> DNA
   <213> Staphylococcus aureus
<400> 25
   aacgaaggta tcatc 15
<210> 26
   <211> 16
   <212> DNA
   <213> Staphylococcus aureus
<400> 26
   caataactgc atcatc 16
<210> 27
   <211> 16
   <212> DNA
   <213> Staphylococcus aureus
<400> 27
   atcttcatgt tggagc 16
<210> 28
   <211> 16
   <212> DNA
   <213> Staphylococcus aureus
<400> 28
   atcttcatgt tggagc 16
<210> 29
   <211> 16
   <212> DNA
   <213> Staphylococcus aureus
<400> 29
   acgatgccta tctcat 16
<210> 30
   <211> 16
   <212> DNA
   <213> Staphylococcus aureus
<400> 30
   gatagttacg actttc 16
<210> 31
   <211> 16
   <212> DNA
   <213> Staphylococcus aureus
<400> 31
   atgtatgtgc gattgt 16
<210> 32
   <211> 16
   <212> DNA
   <213> Staphylococcus aureus
<400> 32
   gatcaatgtt accgta 16
<210> 33
   <211> 16
   <212> DNA
   <213> Staphylococcus aureus
<400> 33
   cgctatgatc ccaatc 16